# EUROPÄISCHE PATENTSCHRIFT

(11) **EP 0 393 438 B1**
(45) Veröffentlichungstag und Bekanntmachung des Hinweises auf die Patenterteilung: **16.02.2005**
(21) Anmeldenummer: 90106624.1
(22) Anmeldetag: 06.04.1990
(51) Int. Cl.: C12N 15/12, C12P 21/02, C07K 14/00

(54) **TNF-Rezeptor, TNF bindende Proteine und dafür kodierende DNAs**
TNF-receptor, TNF-binding protein and DNA coding therefor
Récepteur du TNF, protéine liant le TNF at ADN codant pour ceux-ci

(30) Priorität: 21.04.1989 DE 3913101; 21.06.1989 DE 3920282
(43) Veröffentlichungstag der Anmeldung: 24.10.1990
(73) Patentinhaber: Amgen Inc., Thousand Oaks, CA 91320-1799 (US)
(72) Erfinder: Hauptmann, Rudolf, Dr., A-2483 Ebreichsdorf (AT); Himmler, Adolf, Dr., A-1236 Wien (AT); Maurer-Fogy, Ingrid, Dr., A-1238 Wien (AT); Stratowa, Christian, Dr., A-1010 Wien (AT)
(74) Vertreter: Grünecker, Kinkeldey, Stockmair & Schwanhäusser Anwaltssozietät

(56) Entgegenhaltungen:
- EP-A- 0 162 699
- EP-A- 0 308 378
- EP-A- 0 417 563
- EP-A- 0 433 900
- IL-A- 92 697
- SHIMIZU K. ET AL: 'Three transforming genes are related to the viral ras oncogenes' PROC. NATL. ACAD. SCI. USA Bd. 80, April 1983, Seiten 2112 - 2116
- Thesis for the Degree of Doctor of Philosophy by Oliver Kemper. Copy of front page and page 29. The Weizmann Institute of Science. Rehovot, Israel. November, 1994.

## Beschreibung

Die Erfindung bezieht sich auf eine DNA, die für ein Polypeptid mit der Fähigkeit, TNF zu binden, kodiert, eine Nukleinsäure, ein rekombinantes DNA-Molekül, wirtszellen, Verfahren zum Herstellen eines rekombinanten TNF-bindenden Proteins, ein rekombinantes Polypeptid, die Verwendung des rekombinanten Polypeptides und eine pharmazeutische Zusammensetzung.

Tumorkrosefaktor (TNF-α) wurde erstmals im Serum von Mäusen und Kaninchen gefunden, die mit Bacillus Calmette-Guerin infiziert und denen Endotoxin injiziert worden war, und auf Grund seiner cytotoxischen und Antitumoreigenschaften erkannt (Carswell et al., 1975). Er wird vor allem von aktivierten Makrophagen und Monozyten produziert. Zahlreiche Zelltypen, die Ziele für TNF sind, wiesen Oberflächenrezeptoren mit hoher Affinität für dieses Polypeptid auf (Old et al., 1987); es wurde angenommen, dass Lymphotoxin (TNF-β) an denselben Rezeptor bindet (Aggarwal et al., 1985, Gullberg et al., 1987). TNF-α ist identisch mit einem als Cachectin bezeichneten Faktor (Beutler et al., 1985), der die Lipoproteinlipase unterdrückt und bei chronisch-entzündlichen und malignen Erkrankungen zur Hypertriglyceridämie führt (Torti et al., 1985, Mahoney et al., 1985). TNF-α dürfte an der Regulation des Wachstums sowie an der Differenzierung und Funktion von Zellen, die bei Entzündungen, Immunvorgängen und Hämatopoese eine Rolle spielen, beteiligt sein.

TNF kann auf den Wirtsorganismus durch Stimulation von Neutrophilen (Shalaby et al., 1985, Klebanoff et al., 1986) und Monocyten sowie durch Hemmung der Replikation von Viren (Mestan et al., 1986, Wong et al., 1986) eine positive Wirkung ausüben. Darüber hinaus aktiviert TNF-α die Immunabwehr gegen Parasiten und wirkt direkt und/oder indirekt als Mediator bei Immunreaktionen, entzündlichen Prozessen und anderen Vorgängen im Organismus, wobei die Wirkmechanismen in vielen Fällen noch ungeklärt sind. Die Verabreichung von TNF-α (Cerami et al., 1988) kann jedoch auch von schädlichen Erscheinungen (Tracey et al., 1986) wie Schock- und Gewebeschädigungen begleitet sein, die durch Antikörper gegen TNF-α aufgehoben werden können (Tracey et al., 1987). Eine Reihe von Beobachtungen läßt auf eine Rolle von endogen freigesetztem TNF-α bei verschiedenen pathologischen Zuständen schließen. So scheint TNF-α ein Mediator der Kachexie zu sein, die bei chronisch-invasiven, z.B. parasitären Erkrankungen auftreten kann. TNF-α scheint auch eine wesentliche Rolle bei der Pathogenese des durch gram-negative Bakterien verursachten Schocks (Endotoxin-Schock) zu spielen; er dürfte an einigen, wenn nicht allen Wirkungen von Lipopolysacchariden beteiligt sein (Beutler et al., 1988). Ebenso wurde eine Funktion von TNF bei den im Rahmen von entzündlichen Prozessen in Gelenken und anderen Geweben auftretenden Gewebeschädigungen sowie bei der Letalität und Morbidität der Graft-versus-host reaction (GVHR, Transplantat-Abstoßung (Piguet et al., 1987) postuliert. Auch wurde ein Zusammenhang zwischen der Konzentration von TNF im Serum und dem tödlichen Ausgang von Meningokokkenerkrankungen berichtet (Waage et al., 1987).

Weiters wurde beobachtet, daß die Verabreichung von TNF-α über einen längeren Zeitraum einen Zustand von Anorexie und Auszehrung verursacht, die eine ähnliche Symptomatik aufweist wie die Kachexie, die mit neoplastischen und chronischen infektiösen Erkrankungen einhergeht (Oliff et al., 1987).

Es wurde über eine TNF inhibierende Aktivität eines Proteins aus dem Harn von Fieberpatienten berichtet, von dessen Wirkung vermutet wird, daß sie auf einen kompetitiven Mechanismus auf Rezeptorebene selbst (ähnlich der Wirkung des Interleukin-1 Inhibitors (Seckinger et al., 1987)) zurückzuführen ist (Seckinger et al., 1988).

In der EP-A2 308 378 wird ein TNF inhibierendes Protein mit einem Molekulargewicht von nur 26 bis 28 kD beschrieben, das aus menschlichem Harn gewonnen wurde. Seine Wirkung wurde im Harn gesunder und kranker Personen nachgewiesen und aufgrund der Fähigkeit bestimmt, die Bindung von TNF-α an seine Rezeptoren auf humanen HeLa Zellen und FS 11 Fibroblasten sowie die zytotoxische Wirkung von TNF-α auf murine A9 Zellen zu inhibieren. Das Protein wurde im Wesentlichen zur Homogenität gereinigt und durch seinen N-Terminus charakterisiert. In dieser Patentveröffentlichung werden zwar grundsätzlich mögliche Wege dargelegt, zur der für das Protein kodierenden DNA und zum rekombinanten Protein zu gelangen; es werden jedoch keine konkreten Angaben gemacht, welcher der theoretisch möglichen Lösungswege zum Ziel führt.

In Vorversuchen zur vorliegenden Erfindung konnte aus Dialyseharn von Urämiepatienten ebenfalls ein Protein identifiziert werden, das die biologischen Wirkungen von TNF-α hemmt, indem es durch Wechselwirkung mit TNF-α dessen Bindung an seinen Zelloberflächenrezeptor verhindert (Olsson et al., 1988). Von diesem Protein wurde auch eine Affinität zu TNF-β festgestellt.

Die Anwesenheit dieses Proteins (im folgenden TNF-BP genannt) im konzentrierten Dialyseharn wurde durch Kompetition mit der Bindung von radioaktiv markiertem rekombinantem TNF-α an einen Subklon von HL-60 Zellen nachgewiesen, wobei der Einfluß von dialysiertem Harn auf die Bindung von ¹²⁵I-TNF-α an die Zellen gemessen wurde. Die durchgeführten Bindungsversuche zeigten eine dosisabhängige Hemmung der TNF-α-Bindung an die Zelle durch konzentrierten Dialyseharn (die Möglichkeit der Interpretation, daß die beobachtete Verringerung der Bindung durch gegebenenfalls im Harn vorhandenen TNF-α selbst oder TNF-β, der um die Bindung konkurriert, verursacht werden könnte, wurden durch den Befund, daß die Verringerung der Bindung durch Anwendung von TNF-α-und TNF-β-Antikörpern nicht aufgehoben werden konnte, ausgeschlossen).

In analoger Weise wurde in Vorversuchen zur vorliegenden Erfindung nachgewiesen, daß TNF-BP auch Affinität zu TNF-β aufweist, sie beträgt ca. 1/50 seiner Affinität zu TNF-α.

Mittels Gelchromatographie auf Sephacryl 200 wurde festgestellt, daß eine Substanz im Harn und Serum von Dialysepatienten sowie im Serum von gesunden Personen mit rekombinantem TNF-α einen Komplex mit einem Molekulargewicht von ca. 75 000 bildet.

TNF-BP wurde aus mehreren Proben Dialyseharn von Urämiepatienten durch partielle Reinigung mittels Druckultrafiltration, Ionenaustauschchromatographie und Gelchromatographie 62fach angereichert.

Die erhaltenen Präparationen wurden zum Nachweis der biologischen Aktivität von TNF-BP durch Hemmung der wachstumshemmenden Wirkung von TNF-α auf HL-60-10 Zellen verwendet. Es zeigte sich eine dosisabhängige Wirkung von TNF-BP auf die biologische Wirkung von TNF-α. Es wurde weiters das Bindungsverhalten von Zellen durch Vorbehandlung mit TNF-BP und ausschließendem Kompetitionsbindungstest untersucht. Dabei wurde nachgewiesen, daß Vorbehandlung der Zellen mit TNF-BP die Bindung von TNF-α an die Zelle nicht beeinträchtigt. Dies zeigt, daß die Wirkung von TNF-BP nicht auf seiner etwaigen Bindung an die Zelle und Konkurrenz mit TNF-α um die Bindung an den Rezeptor beruht.

Das im wesentlichen homogene Protein wurde in hochgereinigter Form erhalten, indem Harn von Dialysepatienten durch Ultrafiltration konzentriert, der konzentrierte Harn dialysiert und zunächst in einem ersten Reinigungsschritt mittels DEAE-Sephacel-Chromatographie auf das Vierfache angereichert wurde. Die weitere Anreicherung erfolgte mittels Affinitätschromatographie durch an Sepharose gebundenen TNF-α. Die Endreinigung wurde mittels Reverse Phase Chromatographie (FPLC) durchgeführt.

Es konnte gezeigt werden, daß das im wesentlichen hochgereinigte Protein die zytotoxische Wirkung von TNF-α auf WEHI 164 Klon 13 Zellen hemmt (Olsson et al., 1989).

Vom im wesentlichen hochgereinigten Protein wurde die N-terminale Aminosäuresequenz aufgeklärt. Sie wurde mit Asp-Ser-Val-Xaa-Pro-Gln-Gly-Lys-Tyr-Ile-His-Pro-Gln-(Hauptsequenz) bestimmt (daneben wurde in Spuren die folgende N-terminale Sequenz nachgewiesen:
Leu-(Valj-(Pro)-(His)-Leu-Gly-Xaa-Arg-Glu-(Nebensequenz)). Der Vergleich der Hauptsequenz mit der N-terminalen Sequenz des in der EP-A2 308 378 geoffenbarten TNF inhibierenden Proteins zeigt die Identität der N-terminalen Sequenz der beiden Proteine.

Es wurde folgende Aminosäurezusammensetzung, angegeben in Mol-Aminosäure pro Mol Protein und in Mol % Aminosäure, bestimmt als Mittelwert einer 24- und 48-stündigen Hydrolyse, ermittelt:

| | Mol Aminosäure/ Mol Protein | Mol % Aminosäure |
|---|---|---|
| Asp + Asn | 27,5 | 10,9 |
| Thr | 15,8 | 6,3 |
| Ser | 20,7 | 8,2 |
| Glu + Gln | 35,0 | 13,8 |
| Pro | 9 , 5 | 3 , 8 |
| Gly | 16,0 | 6,3 |
| Ala | 4,2 | 1,7 |
| Cys | 32,3 | 12,8 |
| Val | 10,8 | 4,3 |
| Met | 1,1 | 0,4 |
| Ile | 7,0 | 2,8 |
| Leu | 20,2 | 8,0 |
| Tyr | 6,1 | 2,4 |
| Phe | 8,1 | 3,2 |
| His | 11,1 | 4,4 |
| Lys | 15,7 | 6,2 |
| Arg | 11,8 | 4,7 |
| Total | 252,9 | 100 |

Ein Gehalt an Glukosamin wurde mittels Aminosäureanalyse nachgewiesen. Die Ergebnisse eines mit Concanavalin A und Weizenkeimlektin durchgeführten Affinoblots zeigten ebenfalls, daß es sich bei TNF-BP um ein Glykoprotein handelt.

Das im wesentlichen homogene Protein wurde tryptisch verdaut und von 17 der erhaltenen Spaltpeptide die Aminosäuresequenzen bestimmt. Weiters wurde der C-Terminus analysiert.

TNF-BP kommt offensichtlich die Funktion eines Regulators der TNF-Aktivität mit der Fähigkeit zu, die Konzentrationsänderungen von freiem, biologisch aktivem TNF-α abzupuffern. TNF-BP dürfte auch die Ausscheidung von TNF durch die Niere beeinflussen, weil der mit TNF gebildete Komplex, dessen Molekulargewicht mittels Gelpermeationschromatographie auf Sephadex G 75 mit ca. 75000, bestimmt wurde, im Gegensatz zu TNF offensichtlich nicht durch den Glomerulus zurückgehalten wird.

Das TNF-BP wurde aus dem Harn von Dialysepatienten als eine von drei Hauptproteinkomponenten, die Affinität zu TNF aufweisen und die gemeinsam mit TNF-BP von der TNF-Affinitätschromatographiesäule eluieren, nachgewiesen. Die beiden anderen Proteine binden jedoch offensichtlich in einer Weise, die die Bindung von TNF-α an seinen Zelloberflächenrezeptor nicht beeinträchtigt.

Die zur biologischen Wirkung des TNF-BP erhaltenen Ergebnisse, insbesondere der Vergleich der Bindungskonstante mit der für den TNF-Rezeptor beschriebenen Bindungskonstante (Creasey et al., 1987) lieferten einen ersten Hinweis dafür, daß es sich bei diesem Protein um den löslichen Teil eines TNF-Rezeptors handeln könnte.

Auf Grund seiner Fähigkeit, die biologische Wirkung von TNF-α und TNF-β zu inhibieren, ist das TNF bindende Protein geeignet, bei Indikationen eingesetzt zu werden, bei denen eine Herabsetzung der TNF-Aktivität im Organismus angezeigt ist. Geeignet zur Anwendung bei diesen Indikationen sind auch Derivate oder Fragmente des TNF bindenden Proteins mit der Fähigkeit, die biologische Wirkung von TNF zu inhibieren.

TNF-BP (bzw. seine funktionellen Derivate oder aktiven Fragmente) kann zur prophylaktischen und therapeutischen Behandlung des menschlichen oder tierischen Körpers bei Indikationen, bei denen eine schädigende Wirkung von TNF-α auftritt, eingesetzt werden. Zu diesen Erkrankungen zählen insbesondere entzündliche sowie infektiöse und parasitäre Erkrankungen oder Schockzustände, bei denen endogenes TNF-α freigesetzt wird, weiter Kachexie, GVHR, ARDS (Adult Respiratory Distress Symptom) und Autoimmunerkrankungen wie Rheumatoide Arthritis, etc. Es sind darunter auch pathologische Zustände zu verstehen, die als Nebenwirkungen bei der Therapie mit TNF-α, besonders bei hoher Dosierung, auftreten können, z.B. schwere Hypotension oder Störungen des Zentralnervensystems.

Auf Grund seiner TNF bindenden Eigenschaften ist TNF-BP auch als Diagnostikum für die Bestimmung von TNF-α und/oder TNF-β geeignet, z.B. als eine der Komponenten in Radioimmunoassays oder Enzymimmunoassays, gegebenenfalls zusammen mit Antikörpern gegen TNF.

Auf Grund seiner Eigenschaften ist dieses Protein ein pharmakologisch wertvoller Wirkstoff, der aus natürlichen Quellen nicht in ausreichender Menge mittels proteinchemischer Methoden darstellbar ist.

Es bestand daher das Bedürfnis, dieses Protein (bzw. verwandte Proteine mit der Fähigkeit, TNF zu binden) auf rekombinantem Weg herzustellen, um es für die therapeutische Anwendung in ausreichender Menge zur Verfügung zu stellen.

Unter der "Fähigkeit, TNF zu binden" ist im Rahmen der vorliegenden Erfindung die Eigenschaft eines Proteins zu verstehen, an TNF-α derart zu binden, daß die Bindung von TNF-α an den funktionellen Teil des Rezeptors verhindert und die Wirkung von TNF-α im menschlichen oder tierischen Organismus gehemmt oder aufgehoben wird. Durch diese Definition ist die Fähigkeit eines Proteins, auch an andere Proteine, z.B. an TNF-β, zu binden und deren Wirkung inhibieren zu können, mit eingeschlossen.

Der vorliegenden Erfindung lag die Aufgabe zugrunde, die für TNF-BP kodierende DNA zur Verfügung zu stellen, um auf deren Basis die Herstellung rekombinanter DNA-Moleküle zu ermöglichen, mit denen geeignete Wirtsorganismen transformiert werden können, um TNF-BP bzw. funktionelle Derivate und Fragmente davon zu produzieren.

Im Rahmen dieser Aufgabenstellung sollte auch festgestellt werden, ob es sich beim TNF-BP um den löslichen Teil eines TNF-Rezeptors handelt. Diese Annahme wurde bestätigt, womit die Grundlage für die Aufklärung der Rezeptorsequenz geschaffen war.

Eine weitere Aufgabenstellung im Rahmen der vorliegenden Erfindung bestand in der Bereitstellung der für einen TNF-Rezeptor kodierenden cDNA für die Herstellung von rekombinantem humanen TNF-Rezeptor.

Das Vorhandensein eines spezifischen Rezeptors mit hoher Affinität zu TNF-α auf verschiedenen Zelltypen wurde von mehreren Arbeitsgruppen gezeigt. Kürzlich wurde erstmals von der Isolierung und vorläufigen Charakterisierung eines TNF-α Rezeptors berichtet (Stauber et al., 1988). Da die Bindung von radioaktiv markiertem TNF-α durch einen Überschuß an TNF-β aufgehoben werden kann (Aggarwal et al., 1985), wurde vorgeschlagen, daß TNF-α und TNF-β einen gemeinsamen Rezeptor teilen. Da jedoch andererseits gezeigt werden konnte, daß bestimmte Zelltypen, die auf TNF-α ansprechen, gegen TNF-β teilweise oder gänzlich unempfindlich sind (Locksley et al., 1987), wurde die Existenz eines gemeinsamen Rezeptors wieder in Zweifel gezogen.

Im Gegensatz dazu scheinen kürzlich erhaltene Ergebnisse zu den Bindungseigenschaften von TNF-β an Rezeptoren die Theorie eines gemeinsamen Rezeptors wieder zu erhärten (Stauber et al., 1989), wobei in dieser Arbeit vorgeschlagen wird, daß zwischen TNF-α und TNF-β Unterschiede hinsichtlich der Wechselwirkung mit dem Rezeptor bzw. zusätzlich hinsichtlich der in der Zelle nach der Ligand-Rezeptorwechselwirkung eintretenden Ereignisse bestehen.

Kürzlich wurde von einem weiteren TNF bindenden Protein berichtet, von dem vermutet wird, daß es sich dabei um die lösliche Form eines anderen TNF-Rezeptors handelt (Engelmann et al., 1990).

Die Verfügbarkeit der für einen TNF-Rezeptor kodierenden DNA stellt die Voraussetzung für die Herstellung von rekombinantem Rezeptor und damit u.a. eine wesentliche Erleichterung für die Durchführung vergleichender Untersuchungen verschiedener Zelltypen auf ihre(n) TNF-α- und/oder TNF-β-Rezeptor(en) bzw. auf die durch die Bindung von TNF an den Rezeptor in der Zelle ausgelösten Reaktionen dar. Dadurch wird weiter die Aufklärung der dreidimensionalen Struktur des Rezeptors ermöglicht und damit die Voraussetzung für ein rationales Design für die Entwicklung von Agonisten und Antagonisten der TNF-Wirkung geschaffen.

Bei der Lösung der gestellten Aufgabe wurde von der Feststellung ausgegangen, daß das Durchsuchen von cDNA-Bibliotheken mit Hilfe von Hybridisierungssonden, die von Aminosäuresequenzen kurzer Peptide abgeleitet sind, auf Grund der Degeneration des genetischen Codes mitunter auf größere Schwierigkeiten stößt. Zusätzlich erschwert wird diese Vorgangsweise dann, wenn von einem Protein, wie z.B. dem TNF-BP, nicht bekannt ist, in welchen Geweben es synthetisiert wird. In diesem Fall kann bei einem Versagen dieser Methode unter Umständen nicht mit Bestimmtheit festgestellt werden, ob es auf die Wahl einer ungeeigneten cDNA-Bibliothek oder auf die zu geringe Spezifität der Hybridisierungssonden zurückzuführen ist.

Um die für TNF-BP kodierende DNA zu erhalten, wurde daher zunächst erfindungsgemäß wie folgt vorgegangen:

Als cDNA-Bibliothek wurde eine Bibliothek der Fibrosarkomzellinie HS913 T, die mit TNFα induziert worden war und in λ gt11 vorlag, eingesetzt. Um aus dieser Bibliothek λ DNA mit TNF-BP Sequenzen zu erhalten, wurde die große Empfindlichkeit der Polymerase Kettenreaktion (PCR, (Saiki, 1988)) ausgenützt. (Mit Hilfe dieser Methode kann aus einer gesamten cDNA-Bibliothek eine unbekannte DNA-Sequenz erhalten werden, die flankiert ist von Oligonukleotiden, die auf Basis bekannter Aminosäureteilsequenzen entworfen und als Primer eingesetzt wurden. Ein solches längeres DNA-Fragment kann nachfolgend als Hybridisierungssonde, z.B. zur Isolierung von cDNA-Klonen, insbesondere des ursprünglichen cDNA-Klons, eingesetzt werden).

Auf Basis der N-terminalen Aminosäuresequenz (Hauptsequenz) und Aminosäuresequenzen von tryptischen Peptiden, die vom hochgereinigten TNF-BP erhalten worden waren, wurden Hybridisierungssonden hergestellt. Mit Hilfe dieser Sonden wurde mittels PCR aus der cDNA-Bibliothek HS913T eine cDNA, die einen Teil der für TNF-BP kodierenden cDNA darstellt, erhalten. Diese cDNA weist die folgende Nukleotidsequenz auf:

CAG GGG AAA TAT ATT CAC CCT CAA AAT AAT TCG ATT TGC TGT
ACC AAG TGC CAC AAA GGA ACC TAC TTG TAC AAT GAC TGT CCA
GGC CCG GGG CAG GAT ACG GAC TGC AGG GAG TGT GAG AGC GGC
TCC TTC ACA GCC TCA GAA AAC AAC AAG .

Diese DNA stellt eine von möglichen Varianten dar, die geeignet sind, mit TNF-BP-DNAs bzw. TNF-BP-RNAs zu hybridisieren (solche Varianten umfassen z.B. diejenigen DNA-Moleküle, die durch PCR-Amplifikation mit Hilfe von Primern erhalten werden, deren Nukleotidsequenz nicht exakt mit der gesuchten Sequenz übereinstimmt, etwa aufgrund von zu Klonierungszwecken vorgesehenen Restriktionsschnittstellen oder aufgrund von bei der Aminosäuresequenzanalyse etwa nicht eindeutig ermittelten Aminosäuren). Unter "TNF-BP-DNAs" bzw. "TNF-BP-RNAs" sind Nukleinsäuren zu verstehen, die für TNF-BP bzw. verwandte Proteine mit der Fähigkeit, TNF zu binden kodieren bzw. die für ein solches Protein kodierende Sequenz enthalten.

Unter TNF-BP-DNAs (bzw. TNF-BP-RNAs) sind auch cDNAs, abgeleitet von mRNAs, die durch alternatives Splicing entstanden sind (bzw. diese mRNAs selbst), mitumfaßt. Unter "alternativem Splicing" wird die Entfernung von Introns verstanden, bei der vom gleichen mRNA-Precursor verschiedene Spliceacceptor- und/oder Splicedonorstellen verwendet werden. Die dabei entstehenden mRNAs unterscheiden sich voneinander durch das gänzliche oder teilweise Vorhandensein oder Fehlen von bestimmten Exonsequenzen, wobei es gegebenenfalls zu einer Verschiebung des Leserasters kommen kann.

Die erfindungsgemäß zunächst erhaltene, einen Teil der für TNF-BP kodierenden Sequenz enthaltende cDNA (bzw. Varianten davon) kann somit als Hybridisierungssonde verwendet werden, um aus cDNA-Bibliotheken cDNA-Klone, enthaltend TNF-BP-DNAs, zu erhalten. Weiter kann sie als Hybridisierungssonde für mRNA-Präparationen eingesetzt werden, um TNF-BP-RNAs zu isolieren und daraus z.B. angereicherte cDNA-Bibliotheken herzustellen, die ein wesentlich vereinfachtes und effizienteres Screening ermöglichen. Ein weiteres Anwendungsgebiet ist die Isolierung der gewünschten DNAs aus genomischen DNA-Bibliotheken mit Hilfe dieser DNAs als Hybridisierungssonden.

Die oben definierte DNA (bzw. ihre Varianten) ist in der Lage, mit DNAs (bzw. RNAs) zu hybridisieren, die für TNF-BP kodieren bzw. die für TNF-BP kodierende Sequenz enthalten. Mit Hilfe dieser DNA als Sonde können auch cDNAs erhalten werden, die für Proteine kodieren, deren Prozessierung TNF-BP ergibt. Unter Prozessierung ist die in vivo Abspaltung von Teilsequenzen zu verstehen. Dabei kann es sich N-terminal um die Signalsequenz und/oder andere Sequenzen und gegebenenfalls zusätzlich C-terminal um die transmembrane und zytoplasmatische Region des Rezeptors handeln. Mit Hilfe dieser Hybridisierungssonde ist es daher möglich, geeignete cDNA-Bibliotheken auf das Vorhandensein von cDNA, die die vollständige für einen TNF-Rezeptor kodierende Sequenz enthält, zu durchsuchen (dieser Vorgang kann erforderlichenfalls in mehreren Schritten erfolgen).

Erfindungsgemäß wurde die cDNA der oben definierten Sequenz, die mittels PCR aus der cDNA-Bibliothek der TNF-α induzierten Fibrosarkomzellinie HS913 T (in λ gtll) erhalten worden war, zum nochmaligen Durchsuchen der cDNA-Bibliothek verwendet, von den hybridisierenden Klonen die Lamda-DNA präpariert, subkloniert und sequenziert. Es wurde ein 1334 Basen langes cDNA Insert erhalten, das die für TNF-BP kodierende Sequenz enthält.

Es wurden somit zunächst DNAs, kodierend für ein Polypeptid mit der Fähigkeit, TNF zu binden, bzw. für ein Polypeptid, von dem dieses TNF bindende Protein eine Teilsequenz darstellt, bereitgestellt. Darunter sind auch solche DNAs zu verstehen, die für Teile dieser Polypeptide kodieren.

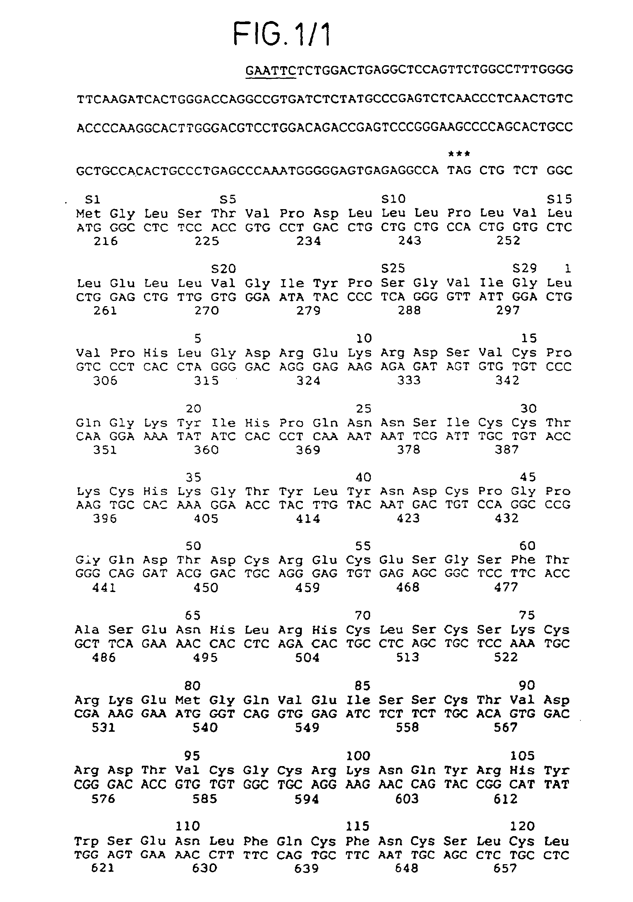

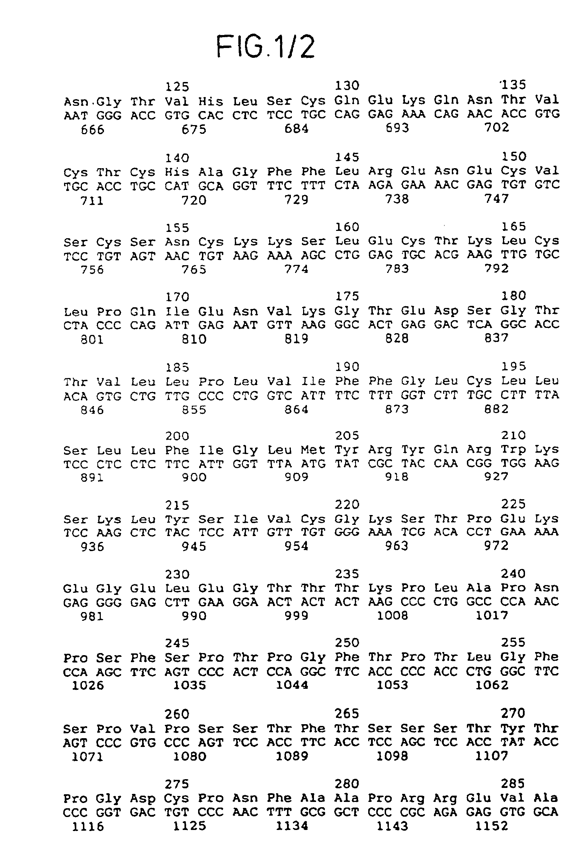

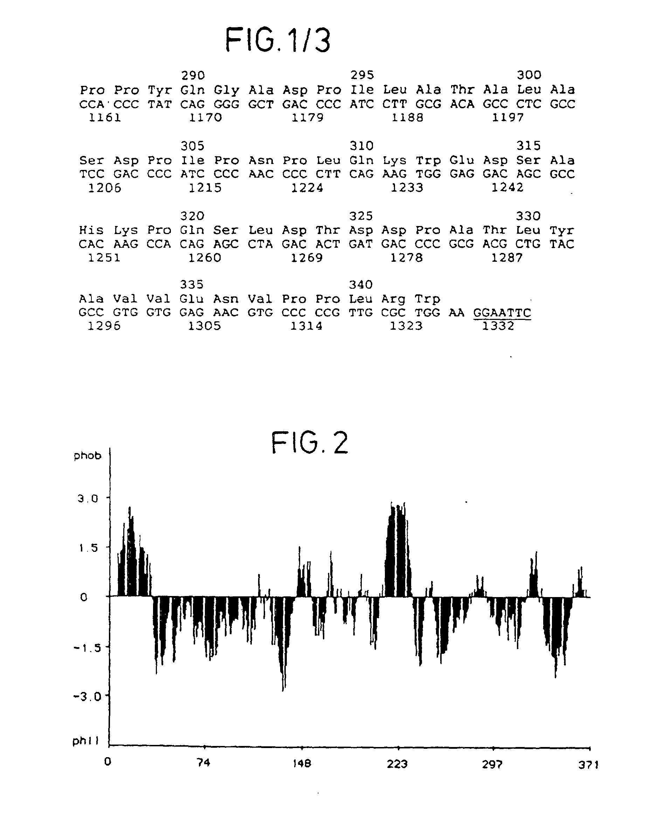

Die vollständige Nukleotidseqenz des längsten erhaltenen cDNA-Inserts ist in Fig.1 abgebildet.

Diese Nukleotidsequenz weist einen durchgehenden offenen Leserahmen auf, beginnend mit Base 213, bis zum Ende des 1334 bp langen cDNA Inserts. Da sich in demselben Leserahmen 4 Codons vor dem potentiellen Translationsstartcodon ATG (213-215) ein Stopcodon (TAG) befindet, wurde davon ausgegangen, daß es sich bei dem Startcodon tatsächlich um den in vivo verwendeten Translationsstart handelt.

Der Vergleich der von der Nukleotidsequenz abgeleiteten Aminosäuresequenz mit den Aminosäuresequenzen, die vom aminoterminalen Ende von TNF-BP und von tryptischen Peptiden bestimmt worden waren, zeigt hohe Übereinstimmung. Daraus ergibt sich, daß die isolierte cDNA die für das authentische TNF-BP kodierende Sequenz enthält.

Vom N-Terminus ausgehend ist die erste Sequenz, die eine Übereinstimmung mit einer tryptischen Spaltpeptidsequenz zeigt, die Sequenz von Fraktion 12 (Leu-Val-Pro-...), die auch als Nebensequenz bei der Analyse des N-Terminus von TNF-BP erhalten worden war. Dieses N-terminale Leucin entspricht der 30. Aminosäure in der cDNA Sequenz. Da der vorangehende Abschnitt von 29 Aminosäuren einen stark hydrophoben Charakter aufweist und es sich bei TNF-BP um ein sekretiertes Protein handelt, kann gefolgert werden, daß diese 29 Aminosäuren das für den Sekretionsvorgang benötigte Signalpeptid darstellen, das bei der Sekretion abgespalten wird (in Figur 1 mit S1-S29 bezeichnet). Die als Hauptsequenz bei der N-terminalen Analyse von TNF-BP erhaltene Aminosäuresequenz entspricht den Aminosäuren beginnend mit Asp-12 in der cDNA Sequenz. Dieser Asparaginsäurerest folgt direkt dem basischen Dipeptid Lys-Arg. Da nach diesem Dipeptid in vivo sehr viele Proteine proteolytisch gespalten werden, ist anzunehmen, daß TNF-BP mit N-terminalem Asp nicht direkt durch Prozessierung eines Precursors beim Sekretionsvorgang entsteht, sondern daß vom prozessierten Protein die N-terminalen 11 Aminosäuren zu einem späteren Zeitpunkt durch extrazelluläre Proteasen abgespalten werden.Das carboxy-terminale Ende von TNF-BP war mit Ile-Glu-Asn bestimmt wordew (C-terminale Analyse;tryptisches Peptid Fraktion 27: Aminosäuren 159-172, tryptisches Peptid Fraktion 21: Aminosäuren 165-172), wobei Asn der Position 172 in der cDNA Sequenz entspricht.

Potentielle N-Glykosylierungsstellen der allgemeinen Formel Asn-Xaa-Ser/Thr, wobei Xaa jede beliebige Aminosäure außer Prolin sein kann, befinden sich an den Positionen 25-27 (Asn-Asn-Ser), 116-118 (Asn-Cys-Ser) und 122-124 (Asn-Gly-Thr) der TNF-BP cDNA Sequenz. (Daß Asn-25 glykosyliert ist, ergibt sich daraus, daß bei der Sequenzierung des entsprechenden tryptischen Spaltpeptids an dieser Stelle Asn nicht identifiziert werden konnte.)

Die Analyse der Nukleotid- bzw. der davon abgeleiteten Aminosäuresequenz im Zusammenhang mit den durchgeführten proteinchemischen Untersuchungen zeigt, daß es sich bei TNF-BP um ein glykosyliertes Polypeptid mit 172 Aminosäuren, das durch proteolytische Spaltung nach der 11. Aminosäure in ein Glykoprotein mit 161 Aminosäuren umgewandelt wird, handelt.
Die nachfolgende Tabelle zeigt die sequenzierten tryptischen Peptide und die aus der cDNA Sequenz abgeleiteten korrespondierenden Aminosäurensequenzen:

| Fraktion | Aminosäuren | |
|---|---|---|
| 12 | 1- 8 | |
| 1 | 12- 19 | |
| 8 | 20- 32 | |
| 14/I | 36- 48 | |
| 20 | 36- 53 | |
| 11 | 54- 67 | (Aminosäuren 66-67 waren am Peptid nicht korrekt bestimmt worden) |
| 14/II | 79- 91 | |
| 26 | 133-146 | |
| 5 | 147-158 | |
| 27 | 159-172 | |

Die erhaltene cDNA stellt die Voraussetzung für die Herstellung von rekombinantem TNF-BP dar.

Die zunächst erfindungsgemäß isolierte cDNA enthält, wie bereits erwähnt, nach dem Codon für Asn-172 nicht das Stopcodon, das aufgrund der Analyse des C-Terminus zu erwarten wäre, sondern der offene Leserahmen wird fortgesetzt. Die Region zwischen Val-183 und Met-204 hat einen stark hydrophoben Charakter. Dieser hydrophobe Bereich von 22 Aminosäuren, gefolgt von einem Abschnitt mit einem Gehalt an positiv geladenen Aminosäuren (Arg-206, Arg-209) weist die typischen Merkmale einer Transmembrandomäne auf, die Proteine in der Zellmembran verankert. Der in Richtung C-Terminus folgende Proteinanteil ist dagegen wieder stark hydrophil.

Das Hydrophobizitätsprofil ist in Fig.2 abgebildet (der Hydrophobizitätsplot wurde mit Hilfe des Mac Molly Programms (Fa.Soft Gene Berlin) erstellt; die Fensterweite für die Berechnung der Werte betrug 11 Aminosäuren. Hydrophobe Bereiche entsprechen positiven, hydrophile Bereiche negativen Werten auf der Ordinate. Auf der Abszisse ist die Zahl der Aminosäuren, beginnend mit dem Startmethionin S1, dargestellt).

Aus der Proteinstruktur ergibt sich, daß die für das lösliche, sekretierte TNF-BP kodierende DNA Teil einer für ein größeres Protein kodierenden DNA ist: Dieses Protein weist die Merkmale eines in der Zellmembran verankerten Proteins auf, enthält TNF-BP in für eine extrazelluläre Domäne typischen Weise und weist einen beträchtlichen für zytoplasmatische Domänen typischen Abschnitt auf. Lösliches TNF-BP wird offensichtlich von dieser membrangebundenen Form durch proteolytische Spaltung knapp außerhalb der Transmembrandomäne erhalten.

Die Struktur des von der erhaltenen cDNA kodierten Proteins im Zusammenhang mit der Fähigkeit von TNF-BP, TNF zu binden, bestätigen die Annahme, daß es sich bei TNF-BP um einen Teil eines zellulären Oberflächenrezeptors für TNF handelt, dessen extrazelluläre, für die Bindung von TNF verantwortliche Domäne proteolytisch abgespalten werden kann und in Form des löslichen TNF-BP wiedergefunden wird. (Dabei soll nicht ausgeschlossen werden, daß im Hinblick auf die Funktionsfähigkeit des Rezeptors dieses Protein gegebenenfalls mit einem oder mehreren anderen Proteinen assoziiert ist).

Für Zwecke der Produktion von TNF-BP in größerem Maßstab wird vorteilhafterweise nicht von der gesamten cDNA ausgegangen, weil das Erfordernis der Abspaltung von TNF-BP von dem Teil des Proteins, das den membrangebundenen Teil des TNF-Rezeptors darstellt, berücksichtigt werden müßte. Es wird vielmehr, wie bereits angeführt, zweckmäßigerweise nach dem Codon für Asn-172 durch gerichtete Mutagenese ein Translationsstopcodon eingeführt, um eine über das C-terminale Ende von TNF-BP hinausgehende Proteinsynthese zu verhindern.

Mit der zunächst erfindungsgemäß erhaltenen cDNA, die eine Teilsequenz der für einen TNF-Rezeptor kodierenden DNA darstellt, kann man zur vollständigen Rezeptorsequenz gelangen, indem man z.B. mittels modifizierter PCR (RACE = "rapid amplification of cDNA ends" (Frohman et al., 1988)) das fehlende 3'-Ende mit Hilfe eines Primers, der aufgrund einer möglichst weit in Richtung 3'-Ende der vorhandenen cDNA gelegenen Sequenz konstruiert wurde, amplifiziert. Eine alternative Methode besteht im konventionellen Screenen der cDNA-Bibliothek mit der verfügbaren cDNA bzw. Teilen davon als Sonde.

Erfindungsgemäß wurde zunächst die Ratten-TNF-Rezeptor cDNA isoliert,mit einer Teilsequenz daraus die vollständige humane TNF-Rezeptor cDNA erhalten und zur Expression gebracht.

Gegenstand der Erfindung ist ein humaner TNF-Rezeptor sowie die dafür kodierende DNA. Unter diese Definition fallen auch DNAs, die für C- und/oder N-terminal verkürzte, z.B.prozessierte, oder für modifizierte (z.B. durch Änderungen an proteolytischen Spaltstellen, Glykosylierungsstellen oder bestimmten Domänenbereichen) Formew bzw. für Fragmente, z.B. die verschiedenen Domänen, des TNF-Rezeptors kodieren. Diese DNAs können in Verbindung mit den für die Expression erforderlichen Kontrollsequenzen als Bestandteil rekombinanter DNA-Moleküle, die ebenfalls Gegenstand der vorliegenden Erfindung sind, zur Transformation von prokaryotischen oder eukaryotischen Wirtsorganismen verwendet werden. Dadurch wird einerseits die Voraussetzung geschaffen, den TNF-Rezeptor bzw. Modifikationen oder Fragmente davon in größeren Mengen auf rekombinantem Weg herzustellen, um z.B. die Aufklärung der dreidimensionalen Struktur des Rezeptors zu ermöglichen. Andererseits können mit diesen DNAs höhere eukaryotische Zellen transformiert werden, um Studien über Mechanismen und Dynamik der TNF/Rezeptor-Wechselwirkung, der Signalübertragung bzw. über die diesbezügliche Relevanz der verschiedenen Rezeptordomänen bzw. Abschnitten davon zu erhalten.

Der rekombinante TNF-Rezeptor (bzw. Fragmente oder Modifikationen davon) kann dazu verwendet werden, Substanzen auf ihre Wechselwirkung mit TNF oder dem TNF-Rezeptor bzw. auf ihren Einfluß auf die durch TNF induzierte Signalübertragung zu untersuchen. Derartige Screenings (unter Verwendung von Proteinen/Fragmenten bzw. von entsprechend transformierten höheren eukaryotischen Zellen) schaffen die Voraussetzung für die Identifizierung von Substanzen, die TNF substituieren, seine Bindung an den Rezeptor hemmen bzw. solche, die den Mechanismus der durch TNF ausgelösten Signalübertragung blockieren oder verstärken können.

Eine Möglichkeit für das Auffinden von Agonisten und Antagonisten von TNF bzw. dem TNF-Rezeptor besteht in der Etablierung eines High Capacity Screening. Dabei wird eine geeignete Zellinie, vorzugsweise eine solche, die keinen endogenen humanen TNF-Rezeptor exprimiert, mit einem Vektor transformiert, der die für einen funktionellen TNF-Rezeptor kodierende, gegenüber der natürlichen Sequenz gegebenenfalls modifizierte, DNA enthält. Die Wirkung von Agonisten bzw. Antagonisten kann in einem derartigen Screening untersucht werden, indem die Antwort auf die Wechselwirkung der Substanz mit dem Rezeptor über einen geeigneten Reporter (veränderte Enzymaktivität, z.B. Proteinkinase C, oder Genaktivierung, z.B. Mangan-Superoxiddismutase, NF-κB) verfolgt wird.

Untersuchungen über Mechanismen und Dynamik der TNF/Rezeptor-Wechselwirkung, der Signalübertragung bzw. die diesbezügliche Rolle der Rezeptordomänen können z.B. auch durchgeführt werden, indem DNA-Abschnitte, kodierend für die extrazelluläre Domäne des TNF-Rezeptors (bzw. Teile davon) mit DNA-Abschnitten, kodierend für verschiedene Transmembrandomänen und/oder verschiedene zytoplasmatische Domänen kombiniert und in eukaryotischen Zellen zur Expression gebracht werden. Die dabei erhältlichen hybriden Expressionsprodukte können geeignet sein, aufgrund gegebenenfalls veränderter Eigenschaften für die Signaltransduktion Aufschluß über die diesbezügliche Relevanz der verschiedenen Rezeptordomänen zu geben, womit ein gezieltes Screening erleichtert wird.

Die Verfügbarkeit der für den TNF-Rezeptor kodierenden cDNA bzw. Abschnitten davon stellt die Voraussetzung dar, zur genomischen DNA zu gelangen. Dazu wird unter stringenten Bedingungen eine DNA-Bibliothek gescreent und die erhaltenen Klone darauf untersucht, ob sie zusätzlich zu den kodierenden Regionen die für die Genexpression erforderlichen regulatorischen Sequenzelemente aufweisen (z.B. Überprüfung auf Promotorfunktion durch Fusion mit kodierenden Regionen geeigneter Reportergene). Der Erhalt der genomischen DNA-Sequenz bietet die Möglichkeit, die im nicht für den TNF-Rezeptor kodierenden Bereich, insbesondere in der 5'-flankierenden Region, gelegenen regulatorischen Sequenzen auf eine etwaige Wechselwirkung mit bekannten, die Genexpression modulierenden Substanzen, z.B. Transkriptionsfaktoren, Steroide, zu untersuchen bzw. gegebenenfalls neue Substanzen, die möglicherweise für die Expression dieses Gens spezifische Wirkung haben, aufzufinden. Die Ergebnisse solcher Untersuchungen bieten die Grundlage für den gezielten Einsatz derartiger Substanzen für die Modulation der TNF-Rezeptor-Expression und damit für eine direkte Beeinflussung der Fähigkeit der Zelle zur Interaktion mit TNF. Damit können die spezifische Reaktion mit dem Liganden und die daraus resultierenden Wirkungen unterbunden werden.

Im Rahmen der vorliegenden Erfindung sind auch DNAs mitumfaßt, die für Subtypen des TNF-Rezeptors bzw. seiner löslichen Formen kodieren, welche gegebenenfalls unterschiedliche Eigenschaften gegenüber dem vorliegenden TNF-Rezeptor aufweisen.Dabei handelt es sich um Expressionsprodukte, die aufgrund von alternativem Splicing entstanden sind und geänderte Strukturen in Teilbereichen aufweisen, z.B. Strukturen, die eine Veränderung der Affinität und Spezifität für den Liganden (TNF-α/TNF-β) oder eine Veränderung hinsichtlich Art und Effizienz der Signalübertragung bewirken können.

Mit Hilfe der für den TNF-Rezeptor kodierenden cDNA können Nukleinsäuren erhalten werden, die mit der cDNA oder Abschnitten davon unter Bedingungen niedriger Stringenz hybridisieren und für ein Polypeptid mit der Fähigkeit, TNF zu binden, kodieren oder die für ein solches Polypeptid kodierende Sequenz enthalten.

Gegenstand der Erfindung ist in einem weiteren Aspekt das rekombinante TNF-BP, vorzugsweise in sekretierbarer Form, das den löslichen Teil des erfindungsgemäßen TNF-Rezeptors darstellt sowie die dafür kodierende DNA.

Durch Einbringen eines DNA-Konstruktes, enthaltend die für TNF-BP kodierende Sequenz mit einer für ein Signalpeptid kodierenden Sequenz unter Kontrolle eines geeigneten-Promoters in geeignete Wirtsorganismen, zweckmäßigerweise in eukaryotische, bevorzugt höhere eukaryotische Zellen, kann TNF-BP produziert werden, das in den Zellüberstand sekretiert wird.

Im Falle der Verwendung eines Signalpeptids im Hinblick auf die Sekretion des Proteins wird zweckmäßigerweise die für das Signalpeptid kodierende DNA vor das Codon für Asp-12 gefügt, um ein einheitliches Produkt zu erhalten. Grundsätzlich ist jedes Signalpeptid geeignet, das im entsprechenden Wirtsorganismus die Sekretion des reifen Proteins gewährleistet. Gegebenenfalls kann die Signalsequenz auch vor das für Leu-1 kodierende Triplett gesetzt werden, wobei in diesem Fall erforderlich sein kann, die durch Abspaltung des aus 11 Aminosäuren bestehenden Peptids am N-Terminus entstehende Form von TNF-BP vom nicht oder nicht vollständig prozessierten TNF-BP in einem zusätzlichen Reinigungsschritt zu trennen.

Da die cDNA nach dem Codon für Asn-172, das aufgrund der C-terminalen Analyse den C-Terminus darstellt, kein Stopcodon enthält, wird zweckmäßigerweise im Hinblick auf die Expression von TNF-BP nach dem Codon für Asn 172 durch gerichtete Mutagenese ein Translationsstopcodon eingeführt.

Die für TNF-BP kodierende DNA kann durch Mutation, Transposition, Deletion, Addition oder Verkürzung modifiziert werden, sofern derartig modifizierte DNAs für (Poly)peptide mit der Fähigkeit, TNF zu binden, kodieren. Derartige Modifikationen können z.B. darin bestehen, eine oder mehrere der potentiellen, gegebenenfalls für die biologische Aktivität nicht erforderlichen Glykosylierungsstellen zu verändern, indem z.B. das Asn-Codon durch ein für eine andere Aminosäure kodierendes Triplett ersetzt wird. Unter Berücksichtigung der Erhaltung der biologischen Aktivität können auch Modifikationen vorgenommen werden, die in einer Änderung der Disulfidbrücken (z.B. Verringerung deren Anzahl) resultieren.

Die angeführten DNA-Moleküle stellen somit die Voraussetzung für die Konstruktion rekombinanter DNA-Moleküle dar, die ebenfalls Gegenstand der Erfindung sind. Mit solchen rekombinanten DNA-Molekülen in Form von Expressionsvektoren, enthaltend die für ein Protein mit TNF-BP Aktivität kodierende, gegebenenfalls in geeigneter Weise modifizierte DNA, vorzugsweise mit einer vorgeschalteten Signalsequenz, und die für die Expression des Proteins erforderlichen Kontrollsequenzen, können geeignete Wirtsorganismen transformiert, gezüchtet und das Protein gewonnen werden.

Ebenso wie etwaige Modifikationen der DNA-Sequenz erfolgt die Auswahl von für die Expression geeigneten Wirtsorganismen insbesondere im Hinblick auf die biologische Wirkung des Proteins, TNF zu binden. Darüberhinaus gehen die bei der Herstellung rekombinanter Proteine üblichen Kriterien wie Verträglichkeit mit dem gewählten Vektor, Prozessierungsfähigkeit, Isolierung des Proteins, Expressionscharakteristika, Sicherheits- und Kostenaspekte in die Entscheidung über den Wirtsorganismus ein. Die Wahl eines geeigneten Vektors ergibt sich aus dem für die Transformation vorgesehenen Wirt. Grundsätzlich sind alle Vektoren geeignet, die die erfindungsgemäßen für TNF-BP kodierenden DNAs (bzw. Modifikationen davon) replizieren und exprimieren.

Im Hinblick auf die biologische Aktivität des Proteins ist bei der Expression der für TNF-BP kodierenden DNA vor allem der etwaigen Relevanz der beim natürlichen Protein festgestellten Kriterien Glykosylierung und hoher Anteil an Cysteinresten für die Eigenschaft, TNF zu binden, Rechnung zu tragen. Zweckmäßig werden daher für die Expression Eukaryonten, insbesondere geeignete Expressionssysteme höherer Eukaryonten, verwendet.

Im Rahmen der vorliegenden Erfindung wurden sowohl transiente als auch permanente Expression von TNF-BP in eukaryotischen Zellen nachgewiesen.

Das erfindungsgemäße rekombinante TNF-B ebenso wie geeignete Modifikationen davon, die die Fähigkeit aufweisen, TNF zu binden, können bei der prophylaktischen und therapeutischen Behandlung des menschlichen oder tierischen Körpers bei Indikationen eingesetzt werden, bei denen eine schädigende Wirkung von TNF-α auftritt.

Da beim TNF-BP auch eine TNF-β inhibierende Wirkung nachgewiesen wurde, kann es (bzw. die verwandten bzw. modifizierten Polypeptide) in geeigneter Dosierung, gegebenenfalls in im Hinblick auf eine gesteigerte Affinität zu TNF-β modifizierter Form, auch für die Inhibierung der Wirkung von TNF-β im Organismus verwendet werden.

Gegenstand der Erfindung sind daher weiters pharmazeutische Zubereitungen, enthaltend eine die biologische Wirkung von TNF-α und/oder TNF-β wirksam hemmende Menge von rekombinantem TNF-BP bzw. einem verwandten Polypeptid mit der Fähigkeit, TNF zu binden.

Pharmazeutische Zubereitungen kommen insbesondere für die parenterale Anwendung bei den erwähnten Indikationen, bei denen eine schädigende Wirkung von TNF auftritt, in Betracht, z.B. in Form von Lyophilisaten oder Lösungen. Diese enthalten TNF-BP oder ein therapeutisch wirksames funktionelles Derivat in therapeutisch wirksamer Menge, gegebenenfalls zusammen mit physiologisch verträglichen Zusatzstoffen, wie Stabilisatoren, Puffern, Konservierungsmitteln, etc.

Die Dosierung hängt vor allem von der jeweiligen Indikation und der spezifischen Verabreichungsform, z.B. ob sie lokal oder systemisch erfolgt, ab. Die Größe der Einzeldosen erfolgt gegebenenfalls an Hand der individuellen Beurteilung des Krankheitsfalles unter Einbeziehung von Faktoren wie Allgemeinzustand, Anamnese, Alter, Gewicht, Geschlecht des Patienten, etc. Wesentlich für die Bestimmung der therapeutisch wirksamen Dosis ist die Berücksichtigung der für die Erkrankung verantwortlichen Menge an ausgeschüttetem TNF sowie die Menge an endogenem TNF-BP. Grundsätzlich kann davon ausgegangen werden, daß zur wirksamen Behandlung einer durch TNF ausgelösten Erkrankung für die ausgeschüttete Menge TNF mindestens dieselbe molare Menge an TNF-BP, gegebenfalls ein mehrfacher Überschuß erforderlich ist.

Im einzelnen wurde die gestellte Aufgabe wie folgt gelöst:

Vom hochgereinigten TNF-BP wurden die N-terminale Aminosäuresequenz sowie die Aminosäuresequenzen von durch tryptischen Verdau des Proteins erhaltenen Peptiden bestimmt.

Weiter wurde der C-Terminus durch Carboxypeptidase P Verdau, Derivatisierung der abgespaltenen Aminosäuren und chromatographische Auftrennung bestimmt.

Aus den durch tryptischen Verdau erhaltenen Peptidsequenzen wurden im Hinblick auf ihren Einsatz in der PCR für die Herstellung von Oligonukleotiden Bereiche einerseits aus dem N-Terminus und andererseits aus einem tryptischen Peptid derart ausgewählt, daß die Komplexität von Mischoligonukleotiden für die Hybridisierung mit cDNA möglichst gering ist. Auf Basis dieser beiden Bereiche wurde je ein Satz Mischoligonukleotide hergestellt, wobei der vom N-terminal gelegenen Bereich abgeleitete entsprechend der mRNA und der vom tryptischen Peptid abgeleitete revers komplementär zur mRNA synthetisiert wurde. Um das nachfolgende Klonieren eines mit PCR amplifizierten Segments zu erleichtern, wurde der vom tryptischen Peptid abgeleitete Satz von Oligonukleotiden mit einer BamHI-Restriktionsstelle versehen. Darauf wurde λ DNA aus der TNF-α induzierten Fibrosarkom cDNA-Bibliothek isoliert und daraus eine TNF-BP-Sequenz mittels PCR amplifiziert. Das erhaltene Fragment wurde kloniert und sequenziert; es weist 158 Nukleotide auf und enthält zwischen den beiden von den Primer Oligonukleotiden stammenden Sequenzabschnitten die für das tryptische Peptid 20 kodierende Sequenz.

Dieses DNA-Fragment wurde nachfolgend radioaktiv markiert und als Sonde zur Isolierung von cDNA-Klonen aus der Fibrosarkom-Bibliothek verwendet. Es wurde dazu so verfahren, daß zunächst Plaques mit der Sonde hybridisiert, Phagen von hybridisierenden Plaques vereinzelt und daraus λ DNA gewonnen wurden. Einzelne cDNA-Klone wurden subkloniert und sequenziert; zwei der charakterisierten Klone enthielten die für TNF-BP kodierende Sequenz.

Diese Sequenz stellt einen Teil der für einen TNF-Rezeptor kodierenden Sequenz dar.

Nach Verkürzen der 5'-nicht kodierenden Region und Einführen eines Stop-Codons nach dem Codon für die C-terminale Aminosäure des natürlichen TNF-BP wurde die cDNA in eih geeignetes Expressionsplasmid insertiert, damit eukaryotische Zellen transformiert und die Expression von TNF-BP mittels ELISA nachgewiesen.

Die noch fehlende 3'-Region des TNF-Rezeptors wurde erhalten, indem eine Rattenhirn cDNA-Bibliothek aus der Ratten Glia Tumorzellinie C6 mit einer TNF-BP Sonde durchsucht und die vollständige für den Ratten-TNF-Rezeptor kodierende cDNA isoliert wurde.

Der 3'gelegene Abschnitt dieser cDNA, von dem angenommen wurde, daß er der fehlenden 3'-Region hinter der EcoRI Schnittstelle des humanen TNF-Rezeptors entspricht, wurde als Sonde zum nochmaligen Durchsuchen der HS913T cDNA-Bibliothek verwendet. Dabei wurde ein Klon erhalten, der die gesamte für den TNF-Rezeptor kodierende DNA enthält.

Nach Verkürzen der 5'-nicht kodierenden Region wurde die cDNA in ein Expressionsplasmid insertiert und die Expression von humanem TNF-Rezeptor in eukaryotischen Zellen über die Bindung von radioaktiv markiertem TNF nachgewiesen.

Mittels Northern Blot-Analyse wurde bestätigt, daß die isolierte cDNA nahezu der gesamten TNF-R mRNA entspricht (die geringfügige Diskrepanz ergibt sich aus dem Fehlen eines Teils der 5'-nichtkodierenden Region). Daraus kann gefolgert werden, daß es sich beim exprimierten Protein um den vollständigen TNF-Rezeptor handelt.

Die Erfindung wird an Hand der folgenden Vorversuche und Beispiele illustriert.

### Vorversuch 1

### Herstellung von hochgereinigtem TNF-BP

### a) Konzentration des Harns

200 l Dialyseharn von Urämiepatienten, aufbewahrt in Flaschen enthaltend EDTA (10 g/l), Tris (6 g/l), NaN3 (1 g/l) und Benzamidinhydrochlorid (1 g/l) sowie kühlgelagert, wurden durch Ultrafiltration mittels einem hochdurchlässigen Hämokapillarfilter mit einer asymmetrischen Hohlfasermembran (FH 88H, Gambro) auf 4,2 1 mit einem Proteingehalt von 567 g konzentriert. Der konzentrierte Harn wurde gegen 10mM/l Tris HCl, pH 8 dialysiert. Während dieses Vorgangs wurde, wie in den nachfolgenden Schritten (außer bei der Reverse Phase Chromatographie), 1mM/l Benzamidinhydrochlorid zugefügt, um proteolytischem Verdau entgegenzuwirken. Alle nachfolgenden Reinigungsschritte wurden, wenn nicht anders angegeben, bei 4 °C durchgeführt.

### b) Ionenaustauschchromatographie

Dieser Schritt wurde durchgeführt, indem DEAE-Sephacel-Säulen (2,5 x 40 cm) mit Proben konzentrierten und dialysierten Harns, enthaltend je ca. 75 g Protein, beschickt wurden. Eluiert wurde mit 800 ml eines NaCl /10mM Tris/HCl pH-8-Gradienten, wobei die NaCl Konzentration 0 bis 0,4 M betrug. Die das TNF-BP enthaltenden Fraktionen von sieben Säulen mit einem Gesamtproteingehalt von 114 g wurden bei -20 °C gelagert.

### c) Affinitätschromatographie

Zur Herstellung der TNF-Sepharosesäule wurde rTNF-α (15 mg) in 0,1 M NaHCO₃, 1 M NaCl, pH 9 (Kopplungspuffer) an 1,5 g cyanogenbromidaktivierte Sepharose 4B (Pharmacia) gekoppelt. Die Sepharose wurde in 1mM HCl gequollen und mit Kopplungspuffer gewaschen. Nach Zusatz von rTNF-α wurde die Suspension 2 Stunden lang bei Raumtemperatur rotieren gelassen. Der Überschuß an CNBr-Gruppen wurde durch eineinhalbstündige Rotation mit 1M Ethanolamin, pH 8 blockiert. Die TNF-Sepharose wurde einige Male abwechselnd in 1M NaCl, 0,1 M Natriumacetat pH 8 und 1 M NaCl, 0,1 M Borsäure pH 4 gewaschen und anschließend in phosphatgepufferter Kochsalzlösung mit 1mM Benzamidinhydrochlorid gelagert. Die aus Schritt b) erhaltenen Fraktionen wurden auf eine Konzentration von 0,2 M NaCl, 10mM Tris/HCl, pH 8 eingestellt. Die TNF-Sepharose wurde in eine Säule gepackt und mit 0,2 M NaCl, 10mM Tris HCl, pH 8 gewaschen und die TNF-BP enthaltenden Fraktionen, entsprechend ca. 30 g Protein, bei einer Durchflußrate von 10 ml/h aufgetragen und ausgiebig mit 0,2 M NaCl, 10mM Tris HCl, pH 8 gewaschen, bis im Eluat bei 280nm keine Absorption mehr nachweisbar war. Anschließend wurde TNF-BP mit 0,2 M Glycin/HCl, pH 2,5 eluiert.

TNF-BP enthaltende Fraktionen aus 4 Auftrennungen wurden vereinigt und nach Zusatz von Polyethylenglykol (MG 6000) - bis zu einer Endkonzentration von 10 mg/ml - lyophilisiert. Die lyophilisierte Probe wurde in destilliertem Wasser gelöst und gegen destilliertes Wasser dialysiert. (Die dialysierte Probe (4 ml) wurde in tiefgefrorenem Zustand gelagert.)

Dieser Reinigungsschritt brachte gegenüber dem vorangegangenen eine weitere Anreicherung um das ca. 9000 fache. SDS-PAGE (durchgeführt, wie in Vorversuch 2 beschrieben) der TNF-BP enthaltenden Fraktionen zeigte die Elution von drei Hauptkomponenten mit Molekulargewichten von 28 000, 30 000 und 50 000 .

### d) Reverse Phase Chromatographie

Ein aliquoter Anteil (1 ml) der aus Schritt c) erhaltenen Fraktionen mit einem Zusatz von 0,1 % Trifluoressigsäure wurde auf eine ProRPC HR 5/10 Säule (Pharmacia), die an ein FPLC-System (Pharmacia) angeschlossen war, aufgetragen. Die Säule wurde mit 0,1 %iger Trifluoressigsäure equilibriert und bei Raumtemperatur mit einem linearen 15 ml Gradienten von 10 Vol% bis 50 Vol% Acetonitril, enthaltend 0,1 % Trifluoressigsäure, beschickt; die Durchflußrate betrug 0,3 ml/min. Fraktionen von 0,5 ml wurden gesammelt und die Absorption bei 280nm sowie die Aktivität des TNF-α bindenden Proteins mit Hilfe des Kompetitionsbindungstest, wie im Beispiel 1 angegeben,bestimmt, wobei jeweils 0,01 µl Probe verwendet wurden. TNF-BP eluierte als ein einziger Aktivitätspeak entsprechend einem scharfen UV-Absorptionspeak.
Dieser letzte Reinigungsschritt brachte eine Zunahme der spezifischen Aktivität um das ca. 29 fache, die Gesamtzunahme an Aktivität gegenüber dem Ausgangsmaterial (konzentrierter Dialyseharn) betrug das ca. 1,1 x 10⁶ fache.

SDS-PAGE der reduzierten und nicht reduzierten Probe, durchgeführt wie in Vorversuch 2 angegeben, ergab eine diffuse Bande, die auf das Vorhandensein eines einzigen Polypeptids mit einem Molekulargewicht von ca. 30 000 hinwies. Das diffuse Erscheinungsbild der Bande kann auf das Vorliegen einer oder mehrerer heterogener Glykosylierungen und/oder eines zweiten, in geringer Menge vorhandenen Polypeptids zurückzuführen sein. Die Annahme, dabei könnte es sich um ein Polypeptid mit dem in Vorversuch 3d als Nebensequenz bestimmten N-Terminus handeln, das gegenüber TNF-BP am N-Terminus verlängert ist, wurde durch die Sequenz der cDNA bestätigt, wonach zwischen der Signalsequenz und Asn (Pos.12) ein Abschnitt von 11 Aminosäuren vorliegt, dessen Sequenz mit der N-terminalen Nebensequenz übereinstimmt und der offensichtlich vom prozessierten Protein abgespalten wird.

### Vorversuch 2

### SDS-Polyacrylamidgelelektrophorese (SDS-PAGE)

SDS-PAGE wurde nach der Methode von Laemmli (Laemmli, 1970) auf 18 cm langen, 16 cm breiten und 1,5 mm dicken Flachgelen mit 10 Taschen mittels einer LKB 2001 Elektrophorese-Einheit durchgeführt. Der Proteingehalt der Proben aus den Reinigungsschritten c) und d) (Vorversuch 1) wurde mittels Bio-Rad Protein Assay bestimmt bzw. aus der Absorption bei 280 nm berechnet, wobei einer Absorption von 1,0 ein Gehalt von 1 mg TNF-BP/ml zugeordnet wurde.

Die Proben, enthaltend ca. 25 µg Protein (aus Vorversuch 1c) bzw. ca. 5 µg (aus 1d) in reduzierter (β-Mercaptoethanol) und nicht reduzierter Form wurden auf ein 3%iges Sammelgel und ein 5 bis 20%iges lineares Polyacrylamidgradientengel aufgetragen. Die Elektrophorese wurde bei 25mA/Gel ohne Kühlung gefahren. Als Molekulargewichtsmarker (Pharmacia) wurden Phosphorylase B (MG 94 000), Rinderserumalbumin (MG 67 000), Ovalbumin (MG 43 000), Karboanhydrase (MG 30 000), Sojabohnen-Trypsininhibitor (MG 20 100) und a-Laktalbumin (MG 14 400) verwendet. Die Gele wurden mit Coomassie Blue in 7%iger Essigsäure/40%igem Ethanol gefärbt und in 7%iger Essigsäure/25%igem Ethanol entfärbt.

Das Ergebnis der SDS-PAGE zeigte TNF-BP als Polypeptidkette mit einem Molekulargewicht von ca. 30.000 .

### Vorversuch 3

### a) Probenvorbereitung

15 µg des nach Vorversuch 1d) gereinigten Proteins wurden über Reverse Phase HPLC entsalzt und weiter gereinigt. Dazu wurden eine Bakerbond WP C18 Säule (Baker; 4,6 x 250 mm) und 0,1 %ige Trifluoressigsäure in Wasser (Eluens A) bzw. in Acetonitril (Eluens B) als mobile Phase verwendet. Die Gradientensteigerung betrug 20 bis 68 % Eluens B in 24 min. Die Detektion erfolgte parallel bei 214 nm und bei 280 nm. Die TNF-BP enthaltende Fraktion wurde gesammelt, getrocknet und in 75 µl 70 %iger Ameisensäure gelöst und direkt für die Aminosäuresequenzanalyse verwendet.

### b) Aminosäuresequenzanalyse

Die automatische Aminosäuresequenzanalyse wurde mit einem Applied Biosystems 477 A Flüssigphasensequenator durch On-line Bestimmung der freigesetzten Phenylthiohydantoin-Derivate mittels Applied Biosystems Analysator, Modell 120 A PTH, durchgeführt. Sie ergab die folgende N-terminale Sequenz als Hauptsequenz
(ca. 80 % der Proteinmenge): Asp-Ser-Val-Xaa-Pro-Gln-Gly-Lys-Tyr-Ile-His-Pro-Gln-.
Daneben war folgende Nebensequenz nachzuweisen: Leu-(Val)-(Pro)-(His)-Leu-Gly-Xaa-Arg-Glu-. (Die in Klammer stehenden Aminosäuren konnten nicht eindeutig identifiziert werden.)

### Vorversuch 4

### SDS-PAGE

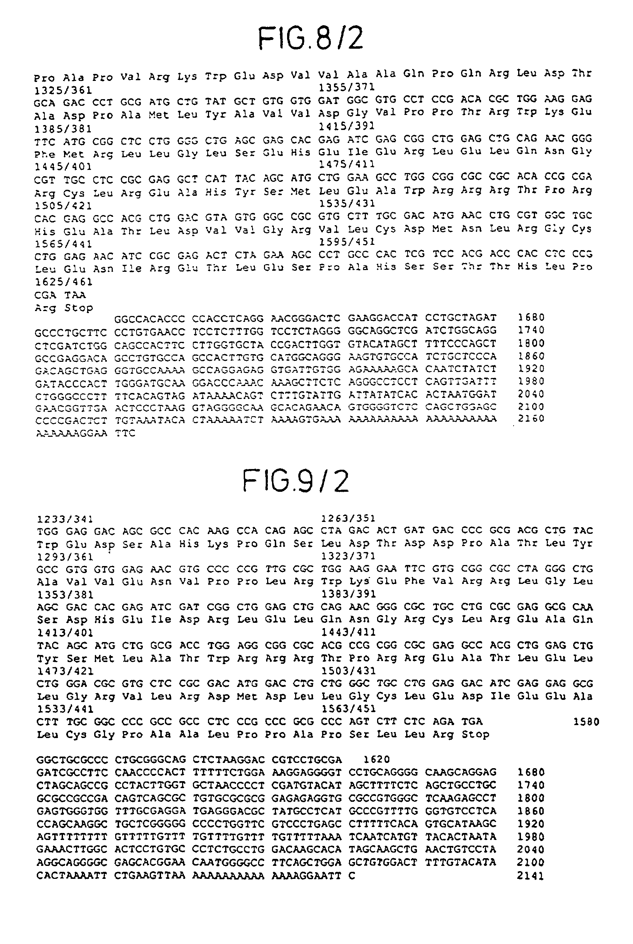

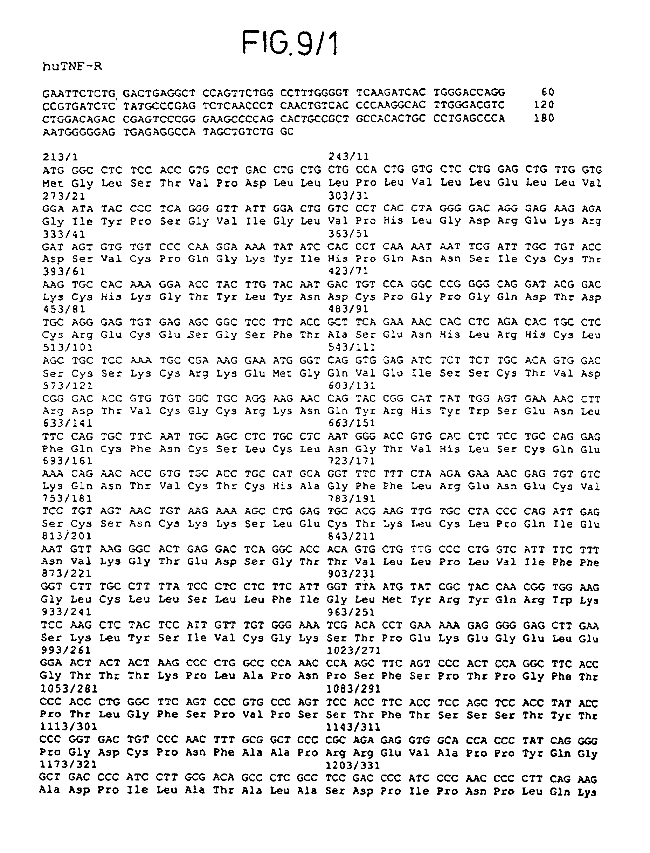

Die Probenvorbereitung wurde wie im Vorversuch 3 durchgeführt mit dem Unterschied, daß die Probenmenge 10 µg betrug. Die Probe wurde in 50 µl Wasser aufgenommen und in 4 Portionen geteilt. Einer der vier aliquoten Teile wurde zur Reinheitsbestimmung mittels SDS-PAGE nach der Methode von Laemmli (24) mit DTT (Dithiothreitol) reduziert und auf Minigelen (Höfer, 55x80x0,75 mm, 15 %) getrennt; als Molekulargewichtsmarker wurde der im Vorversuch 8 angegebene verwendet. Die Färbung erfolgte nach der Methode von Oakley (Oakley, et al., 1986). Das Elektropherogramm ist in Fig. 9 dargestellt. Es zeigt eine einzige Bande bei einem Molekulargewicht von ca. 30 000.

### Beispiel 1

### a) Tryptic Peptide Mapping

Etwa 60 µg des nach Vorversuch 1d) gereinigten Proteins wurden über Reverse Phase HPLC entsalzt und damit weiter gereinigt. Dazu wurden eine Bakerbond WP C18 Säule (Baker; 4,6 x 250 mm) und 0,1%ige Trifluoressigsäure in Wasser (Eluens A) bzw. in Acetonitril (Eluens B) als mobile Phase verwendet. Die Gradientensteigerung betrug 20 bis 68% Eluens B in 24 min. Die Detektion erfolgte parallel bei 214 nm und bei 280 nm. Die TNF-BP enthaltende Fraktion (Retentionszeit etwa 13,0 min) wurde gesammelt, getrocknet und in 60 µl 1%igem Ammoniumbicarbonat gelöst.

Dieser Lösung wurden 1% w/w, entsprechend 0,6 µg Trypsin (Boehringer Mannheim) zugesetzt und die Reaktionsmischung 6 Stunden bei 37°C inkubiert. Danach wurden nochmals 1% w/w Trypsin zugesetzt und die Inkubation über Nacht fortgesetzt.

Zur Reduktion der Disulfidbrücken wurde der Reaktionsansatz anschließend mit 60 µl 6 M Harnstoff und mit 12 µl 0,5 M Dithiothreitol versetzt und 2 Stunden bei Raumtemperatur stehengelassen.

Die Auftrennung der entstandenen tryptischen Spaltpeptide erfolgte über Reverse Phase HPLC, wobei eine Delta Pak C18 Säule (Waters, 3,9 x 150 mm, 5 µm Teilchendurchmesser, 100 Å Porendurchmesser) bei 30°C und 0,1%ige Trifluoressigsäure in Wasser (Eluens A) bzw. in Acetonitril (Eluens B) als mobile Phase verwendet wurden. Die Gradientensteigerung betrug 0 bis 55% Eluens B in 55 min, danach wurde 55% B für 15 min beibehalten. Die Flußrate betrug 1 ml/min, die Detektion erfolgte parallel bei 214 nm (0,5 AUFS) und bei 280 nm (0,05 AUFS).

### b) Sequenzanalyse von tryptischen Peptiden

Einige der nach a) gewonnenen tryptischen Spaltpeptide von TNF-BP wurden der automatischen Aminosäuresequenzanalyse unterworfen. Dazu wurden die entsprechenden Fraktionen aus der Reverse Phase HPLC gesammelt, getrocknet und in 75 µl 70%iger Ameisensäure gelöst. Diese Lösungen wurden direkt für die Sequenzierung in einem Applied Biosystems 477 A Pulsed Liquid Phase Sequenator eingesetzt. Tab.1 enthält die Ergebnisse der Sequenzanalyse der tryptischen Peptide, wobei die in Klammern angeführten Aminosäuren nicht mit Sicherheit nachgewiesen werden konnten. Die Angabe "Xaa" bedeutet, daß an dieser Stelle die Aminosäure nicht identifiziert werden konnte.
In der Fraktion 8 konnte die Aminosäure in Position 6 nicht identifiziert werden. Die Sequenz -Xaa-Asn-Serfür die Position 6-8 läßt vermuten, daß die Aminosäure 6 in glykosylierter Form vorliegt.
In der Fraktion 17 konnte die Aminosäure in Position 6 ebenfalls nicht identifiziert werden. Die Sequenz -Xaa-Asn-Ser- (bereits in Fraktion 8 auftretend) für die Positionen 6 bis 8 läßt vermuten, daß die Aminosäure 6 in glykosylierter Form vorliegt. Die ersten 13 Aminosäuren der Fraktion 17 sind weitgehend identisch mit der Fraktion 8; bei Fraktion 17 dürfte es sich somit um ein Peptid handeln, das durch unvollständige tryptische Spaltung entstanden ist.

Auffallend ist die Identität der Fraktion 21 mit den Positionen 7 bis 14 der Fraktion 27. Sowohl in Fraktion 21 als auch in Fraktion 27 bricht die Sequenz nach der Aminosäure Asparagin (Position 8 bzw. 14) plötzlich ab, obwohl hier keine tryptische Spaltung zu erwarten ist. Dies ist ein Hinweis darauf, daß die Aminosäure Asparagin (Position 8 in Fraktion 21 bzw. Position 14 in Fraktion 27) die C-terminale Aminosäure von TNF-BP sein könnte.

Auffallend ist die weitgehende Identität der Sequenz der nur in geringer Menge auftretenden Fraktion 12 mit der in Vorversuch 10 bestimmten Nebensequenz des N-Terminus. Daß die Proteine der Haupt- und Nebensequenz auf einer analytischen Reverse Phase HPLC-Säule (Vorversuch 3b) nicht trennbar waren, lieferte einen Hinweis dafür, daß es sich bei dem Protein mit der Nebensequenz um eine am N-Terminus verlängerte Form des TNF-BP handelt, die durch Prozessierung zum Großteil in das Protein mit der Hauptsequenz überführt wird.

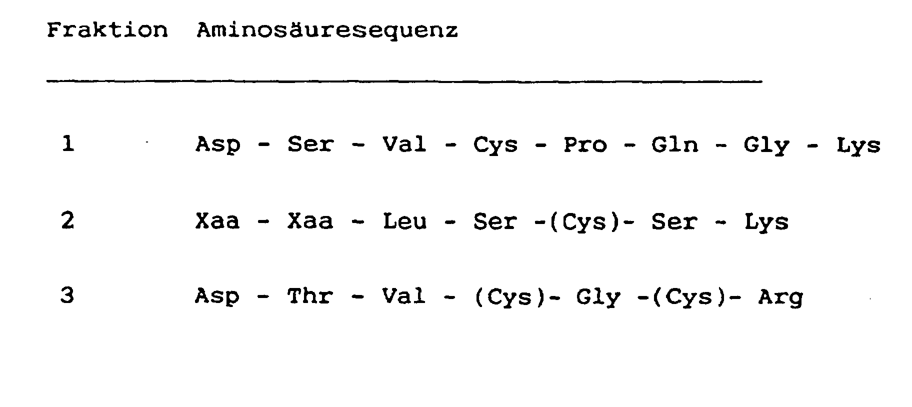

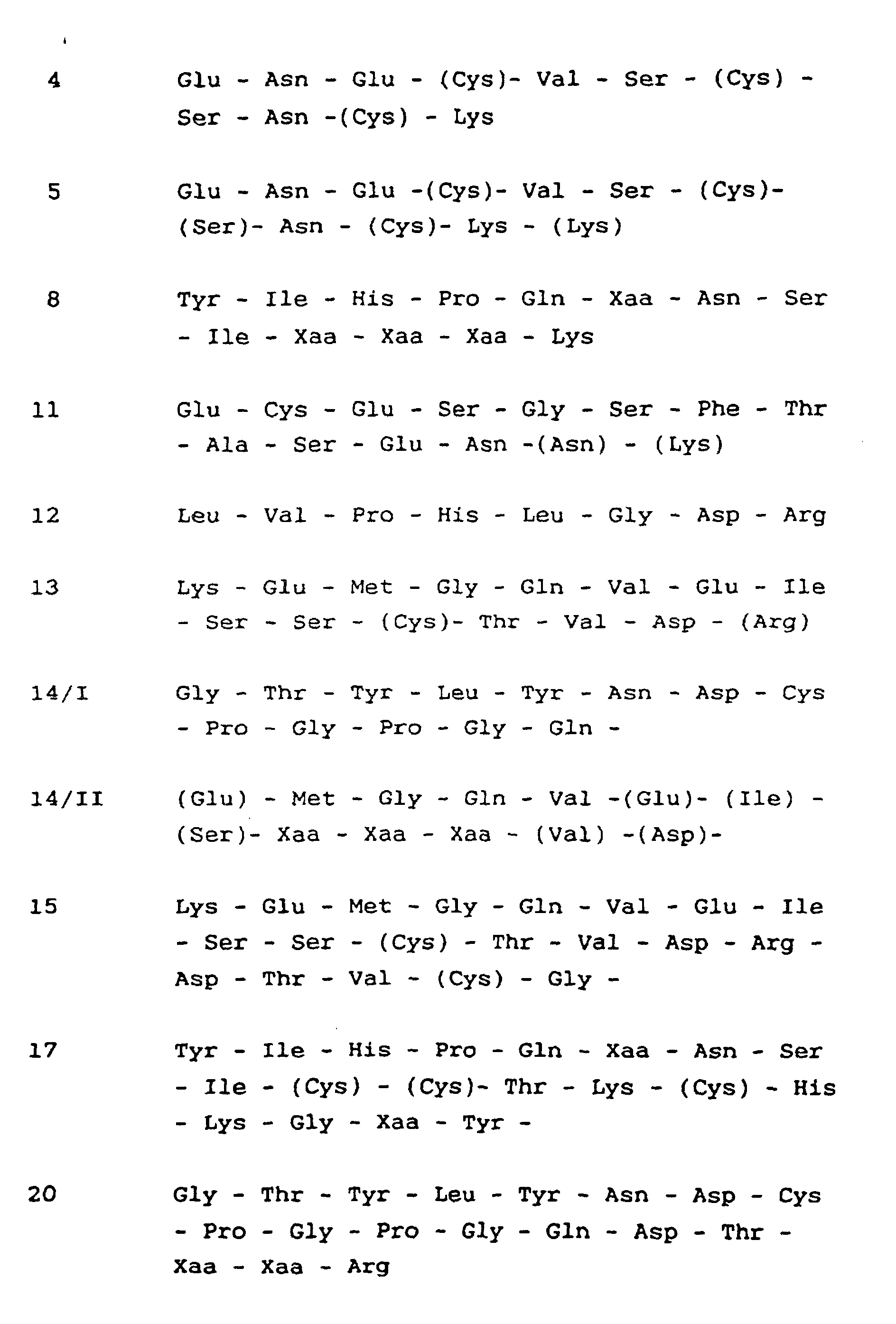

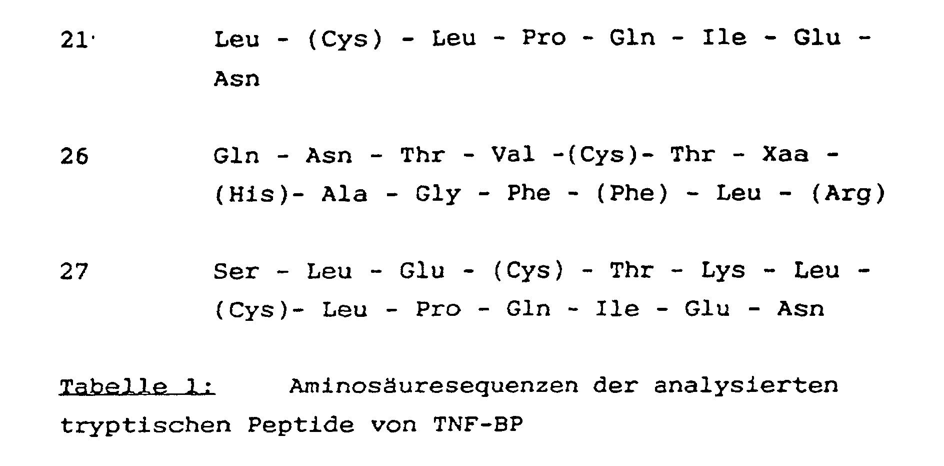

### Beispiel 2

### Analyse des C-Terminus

Diese Analyse wurde nach dem Prinzip der in (Hsieng et al., 1988) beschriebenen Methode durchgeführt.

Etwa 60 pg des nach Vorversuch 2d) gereinigten Proteins wurden über Reverse Phase HPLC entsalzt und damit weiter gereinigt. Dazu wurden eine Bakerbond WP C18 Säule (Baker; 4,6 x 250 mm) und 0,1%ige Trifluoressigsäure in Wasser (Eluens A) bzw. in Acetonitril (Eluens B) als mobile Phase verwendet. Die Gradientensteigerung betrug 20 bis 68% Eluens B in 24 min. Die Detektion erfolgte parallel bei 214 nm und bei 280 nm. Die TNF-BP enthaltende Fraktion (Retentionszeit etwa 13,0 min) wurde gesammelt, getrocknet und in 120 µl 10 mM Natriumacetat (auf pH 4 gestellt mit 1 N HCl) gelöst.

Dieser Lösung wurden 6 µl Brij 35 (10 mg/ml in Wasser) sowie 1,5 µl Carboxypeptidase P (0,1 mg/ml in Wasser, Boehringer Mannheim, Nr. 810142) zugesetzt. Das entspricht einem Gewichtsverhältnis Enzym zu Protein von 1 zu 400 (Frohman et al., 1988).

Sofort nach Zusatz des Enzyms wurde eine Probe von 20 µl der Reaktionsmischung entnommen und darin die enzymatische Reaktion durch Ansäuern mit 2 µl konzentrierter Trifluoressigsäure und durch Gefrieren bei - 20°C unterbrochen.

Die Reaktionsmischung wurde im Kühlschrank (ca. 8°C) stehengelassen und Proben zu je 20 µl nach 10, 20, 60 und 120 Minuten entnommen. Der Rest der Reaktionsmischung wurde weitere 120 Minuten bei Raumtemperatur belassen. Alle Proben wurden sofort nach der Entnahme durch Zusatz von 2 µl konzentrierter Trifluoressigsäure angesäuert und bei -20°C eingefroren, wodurch die enzymatische Reaktion unterbrochen wurde.

Parallel zum beschriebenen Probenansatz mit etwa 60 µg TNF-BP wurde unter identischen Bedingungen ein Reagentienblindwert angesetzt, dem kein Protein zugesetzt worden war.

Nach der letzten Probennahme wurden alle Proben 30 Minuten lang in einem Speed Vac Concentrator getrocknet, mit 10 µl einer Lösung aus 2 Teilen Äthanol, 2 Teilen Wasser und 1 Teil Triäthylamin (= "Redrying solution" des Picotag-Aminosäureanalysesystems der Fa. Waters) versetzt und nochmals kurz getrocknet. Danach wurden die Proben zur Derivatisierung der vom C-Terminus abgespalteten Aminosäuren mit je 20 µl des "Derivatisation Reagens" (7:1:1:1 = Äthanol : Wasser : Triäthylamin : Phenylisothiocyanat; Picotag-System) versetzt, 20 Minuten bei Raumtemperatur stehen gelassen und dann 1 Stunde in einem Speed Vac Concentrator getrocknet.

Zur Analyse der derivatisierten Aminosäuren wurden die Proben in 100 µl "Sample Diluent" (Picotag-System der Fa.Waters) gelöst. Von diesen Lösungen wurden je 50 µl mit Reverse Phase HPLC (Säule, mobile Phase und Gradient nach Originalvorschrift des Picotag-Systems der Fa.Waters) analysiert. Die Chromatogramme der Proben und Reagentienblindwerte wurden mit dem Chromatogramm eines analog derivatisierten Gemisches (100 pMol/Aminosäure) von Standardaminosäuren (Fa.Beckman) verglichen.

Wie aus den quantitativen Ergebnissen der Picotag-Aminosäureanalyse (Tabelle 2) ersichtlich ist, ist Asparagin mit hoher Wahrscheinlichkeit die C-terminale Aminosäure von TNF-BP. Neben Asparagin konnten nach 240 Minuten Reaktionszeit auch Glutaminsäure und in geringerer Menge Isoleucin nachgewiesen werden. Signifikant über dem Reagentienblindwert liegende Mengen von anderen Aminosäuren konnten auch nach 240 Minuten Reaktionszeit nicht gefunden werden. Dieses Ergebnis (-Ile-Glu-Asn als C-Terminus) bestätigt die aus der N-terminalen Sequenzierung der tryptischen Peptide 21 und 27 abgeleitete Vermutung, daß die bei diesen Peptiden C-terminal identifizierten Aminosäuren - Ile-Glue-Asn (Beispiel 1b) den C-Terminus von TNF-BP darstellen.

**Tabelle 2:**

| Quantitative Auswertung der Picotag-Aminosäureanalyse nach Reaktion von Carboxypeptidase P mit TNF-BP | | | |
|---|---|---|---|
| Reaktionszeit | | Integratoreinheiten für die Aminosäuren | |
| Isoleucin | | Glutaminsäure | Aspargin |
| 0 | - | - | - |
| 10 | - | - | - |
| 20 | - | - | 83.304 |
| 60 | - | - | 168.250 |
| 120 | - | - | 319.470 |
| 240 | 85.537 | 152.350 | 416.570 |

### Methoden zu den Beispielen 3 bis 7:

In den nachfolgenden Beispielen wurden, sofern nicht ausdrücklich anders angegeben, molekularbiologische Standardmethoden verwendet, die einschlägigen Handbüchern entnommen werden können bzw. den von den Herstellern empfohlenen Bedingungen entsprechen. Um die Beschreibung der nachfolgenden Beispiele zu vereinfachen, werden oft wiederkehrende Methoden bzw. Bezeichnungen kurz beschrieben:

"Schneiden" oder "Verdauen" von DNA bezieht sich auf die katalytische Spaltung der DNA mittels Restriktionsendonukleasen (Restriktionsenzymen) an für diese spezifischen Stellen (Restriktionsstellen). Restriktionsendonukleasen sind käuflich erhältlich und werden unter den von den Herstellern empfohlenen Bedingungen (Puffer, Rinderserumalbumin (BSA) als Trägerprotein, Dithiothreit (DTT) als Oxidationsschutz) eingesetzt. Restriktionsendonukleasen werden mit einem Großbuchstaben, meist gefolgt von Kleinbuchstaben und normalerweise einer römischen Ziffer, bezeichnet. Die Buchstaben hängen von dem Mikroorganismus ab, aus dem die betreffende Restriktionsendonuklease isoliert wurde (z.B.: Sma I: Serratia marcescens). Üblicherweise wird etwa 1 µg DNA mit einer oder mehreren Einheiten des Enzyms in etwa 20 µl Pufferlösung geschnitten. Normalerweise wird eine Inkubationsdauer von 1 Stunde bei 37°C verwendet, kann aber laut den Verwendungsvorschriften des Herstellers variiert werden. Nach dem Schneiden wird manchmal die 5'Phosphatgruppe durch Inkubation mit alkalischer Phosphatase aus Kalbsdarm (CIP) entfernt. Dies dient zur Verhinderung einer ungewünschten Reaktion der spezifischen Stelle in einer nachfolgenden Ligasereaktion (z.B. Zirkularisierung eines linearisierten Plasmids ohne Insertierung eines zweiten DNA-Fragmentes). Wenn nicht anders angegeben, werden DNA-Fragmente nach dem Schneiden mit Restriktionsendonukleasen normalerweise nicht dephosphoryliert. Reaktionsbedingungen für die Inkubation mit alkalischer Phosphatase sind z.B. dem M13 Cloning und Sequencing Handbuch (Cloning and Sequencing Handbook, Fa Amersham, PI/129/83/12) zu entnehmen.Nach der Inkubation wird Protein durch Extraktion mit Phenol und Chloroform entfernt und die DNA aus der wäßrigen Phase durch Zusatz von Äthanol präzipitiert.

"Isolierung" eines bestimmten DNA Fragments bedeutet die Auftrennung der durch den Restriktionsverdau erhaltenen DNA-Fragmente, z.B. auf einem 1% Agarosegel. Nach der Elektrophorese und dem Sichtbarmachen der DNA im UV-Licht durch Anfärben mit Äthidiumbromid (EtBr) wird das gewünschte Fragment anhand mitaufgetragener Molekulargewichtsmarker lokalisiert und durch weitere Elektrophorese an DE 81 Papier (Schleicher und Schüll) gebunden. Die DNA wird durch Spülen mit Niedrigsalzpuffer (200 mM NaCl, 20 mM Tris pH=7,5, 1 mM EDTA) gewaschen und anschließend mit einem Hochsalzpuffer (1 M NaCl, 20 mM Tris pH=7,5, 1 mM EDTA) eluiert. Die DNA wird durch Zusatz von Äthanol präzipitiert.

"Transformation" bedeutet das Einbringen von DNA in einen Organismus, so daß die DNA dort replizierbar ist, entweder extrachromosomal oder chromosomal integriert. Transformation von E.coli folgt der im M13 Cloning and Sequencing Handbuch (Cloning and Sequencing Handbook, Fa. Amersham, PI/129/83/12) angegebenen Methode.

"Sequenzieren" einer DNA bedeutet die Bestimmung der Nukleotidsequenz. Dazu wird zunächst die zu sequenzierende DNA mit verschiedenen Restriktionsenzymen geschnitten, und die Fragmente werden in entsprechend geschnittene M13 mp8, mp9, mp18 oder mp19 Doppelstrang DNA eingebracht, oder es werden die DNA mittels Ultraschall fragmentiert, die Enden repariert und die größenselektionierten Fragmente in Sma I geschnittene, dephosphorylierte M13 mp8 DNA eingebracht (Shotgun Methode). Nach der Transformation von E.coli JM 101 wird Einzelstrang DNA aus rekombinanten M13 Phagen entsprechend dem M13 Cloning and Sequencing manual (Cloning and Sequencing Handbook, Fa Amersham, PI/129/83/12) isoliert und nach der Didesoxymethode (Sanger et al., 1977) sequenziert. Als Alternative zur Verwendung des Klenowfragment der E.coli DNA Polymerase I bietet sich dabei die T7-DNA Polymerase an ("Sequenase, Fa. United States Biochemical Corporation). Die Sequenzreaktionen werden entsprechend dem Handbuch "Sequenase: Step-by-Step Protocols for DNA Sequencing With Sequenase" (Version 2.0) durchgeführt.

Eine weitere Sequenziermethode besteht im Klonieren der zu sequenzierenden DNA in einen Vektor, der unter anderem einen Replikationsursprung eines DNA-Einzelstrangphagen (M13, fl) trägt (z.B. Bluescribe oder Bluescript M13 von Stratagene). Nach Transformation von E.coli JM101 mit dem rekombinanten Molekül können die Transformanten mit einem Helferphagen, zB. M13K07 oder R408 von Promega) infiziert werden. Als Resultat erhält man eine Mischung aus Helferphagen und verpacktem, einzelsträngigem rekombinanten Vektor. Die Aufarbeitung der Sequenziervorlage (Template) erfolgt in Analogie zu der M13 Methode. Doppelsträngige Plasmid-DNA wurde entsprechend dem oben angeführten Sequenzierhandbuch durch Alkalibehandlung denaturiert und direkt sequenziert.
Die Auswertung der Sequenzen erfolgte mittels der ursprünglich von R. Staden (Staden et al., 1982) entwickelten und von Ch. Pieler (Pieler, 1987) modifizierten Computerprogramme.

"Ligieren" bezieht sich auf den Prozeß der Bildung von Phosphodiesterbindungen zwischen zwei Enden von Doppelstrang-DNA Fragmenten. Üblicherweise werden zwischen 0,02 und 0,2 µg DNA-Fragmente in 10 µl mit etwa 5 units T4-DNA Ligase ("Ligase") in einer geeigneten Pufferlösung ligiert (Maniatis et al., 1982).
"Präparation" von DNA aus Transformanten bedeutet die Isolierung der Plasmid DNA aus Bakterien mittels der alkalischen SDS Methode, modifiziert nach Birnboim und Doly, unter Weglassen des Lysozyms. Dabei werden die Bakterien aus 1,5 bis 50 ml Kultur verwendet.

"Oligonukleotide" sind kurze Polydesoxynukleotide, die chemisch synthetisiert werden. Dazu wurde der Applied Biosystems Synthesizer Modell 381A verwendet. Die Oligonukleotide werden entsprechend dem Modell 381A User Manual (Applied Biosystems) aufgearbeitet. Sequenzprimer werden ohne weitere Reinigung direkt eingesetzt. Andere Oligonukleotide werden bis zu einer Kettenlänge von 70 durch die "OPC"-Methode gereinigt (OPC = Oligonucleotid purification column, Applied Biosystems, Product Bulletin, January 1988). Längere Oligonukleotide werden durch Polyacrylamidgelelectrophorese (6% Acrylamid, 0,15% Bisacrylamid, 6 M Harnstoff, TBE-Puffer) gereinigt und nach der Elution aus dem Gel über eine G-25 Sepharosesäule entsalzt.

### Beispiel 3

### Herstellung von TNF-BP-spezifischen Hybridisierungssonden

Die Auswahl der Oligonukleotide wurde im Hinblick auf deren Verwendung zur Amplifizierung von cDNA mittels PCR getroffen:
a) Aus der N-terminalen Aminosäuresequenz des TNF-Bindungsproteins (Hauptsequenz, erhalten aus Vorversuch 3 und Beispiel 1, Fraktion 1) 
Asp-Ser-Val-Cys-Pro-Gln-Gly-Lys-Tyr-Ile-
His-Pro-Gln-
 wurde ein Heptapeptid-Bereich ausgewählt, der die niedrigste Komplexität eines gemischten Oligonukleotids zum Hybridisieren an cDNA zuläßt: Es sind dies die Aminosäuren 6 bis 12. Um die Komplexität des Mischoligonukleotids herabzusetzen, wurden vier Mischoligonukleotide mit einer Komplexität von jeweils 48 hergestellt. Die Oligonukleotide wurden in Richtung der mRNA hergestellt, sie sind somit zum 3'Ende der Sequenz orientiert und identisch mit dem nichtkodierenden Strang des TNF-BP-Gens: Gln-Gly-Lys-Tyr-Ile-His-Pro 
5'CAA GGT AAA TAT ATT CAT CC 3'TNF-BP #3/1  EBI-1639
    G       G   C   C   C
                    A
 
5'CAA GGC AAA TAT ATT CAT CC 3'TNF-BP #3/2  EBI-1640
    G       G   C   C   C
                    A
 
5'CAA GGA AAA TAT ATT CAT CC 3'TNF-BP #3/3 EBI-1641
G G C C C
A
 
5'CAA GGG AAA TAT ATT CAT CC 3'TNF-BP #3/4  EBI-1642
    G       G   C   C   C
                    A
b) Aus der Aminosäuresequenz eines tryptischen Peptides (Fraktion 11 des tryptischen Verdaus) der Aminosäuresequenz 
Glu-Cys-Glu-Ser-Gly-Ser-Phe-Thr-Ala-Ser-(Glu/Cys)-Asn-
Asn-Lys (vgl. Beispiel 1)
 wurde ein Peptid-Bereich ausgewählt und ein weiterer Satz von Mischoligonukleotiden synthetisiert:

-Phe-Thr-Ala-Ser-Glu-Asn-Asn-Lys
Cys

TNF-BP #4/5 (EBI-1653):
3'AAA TGA CGG AGA CTC TTG TTG TT CCTAGGG 5'
    G   G   T   T   T
        T

TNF-BP #4/6 (EBI-1654):
3'AAA TGA CGG TCA CTC TTG TTG TT CCTAGGG 5'
    G   G   T   G   T
        T

TNF-BP #4/7 (EBI-1657):
3'AAA TGA CGG AGA ACA TTG TTG TT CCTAGGG 5'
    G   G     T   T    G
        T

TNF-BP #4/8 (EBI-1658):
3'AAA TGA CGG TCA ACA TTG TTG TT CCTAGGG 5'
    G  G   T   G   G
       T

Die Oligonukleotide wurden komplementär zur mRNA synthetisiert und sind somit zum 5'Ende der Sequenz orientiert. Um das amplifizierte DNA-Fragment im Anschluß an die PCR effizient klonieren zu können, wurde auch ein BamHI-Linker am 5'Ende der Oligonukleotide vorgesehen. Werden z.B. die Oligonukleotide TNF-BP-#4/5-8 gemeinsam mit TNF-BP #3/1-4 für die PCR an der gesamten λ-DNA einer Bibliothek eingesetzt, kann ein etwa resultierendes DNA Fragment mit BamHI nachgeschnitten werden. Die Partner-Oligonukleotide ergeben ein gerades Ende am 5'Terminus, das Fragment kann somit in die SmaI-BamHI-Stellen eines geeigneten Vektors kloniert werden.

Jedes Mischoligonukleotid TNF-BP #4/5 bis 8 ist eine Mischung aus 48 Einzelnukleotiden und berücksichtigt einige Codons nicht, und zwar:

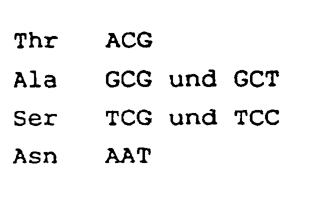

Bei GCT wird die Möglichkeit in Betracht gezogen, daß das zu GCC (Ala) komplementäre Triplett CGG durch Ausbildung einer G-T Brücke wirksam sein kann, bei TCG (Ser) und AAT (Asn) gilt dasselbe bezüglich AGT bzw. TTG.

ACG, GCG und TCG sind äußerst seltene Codons (CG-Regel) und wurden deshalb nicht berücksichtigt.

### Beispiel 4

### Amplifizierung einer für TNF-BP kodierenden Teilsequenz aus einer cDNA-Bibliothek.

### a) Isolierung von λ-DNA einer cDNA Bibliothek

Die Herstellung der cDNA Bibliothek erfolgte nach der in der EP-A1-0293 567 für die humane plazentale cDNA Bibliothek beschriebenen Methode mit dem Unterschied, daß als Ausgangsmaterial 10⁹ Fibrosarkomzellen der Zellinie HS 913 T, die unter Stimulierung mit humanem TNF-α (10 ng/ml) hochgezüchtet worden waren, verwendet wurden. Statt λ gt10 wurde λ gt11 verwendet (cDNA Synthese: Amersham RPN 1256; EcoRI verdaute λ gt11 Arme: Promega Biotech; in vitro Verpacken der ligierten DNA: Gigapack Plus, Stratagene).

5 ml des Phagenüberstandes der amplifizierten cDNA Bibliothek der humanen Fibrosarkom Zellinie HS913T in λ gtll wurden mit 0,5 µg RNase A und 0,5 µg DNase I versetzt und eine Stunde bei 37°C inkubiert. Die Mischung wurde 10 min bei 5000xg zentrifugiert, der Überstand durch Extraktion mit Phenol und Chloroform von Protein befreit und die DNA aus der wässrigen Phase durch Zusatz von Ethanol präzipitiert. Die λ-DNA wurde in TE-Puffer (10 mM Tris pH=7,5; 1 mM EDTA) gelöst.

### b) PCR Amplifizierung einer TNF-BP Sequenz aus einer cDNA Bibliothek

Für die Anwendung der PCR (Saiki et al., 1988) auf DNA der HS913T cDNA Bibliothek wurden 16 Einzelreaktionen durchgeführt, in welchen jeweils eines der 4 Mischoligonukleotide EBI-1639, EBI-1640, EBI-1641, EBI-1642 als erster Primer und eines der vier Mischoligonukleotide EBI-1653, EBI-1654, EBI-1657, EBI-1658 als zweiter Primer eingesetzt wurde. Jedes dieser Mischoligonukleotide enthält 48 verschiedene Oligonukleotide gleicher Länge.
Die Amplifizierung mittels PCR fand in 50 µl Reaktionsvolumen, enthaltend 250 ng λ-DNA der cDNA-Bibliothek, 50 mM KCl, 10 mM Tris pH=8,3, 1,5 mM MgCl₂, 0,01% Gelatine, 0,2 mM jedes der 4 desoxy-Nukleosidtriphosphate (dATP, dGTP, dCTP, dTTP), je 200 pMol erster und zweiter Primer, 1,25 Einheiten Taq Polymerase [Perkin-Elmer Cetus] statt. Um ein Verdunsten zu verhindern, wurde die Lösung mit einigen Tropfen Mineralöl (0,1 ml) überschichtet. Die PCR wurde in einem DNA Thermal Cycler (Perkin Elmer Cetus) folgendermaßen durchgeführt: Die Proben wurden 5 Minuten auf 94°C erhitzt, um die DNA zu denaturieren, und anschließend 40 Amplifikationszyklen unterworfen. Ein Zyklus bestand aus 40 Sekunden Inkubation bei 94°C, 2 Minuten Inkubation bei 55°C und 3 Minuten Inkubation bei 72°C. Am Ende des letzten Zyklus wurden die Proben für weitere 7 Minuten bei 72°C inkubiert, um sicherzustellen, daß die letzte Primer-Verlängerung vollständig verläuft. Nach Abkühlen auf Raumtemperatur wurden die Proben mit Phenol und Chloroform von Protein befreit und die DNA mit Äthanol präzipitiert.

5 µl jeder der 16 PCR-Proben wurden auf ein Agarosegel aufgetragen und die Länge der amplifizierten DNA-Fragmente nach elektrophoretischer Auftrennung bestimmt. Die stärkste DNA Bande, ein Fragment von 0,16 kb Länge, war in den PCR-Proben zu sehen, die mit dem Oligonukleotid EBI-1653 als erstem Primer und einem der Oligonukleotide EBI-1639, EBI-1640, EBI-1641 oder EBI-1642 als zweitem Primer amplifiziert worden waren. Da die mit dem Primerpaar EBI-1653 und EBI-1642 amplifizierte Probe die größte Menge an diesem 0,16 kb DNA-Fragment enthielt, wurde diese Probe für die weitere Aufarbeitung ausgewählt.

### Beispiel 5:

### Klonierung und Sequenzierung eines durch PCR Amplifikation gewonnenen DNA-Fragments

Das erhaltene PCR-Produkt der Primer EBI-1642 und EBI-1653 wurde mit BamHI geschnitten und nachfolgend elektrophoretisch in einem Agarosegel (1,5% NuSieve GTG Agarose plus 1% Seakem GTG Agarose, FMC Corporation) nach der Größe aufgetrennt. Die Hauptbande, ein DNA Fragment von 0,16 kb Länge, wurde aus dem Gel elektroeluiert und mit Ethanol präzipitiert. Dieses DNA Fragment wurde mit BamHI/SmaI geschnittenem Plasmid pUC18 (Pharmacia) ligiert und E. coli JM101 mit dem Ligationsgemisch transformiert. Die nach der Minipräparationsmethode hergestellten Plasmide wurden durch Schneiden mit den Restriktionsenzymen PvuII und EcoRI-BamHI und nachfolgender Elektrophorese in Agarosegelen charakterisiert. Das Plasmid pUC18 enthält zwei Schnittstellen für PvuII, die in einem 0,32 kb DNA-Fragment die Polyklonierstelle flankieren. Sehr kurze DNA-Inserts in der Polyklonierstelle des Plasmids können nach Schneiden mit PvuII leichter im Agarosegel sichtbar gemacht werden, da sich die Länge um 0,32 kb vergrößert. Durch Schneiden mit EcoRI und BamHI kann das in den mit BamHI und SmaI geschnittenen Plasmidvektor ligierte DNA-Fragment inklusive einiger Basenpaare der Polylinkersequenz erhalten werden. Ein Klon mit dem gewünschten Insert wurde als pTNF-BP3B bezeichnet. Das gesamte DNA-Insert dieses Klons wurde nach Subklonieren eines EcoRI-BamHI Fragments in M13mp18 (Pharmacia) nach der modifizierten Didesoxy Methode mit Sequenase (United States Biochemical Corporation) sequenziert.

Die Analyse der durch PCR-amplifizierten DNA ergab folgende Sequenz (nur der nicht kodierende Strang ist abgebildet, darüber die abgeleitete Aminosäuresequenz):

5 10
Gln Gly Lys Tyr Ile His Pro Gln Asn Asn Ser Ile Cys
CAG GGG AAA TAT ATT CAC CCT CAA AAT AAT TCG ATT TGC

15                      20                      25
Cys Thr Lys Cys His Lys Gly Thr Tyr Leu Tyr Asn Asp
TGT ACC AAG TGC CAC AAA GGA ACC TAC TTG TAC AAT GAC

30 35
Cys Pro Gly Pro Gly Gln Asp Thr Asp Cys Arg Glu Cys
TGT CCA GGC CCG GGG CAG GAT ACG GAC TGC AGG GAG TGT

40                    45                    50
Glu Ser Gly Ser Phe Thr Ala Ser Glu Asn Asn Lys
GAG AGC GGC TCC TTC ACA GCC TCA GAA AAC AAC AAG GAT CC

Die ersten 20 und die letzten 29 Nukleotide (in Kursivschrift) entsprechen den Sequenzen der Primer-Oligonukleotide EBI-1642 bzw. dem Komplement von EBI-1653. Die Aminosäuren 38 bis 43 bestätigen die restliche Sequenz des tryptischen Peptides 11.

weiters enthält das mittels PCR erzeugte DNA-Fragment die Sequenz des Peptides der Fraktion 20 des tryptischen Verdaus (Aminosäuren 20 bis 34, unterstrichen). Damit ist erwiesen, daß der Klon pTNF-BP3B von einer cDNA abgeleitet wurde, die für TNF-Bindungsprotein kodiert.
pTNF-BP3B stellt damit eine Sonde, z.B. zum Durchsuchen von cDNA-Bibliotheken nach TNF-BP cDNAs, dar.

### Beispiel 6:

### Isolierung von TNF-BP cDNA Klonen

Ca. 720.000 Phagen der HS913T cDNA Bibliothek in λ gt11 wurden auf E.coli Y1088 (Δ lacU169, pro::Tn5, tonA2, hsdR, supE, supF, metB, trpR, F⁻,λ -, (pMC9)) plattiert (ca. 60.000 Phagen pro 14,5 cm Petrischale, LB-Agar: 10 g/l Trypton, 5 g/l Hefeextrakt, 5 g/l NaCl, 1,5% Agar, Plattieren in Top-Agarose: 10 g/l Trypton, 8 g/l NaCl, 0,8% Agarose). Von jeder Platte wurden zwei Nitrozellulosefilter-Abzüge hergestellt. Die Filter wurden vorgewaschen (16 Stunden bei 65°C) in:

| | |
|---|---|
| 50 mM | Tris/HCl pH=8,0 |
| 1 M | NaCl |
| 1 mM | EDTA |
| 0,1 % | SDS |

Die Filter wurden zwei Stunden bei 65°C prähybridisiert in:

| | |
|---|---|
| 6x | SSC (0,9M NaCl, 0,09 M tri-Na-citrat) |
| 5x | Denhardt's (0,1% Ficoll, 0,1% Polyvinylpyrrolidon, 0,1% BSA (=Rinderserumalbumin)) |
| 0,1% | SDS |

### Herstellung der radioaktiv markierten Sonde:

pTNF-BP 3B wurde mit BamHI und EcoRI doppelt geschnitten und das ca. 0,16 kb Insert isoliert. 0,6 µg des Inserts in 32 µl werden bei 100°C denaturiert und mit je 60 pMol EBI-1642 und EBI-1653 durch Abkühlen auf 80°C über 10 Minuten und jähes Abkühlen in Eiswasser geprimt. Nach Zusatz von

| | |
|---|---|
| 10 µl | α-32P-dCTP (100 µCi, 3,7 MBq) |
| 5 µl | 10x Priming Puffer (0,1 M Tris/HCl pH=8,0, 50 mM MgCl₂) |
| 2 µl | je 1mM dATP, dGTP, dTTP |
| 1 µl | PolIK (Klenow Fragment der E.coli DNA Polymerase I, 5 Einheiten) |

wurde 90 Minuten bei Raumtemperatur inkubiert. Nach Hitzeinaktivierung (10 Minuten auf 70°C) erfolgte das Abtrennen der nichteingebauten Radioaktivität durch Chromatographie über Biogel P6DG (Biorad) in TE Puffer (10 mM Tris/HCl pH=8, 1 mM EDTA). Eingebaut wurden 65x106 cpm.
Das Hybridisieren der Filter erfolgte in einem Gesamtvolumen von 80 ml 6xSSC/5X Denhardt's/0,1% SDS plus hitzedenaturierter Hybridisiersonde während 16 Stunden bei 65°C.
Die Filter wurden zweimal 30 Minuten bei Raumtemperatur in 6xSSC/0,01% SDS und einmal 45 Minuten bei Raumtemperatur in 2xSSC/0,01% SDS und dreimal 30 Minuten bei 65°C in 2xSSC/0,01% SDS gewaschen. Die Filter wurden luftgetrocknet und anschließend an Amersham Hyperfilm 16 Stunden unter Verwendung einer Verstärkerfolie bei -70°C exponiert. Insgesamt wurden 30 hybridisierende Plaques identifiziert (λ-TNF-BP #1-30).
Die Regionen mit den hybridisierenden Plaques wurden möglichst präzise ausgestochen, und die Phagen in 300 µl SM Puffer plus 30 µl Chloroform eluiert.
Durch "Plaquereinigung" (Plattieren von ca. 200 Phagen pro 9 cm Petrischale beim zweiten Durchgang, bzw. ca. 20 Phagen pro 9 cm Petrischale beim dritten Durchgang, Filterabzüge doppelt, Vorbereiten, Hybridisieren und Waschen wie beim erstmaligen Durchsuchen beschrieben) wurden letztlich 25 hybridisierende Phagen vereinzelt (λ-TNF-BP #1-10, 12-24, 29,30).

### Darstellung der rekombinanten λ-DNA von den Klonen λ-TNF-BP #13, 15, 23, 30:

2x10⁶ Phagen wurden auf E.coli Y1088 in Topagarose (10 g/l Trypton, 8 g/l NaCl, 0,8% Agarose) plattiert (14,5 cm Petrischale mit LB-Agarose (1,5% Agarose, 0,2% Glucose, 10 mM MgSO₄, 10 g/l Trypton, 5 g/l Hefeextrakt, 5 g/l NaCl) und 6 Stunden bei 37°C inkubiert. Nach Abkühlen der Platten (30 Minuten bei 4°C) wurde mit 10 ml λ-Diluent (10 mM Tris/HCl pH=8,0, 10 mM MgCl₂, 0,1 mM EDTA) überschichtet und 16 Stunden bei 4°C eluiert. Der Überstand wurde in 15 ml Corex Röhrchen transferiert und 10 Minuten bei 15000 rpm und 4°C zentrifugiert (Beckman J2-21 Zentrifuge, JA20 Rotor). Der Überstand wurde in 10 ml Polycarbonat-Röhrchen dekantiert und bei 50000 rpm, 20°C bis ω²t=3x10¹⁰ zentrifugiert (Beckman L8-70, 50 Ti Rotor). Das Pellet wurde in 0,5 ml λ-Diluent resuspendiert und in Eppendorf Röhrchen (1,4 ml) transferiert. Nach Zusatz von 5 µg RNase A und 0,5 µg DNaseI und Inkubation bei 37°C während 30 Minuten und Zusatz von 25 µl 0,5 M EDTA, 12,5 µl 1 M Tris/HCl pH=8.0, 6,5 µl 20% SDS erfolgte weitere Inkubation bei 70°C für 30 Minuten. Die λ-DNA wurde durch Phenol/Chloroform Extraktion gereinigt und mit Ethanol gefällt. Abschließend wurde die DNA in 100 µl TE-Puffer gelöst.

### Beispiel 7:

### Subklonierung und Sequenzierung von TNF-BP cDNA Klonen 15 und 23

Um die cDNAs der Klone λTNF-BP15 und λTNF-BP23, die bei der Hybridisierung die stärksten Signale gezeigt hatten, näher zu charakterisieren, wurden die cDNA-Inserts mit EcoRI aus der λ-DNA herausgeschnitten, nach elektrophoretischer Auftrennung aus einem Agarosegel eluiert und mit Äthanol präzipitiert. Die DNA-Fragmente von 1.3 kb (von λTNF-BP15) und 1.1 kb (von λTNF-BP23) wurden mit EcoRI geschnittenem und mit alkalischer Phosphatase aus Kalbsdarm dephosphoryliertem Plasmidvektor pT7/T3α-18 (Bethesda Research Laboratories) mit T4 DNA Ligase ligiert und E.coli JM101 transformiert. Von einzelnen Bakterienkolonien, die nach Selektion auf Agaroseplatten mit Ampicillin und X-gal keine blaue Färbung aufwiesen, wurde im Minipräparationsverfahren Plasmid-DNA hergestellt und durch Schneiden mit EcoRI und HindIII das Vorhandensein und die Orientierung des cDNA Inserts festgestellt. Plasmide, die das EcoRI Insert der Phagen λTNF-BP15 bzw. λTNF-BP23 so orientiert enthielten, daß das dem 5'-Ende der mRNA entsprechende Ende dem T7 Promoter zugewandt ist, wurden pTNF-BP15 bzw. pTNF-BP23 benannt. Die EcoRI Inserts von λTNF-BP15 und λTNF-BP23 wurden ebenfalls in mit EcoRI geschnittenen und dephosphorylierten M13mp19 Vektor ligiert und E.coli JM101 transformiert. Von einigen wahllos ausgewählten M13 Klonen wurde Einzelstrang-DNA präpariert und als Vorlage für die Sequenzierung nach der Didesoxy-Methode verwendet.
An M13 Klonen, die die cDNA-Inserts in entgegengesetzter Orientierung enthielten, wurden mit dem universellen Sequenzierprimer und spezifisch synthetisierten Oligonukleotidprimern, die an das cDNA-Insert binden, beide DNA-Stränge vollständig sequenziert.

Die vollständige Nukleotidsequenz von 1334 Basen des cDNA-Inserts von λTNF-BP15 bzw. pTNF-BP15 ist in Fig.1 dargestellt. Die Basen 1-6 und 1328-1334 entsprechen den EcoRI-Linkern, die bei der Herstellung der cDNA-Bibliothek an die cDNA angefügt worden waren.
Die Nukleotidsequenz des cDNA-Inserts von λTNF-BP23 entspricht der von λTNF-BP15 (Basen 22-1100), flankiert von EcoRI-Linkern.

Der Klon λTNF-BP30 wurde ebenfalls untersucht; seine Sequenz entspricht λTNF-BP15 mit dem Unterschied, daß die Sequenz eine Deletion von 74 bp (Nukleotid 764 bis 837) aufweist.

### Beispiel 8

### Konstruktion der Expressionsplasmide pAD-CMV1 und pAD-CMV2

Aus Teilen der Expressionsplasmide pCDM8 (Seed und Aruffo, 1987. Seed, 1987; Invitrogen), pSV2gptDHFR20 (EP-A1 0321 842) und dem Plasmid Bluescript SK+ (Short et al., 1988; Stratagene) wurde ein neues Plasmid konstruiert, das eine Multiklonierstelle für die gerichtete Insertion heterologer DNA-Sequenzen aufweist und sich in E.coli mittels Ampicillinresistenz mit hoher Kopienzahl vermehren läßt. Die intergenische Region von M13 ermöglicht die Herstellung einzelsträngiger Plasmid-DNA mittels Superinfektion der transformierten Bakterien mit einem Helferphagen (z.B. R408 oder M13K07) zur erleichterten Sequenzierung und Mutagenese der Plasmid-DNA. Der T7 Promoter, der der Multiklonierstelle vorangeht, ermöglicht die Herstellung von RNA Transkripten in vitro. In Säugetierzellen erfolgt die Expression heterologer Gene getrieben vom Cytomegalovirus (CMV) Promoter/Enhancer (Boshart et al., 1985). Der SV40 Replikationsursprung ermöglicht in geeigneten Zellinien (z.B. SV40 transformierte Zellen wie COS-7, Adenovirus transformierte Zellinie 293 (ATCC CRL1573) die autonome Replikation des Expressionsplasmides zu hohen Kopienzahlen und damit hohe Raten in transienter Expression. Für die Herstellung permanent transformierter Zellinien und die nachfolgende Amplifikation der Expressionskassette mittels Methotrexat dient ein modifiziertes Hamster-Minigen (Promoter mit kodierendem Bereich und dem ersten Intron) für Dihydrofolatreduktase (DHFR) als Selektionsmarker.

### a) Herstellung der Vektor- und Promoteranteile durch PCR

Das Plasmid Bluescript SK+ wurde mit HindIII linearisiert und 5 ng DNA in einem 100 µl PCR Ansatz eingesetzt (Reaktionspuffer: 50 mM KCl, 10 mM Tris-Cl pH=8,3, 1,5 mM MgCl₂, 0,01% (w/v) Gelatine, 0,2 mM der vier Desoxynukleotidtriphosphate (dATP, dGTP, dCTP, dTTP), 2,5 Einheiten Taq Polymerase pro 100 µl). Als Primer wurden je 50 pmol der synthetischen Oligonukleotide EBI-1786 (5'-GGAATTCAGCCTGAA-TGGCGAATGGG-3'; bindet knapp außerhalb von M13 ori-Region in Bluescript Pos. 475, unabhängig von M13 ori-Orientierung) und EBI-1729 (5'-CCTCGAGCGTTGC-TGGCGTTTTTCC-3'; bindet an Bluescript an Pos. 1195 vor ori, entspricht dem Anfang der Bluescript-Sequenz in pCDM8, 6 Basen 5'ergeben XhoI) eingesetzt. Nach 5 Minuten Denaturieren bei 94°C erfolgte die PCR über 20 Zyklen (40 sec bei 94°C, 45 sec bei 55°C, 5 Min bei 72°C, Perkin Elmer Cetus Thermal Cycler). Die Oligonukleotide flankieren die intergenische Region von M13 bzw. den Replikationsursprung (ori) mit dem dazwischenliegenden Gen für die β-Lactamase. Gleichzeitig wird am Ende des Replikationsursprungs eine XhoI- und am anderen Ende eine EcoRI-Schnittstelle erzeugt. Das Reaktionsgemisch wurde durch Extraktion mit Phenol-Chloroform von Protein befreit und die DNA mit Ethanol präzipitiert. Die erhaltene DNA wurde mit XhoI und EcoRI geschnitten und nach Elektrophorese in einem Agarosegel ein Fragment mit 2,3 kb isoliert.

5 ng mit SacII linearisiertes Plasmid pCDM8 wurden mit den Oligonukleotiden EBI-1733 (5'-GGTCGACATTGA-TTATTGACTAG-3'; bindet an CMV-Promotorregion (Pos. 1542) von pCDM8, entspricht Pos.1 in pAD-CMV, SalI-Stelle für Klonierung) und EBI-1734 (5'-GGAATTCCCTAG-GAATACAGCGG-3'; bindet an Polyoma origin von 3'SV40 polyA-Region in pCDM8 (Pos. 3590)) unter identischen Bedingungen wie für Bluescript SK+ beschrieben, durch PCR amplifiziert. Die Oligonukleotide binden am Beginn der CMV-Promoter/Enhancer-Sequenz und erzeugen eine SalI Schnittstelle (EBI-1733) bzw. binden am Ende der SV40 poly-Adenylierungstelle und erzeugen eine EcoRI Schnittstelle (EBI-1734). Das PCR-Produkt wurde mit SalI und EcoRI geschnitten und ein DNA Fragment von 1,8 kb aus einem Agarosegel isoliert.

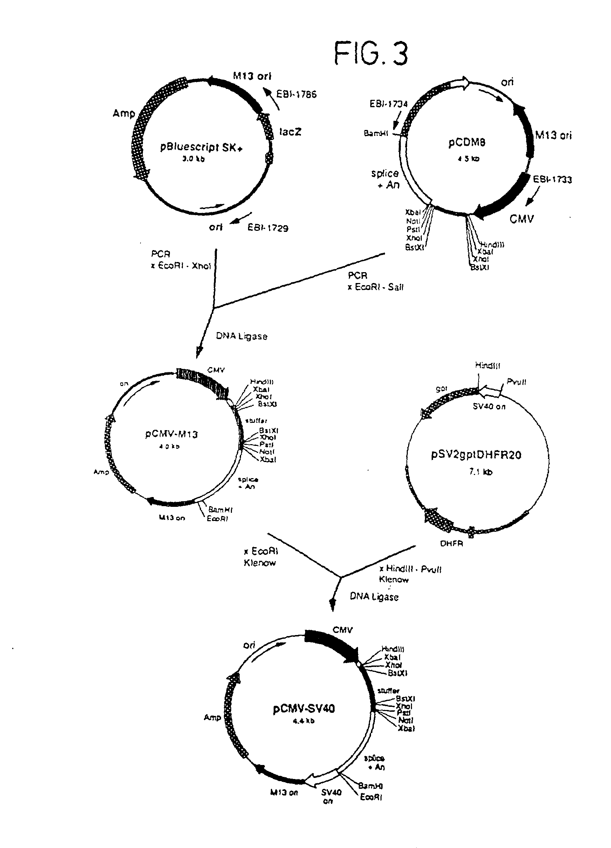

Die beiden PCR Produkte wurden mit T4 DNA-Ligase ligiert, mit dem erhaltenen Ligationsprodukt E.coli HB101 transformiert und nach Standardmethoden Plasmid-DNA amplifiziert und präpariert. Das Plasmid der gewünschten Beschaffenheit (siehe Fig.3) wurde pCMV-M13 benannt.

Der SV40 Replikationsursprung (SV40 ori) wurde aus dem Plasmid pSV2gptDHFR20 (EP-A1 0321842) isoliert. Dazu wurde dieses Plasmid mit HindIII und PvuII doppelt geschnitten und die DNA-Enden durch nachfolgende Behandlung mit dem großen Fragment der E.coli DNA Polymerase (Klenow Enzym) in Gegenwart der vier Desoxynukleotidtriphosphate stumpf gemacht. Ein dabei erhaltenes 0,36 kb DNA Fragment wurde aus einem Agarosegel isoliert und in mit EcoRI linearisiertem pCMV-M13 ligiert. Ein nach Transformation von E.coli HB101 erhaltenes Plasmid mit dem SV40 ori in gleicher Orientierung wie das β-Lactamase Gen und dem CMV-Promoter wurde pCMV-SV40 benannt. Die Konstruktion dieses Plasmids ist in Fig.3 dargestellt.

### b) Mutagenese des DHFR-Gens

Zur Herstellung eines Expressionsplasmids mit einer vielseitigen Multiklonierstelle wurden aus dem DHFR Minigen durch gerichtete Mutagenese zwei und durch Deletion drei Restriktionsenzymschnittstellen entfernt. Dazu wurde aus dem Plasmid pSV2gptDHFR20 ein 1,7 kb BglII Fragment, das die gesamte kodierende Region des Hamster DHFR-Gens enthält, in die BglII Stelle des Plasmids pUC219 (IBI) kloniert und das Plasmid pUCDHFR erhalten. Mit pUCDHFR transformierte E.coli JM109 (Stratagene) Zellen wurden mit etwa 40-fachem Überschuß des Helferphagen R408 (Stratagene) infiziert und 16 Stunden bei 37°C in LB-Medium geschüttelt. Aus dem Bakterienüberstand wurde einzelsträngige Plasmid-DNA isoliert.

Die gerichtete Mutagenese erfolgte in zwei aufeinanderfolgenden Schritten, wobei das in vitro Mutagenese System RPN1523 (Amersham) verwendet wurde. Die am Beginn von Exon 2 befindliche EcoRI Stelle wurde durch Austausch einer Base von GAATTC zu GAGTTC zerstört. Dieser Basenaustausch führt zu keiner Änderung der kodierten Aminosäuresequenz und entspricht außerdem der Nukleotidsequenz im natürlichen murinen DHFR-Gen (McGrogan et al., 1985, Mitchell et al., 1986). Für die Mutagenese wurde ein Oligonukleotid (Antisense-Orientierung) der Sequenz 5'-GTACTTGA-ACTCGTTCCTG-3' (EBI-1751) verwendet. Ein Plasmid mit der gewünschten Mutation wurde, wie oben beschrieben, als Einzelstrang-DNA präpariert und die im ersten Intron befindliche PstI Stelle durch Mutagenese mit dem Oligonukleotid EBI-1857 (Antisense Orientierung, 5'-GGCAAGGGCAGCAGCCGG-3') von CTGCAG in CTGCTG entfernt. Die Mutationen wurden durch Sequenzierung bestätigt und das erhaltene Plasmid pUCDHFR-Mut2 benannt.

Aus dem Plasmid pUCDHFR-Mut2 wurde das 1,7 kb BglII Fragment isoliert und in mit BglII und BamHI doppelt geschnittenes Plasmid pSV2gptDHFR20 ligiert. Nach Transformation von E.coli, Amplifikation und DNA-Isolierung wurde ein Plasmid der gewünschten Beschaffenheit erhalten, das als pSV2gptDHFR-Mut2 bezeichnet wurde. Durch Schneiden mit BamHI wurde in der 3'-nicht-kodierenden Region des DHFR Gens ein auf die BglII Stelle folgendes 0,12 kb DNA-Fragment entfernt, das außerdem noch eine KpnI Schnittstelle enthält. Durch Verknüpfen der mit BglII und BamHI entstandenen überhängenden DNA-Enden wurden auch die Erkennungssequenzen für diese beiden Enzyme zerstört.

Das Plasmid pCMV-SV40 wurde mit EcoRI und BamHI doppelt geschnitten, die DNA-Enden nachfolgend mit Klenow-Enzym stumpf gemacht. Die DNA wurde durch Extraktion mit Phenol-Chloroform und Ethanolfällung gereinigt, anschließend durch Inkubation mit alkalischer Phosphatase dephosphoryliert und die 4,4 kb lange Vektor DNA aus einem Agarosegel isoliert.

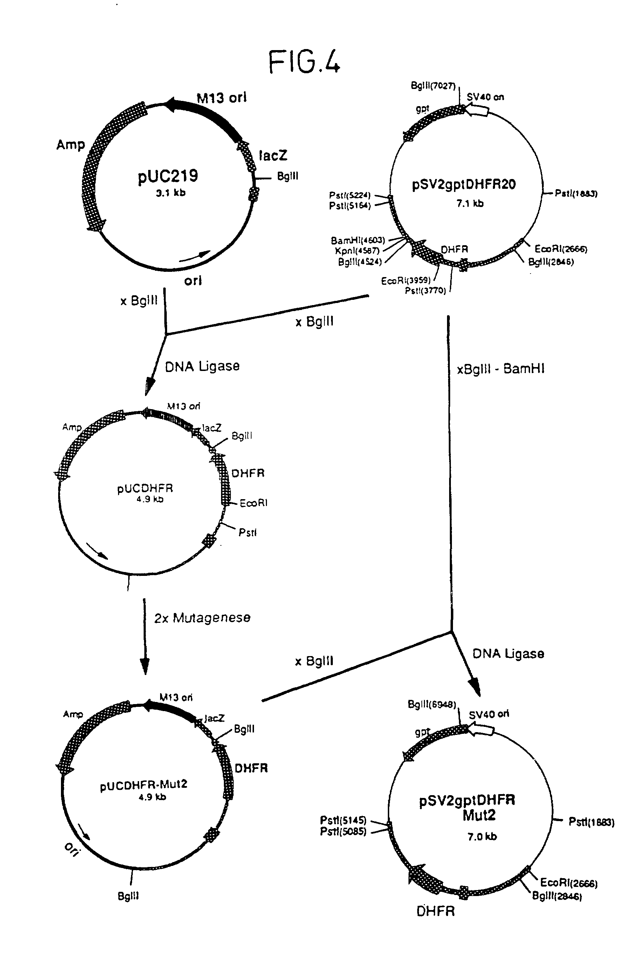

Das Plasmid pSV2gptDHFR-Mut2 (Fig.4) wurde mit EcoRI und PstI doppelt geschnitten und die DNA-Enden durch 20 Minuten Inkubation bei 11°C mit 5 Einheiten T4 DNA-Polymerase (50 mM Tris- HCl pH=8,0, 5 mM MgCl₂, 5 mM Dithiothreit, 0,1 mM jedes der vier Desoxynukleotidtriphosphate, 50 µg/ml Rinderserumalbumin) stumpf gemacht. Das 2,4 kb lange DNA-Fragment mit dem mutierten DHFR-Gen wurde aus einem Agarosegel isoliert und mit dem wie oben beschrieben präparierten pCMV-SV40 ligiert. Ein nach Transformation von E.coli erhaltenes Plasmid, das das DHFR-Gen in der selben Orientierung wie den CMV-Promoter enthielt, wurde pCMV-SV40DHFR benannt.

Im letzten Schritt wurde das 0,4kb "Stuffer"-Fragment nach dem CMV-Promoter, das noch aus dem Ausgangsplasmid pCDM8 stammte, gegen eine Multiklonierstelle ausgetauscht. Dazu wurde das Plasmid pCMV-SV40DHFR mit HindIII und XbaI doppelt geschnitten und der Vektoranteil aus einem Agarosegel isoliert. Die Multiklonierstelle, gebildet aus den beiden Oligonukleotiden EBI-1823 (5'-AGCTTCTGCAGGTCGA-CATCGATGGATCCGGTACCTCGAGCGGCCGCGAATTCT-3') und EBI-1829 (5'-CTAGAGAATTCGCGGCCGCTCGAGGTACCGGATCCATCGATG-TCGACCTGCAGA-3'), enthält inklusive der für die Klonierung in HindIII - XbaI kompatiblen Enden Restriktionsschnittstellen für die Enzyme PstI, SalI, ClaI, BamHI, KpnI, XhoI, NotI und EcoRI.

Je 1 µg der beiden Oligonukleotide wurden in 20 µl Reaktionspuffer (70 mM Tris-Cl pH=7,6, 10 mM MgCl₂, 5 mM Dithiothreit, 0,1 mM ATP) mit 5 Einheiten T4 Polynukleotidkinase eine Stunde bei 37°C inkubiert, um die 5'-Enden zu phosphorylieren. Die Reaktion wurde durch 10 minütiges Erhitzen auf 70°C gestoppt und die komplementären Oligonukleotide miteinander hybridisiert, indem die Probe weitere 10 Minuten bei 56°C inkubiert und anschließend langsam auf Raumtemperatur abgekühlt wurde. 4 µl der hybridisierten Oligonukleotide (100 ng) wurden mit etwa 100 ng Plasmidvektor ligiert und E.coli HB101 transformiert. Ein Plasmid, das sich mit den Enzymen der Multiklonierstelle (ausgenommen NotI) linearisieren ließ, wurde pAD-CMV1 benannt. Von vielen getesteten Klonen konnte keiner identifiziert werden, dessen Plasmid sich mit NotI schneiden ließ. Die Sequenzierung zeigte immer die Deletion von einigen Basen innerhalb der NotI Erkennungssequenz.

In gleicher Weise wurde mit dem Oligonukleotidpaar EBI-1820 (5'-AGCTCTAGAGAATTCGCGGCCGCTCGAGGT-ACCGGATCCATCGATGTCGACCTGCAGAAGCTTG-3') und EBI-1821 (5'-CTAGCAAGCTTCTGCAGGTCGACATCGATGGATCCGGTACCTCGAGCG-GCCGCGAATTCTCTAG-3') das Expressionsplasmid pAD-CMV2 hergestellt, das die Restriktionsschnittstellen innerhalb der Multiklonierstelle in umgekehrter Reihenfolge enthält. Dabei wurde das Plasmid pAD-CMV2 erhalten, das sich mit sämtlichen Restriktionsenzymen, einschließlich NotI, linearisieren ließ.

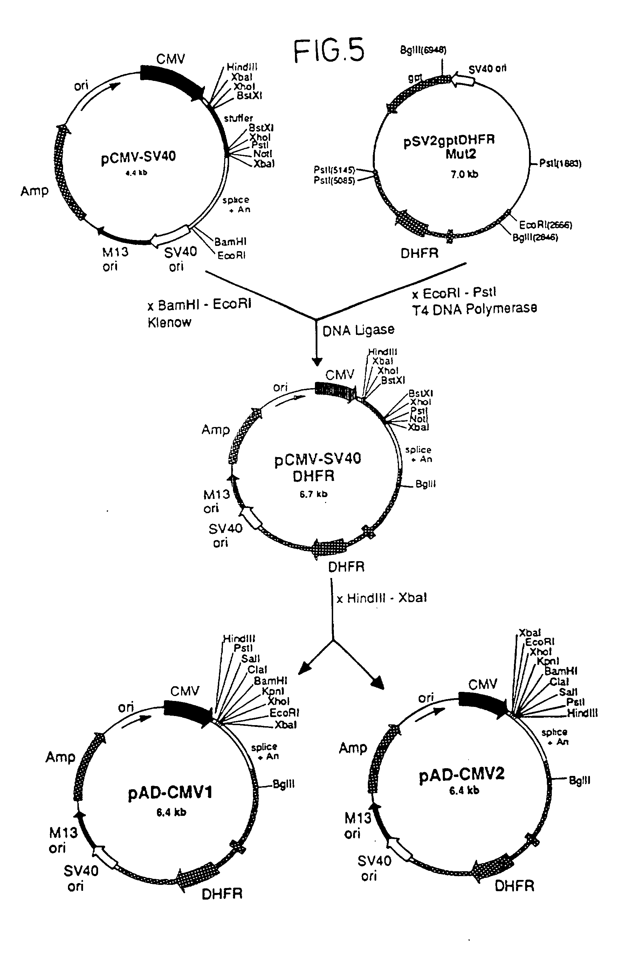

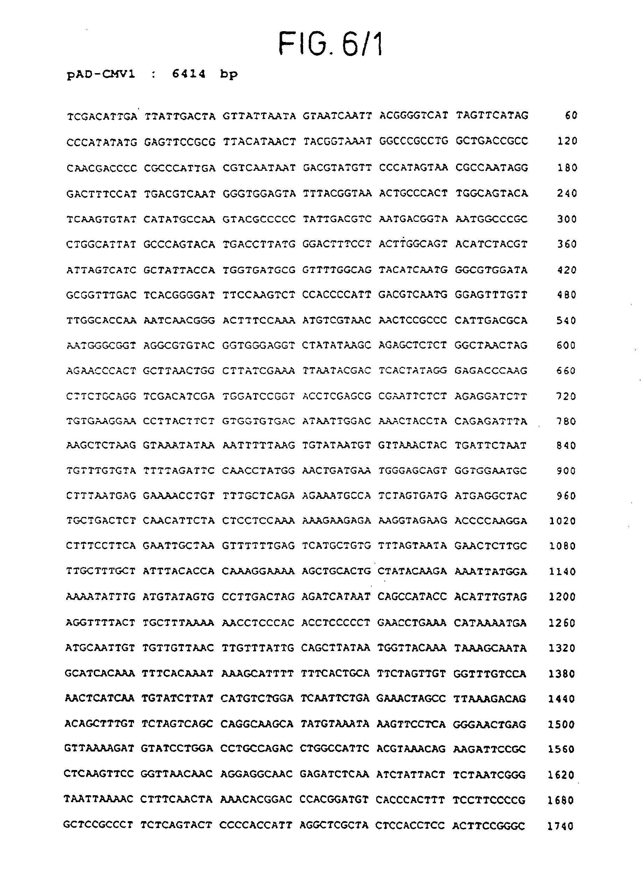

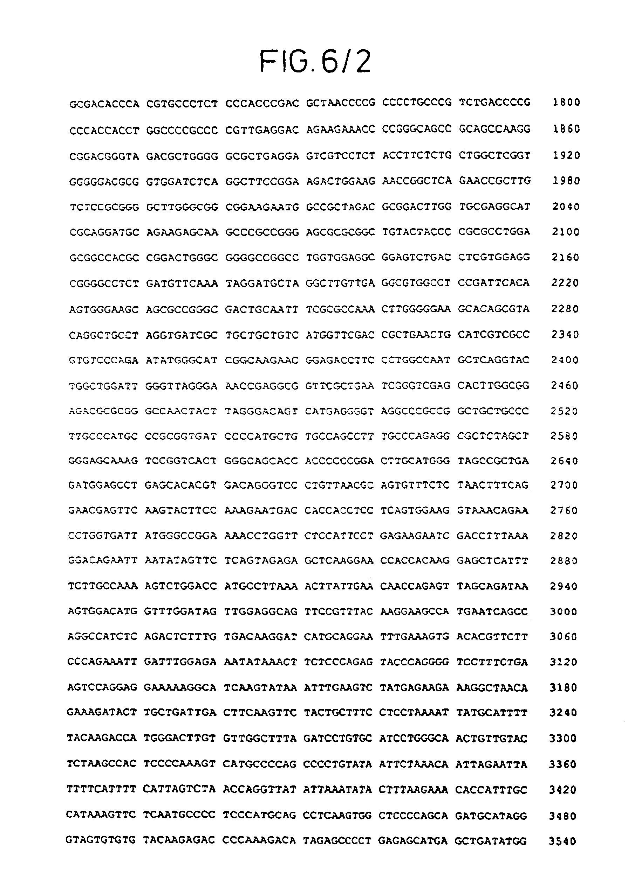

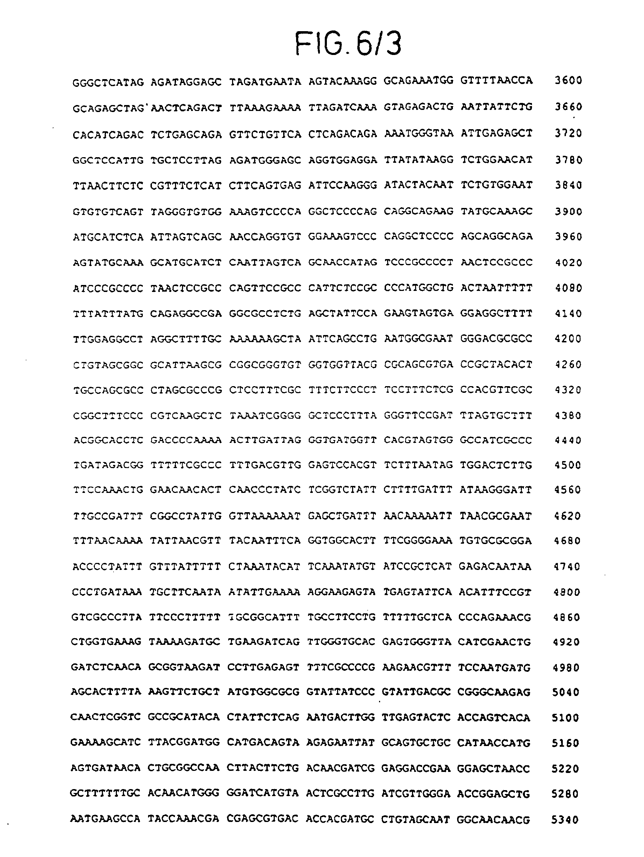

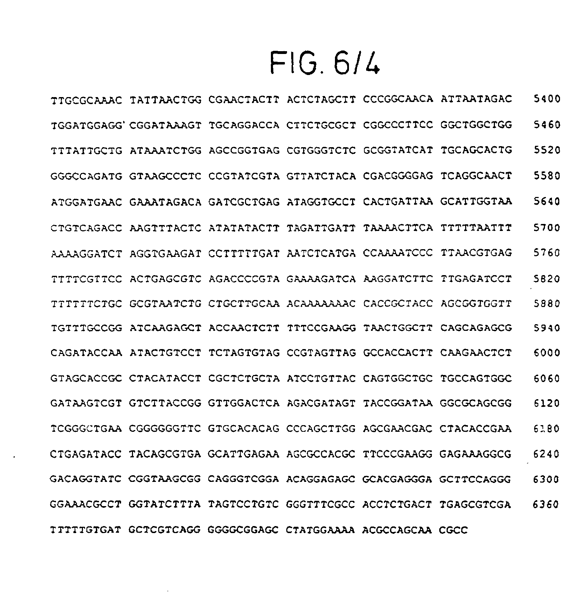

Die Nukleotidsequenz des 6414 bp großen Plasmids pAD-CMV1 (Fig.5) ist zur Gänze in Fig.6 dargestellt.

Die Abschnitte auf dem Plasmid (angegeben in der Numerierung der Basen) entsprechen folgenden Sequenzen:

| | |
|---|---|
| 1- 21 | EBI-1733, Beginn CMV Enhancer - Promotor (aus CDM8) |
| 632-649 | T7 Promotor |
| 658-713 | Multiklonierstelle (HindIII bis XbaI aus EBI-1823, EBI-1829) |
| 714-1412 | SV40 Intron und poly-Adenylierungsstelle (aus CDM8) |
| 1413-2310 | 5'nicht kodierende Region und Promotor des Hamster DHFR Gen (aus pSV2gptDHFR20) |
| 2311-2396 | Hamster DHFR: Exon 1 |
| 2516 | A zu T Mutation zerstört PstI Stelle in DHFR Intron 1 |
| 2701-3178 | DHFR Exons 2-6 (kodierende Region) |
| 2707 | A zu G Mutation zerstört EcoRI Stelle |
| 3272-3273 | Deletion zwischen BglII und BamHI in DHFR 3'nicht kodierender Region |
| 3831 | Ende DHFR Gen (aus pSV2gptDHFR20) |
| 3832-4169 | SV40 ori (aus pSV2gptDHFR20) |
| 4170-4648 | M13 ori (aus pBluescript SK+) |
| 4780-5640 | β-Lactamase (kodierende Region) |
| 6395-6414 | EBI-1729, Ende der pBluescript Vektorsequenz |

Die Herstellung der Plasmide pAD-CMV1 und pAD-CMV2 ist in Fig.5 dargestellt.

### Beispiel 9

### Konstruktion des Plasmids pADTNF-BP für die Expression der löslichen Form von TNF-BP

Um auf direktem Weg die sekretierte Form von TNF-BP herzustellen, wurde in der für einen Teil des TNF-Rezeptors kodierenden cDNA (vgl.Beispiel 7; im folgenden als TNF-R cDNA bezeichnet) nach dem Codon der C-terminalen Aminosäure des natürlichen TNF-BP (AAT, Asn-172; entspricht Pos.201 in Fig.9) ein Translationsstopcodon eingeführt. Dadurch wird die Proteinsynthese an dieser Stelle abgebrochen und ermöglicht, TNF-BP direkt in den Zellüberstand zu sekretieren, ohne eine nachfolgende, möglicherweise geschwindigkeitsbestimmende Reaktion einer proteolytischen Abspaltung in C-terminaler Richtung gelegener Abschnitte des TNF-Rezeptors durchlaufen zu müssen.

Gleichzeitig mit der Einführung des Stopcodons mittels PCR wurde die 5'-nicht kodierende Region der TNF-R cDNA verkürzt, um das Translationsstartcodon eines weiteren offenen Leserahmens (Basen 72-203 in Fig.9), der sich 5' von dem des TNF-R befindet, zu entfernen, und am 5'- bzw. 3'-Ende der cDNA eine BamHI bzw. EcoRI Schnittstelle eingeführt.

100 ng mit XmnI linearisiertes Plasmid pTNF-BP15 (vgl. Beispiel 7) wurden mit je 50 pmol der Oligonukleotide EBI-1986 (Sense, 5'-CAGGATCCGAGTCTCAACCCTCAAC-3') und EBI-1929 (Antisense, 5'-GGGAATTCCTTATCAATTCTCAATCT-GGGGTAGGCACAACTTC-3'; Einführung zweier Stop-Codons und einer EcoRI Stelle) in einem 100 µl PCR-Ansatz über 10 Zyklen amplifiziert. Die Zyklusbedingungen waren 40 Sekunden bei 94°C, 45 Sekunden bei 55°C und 5 Minuten bei 72°C. Nach dem letzten Zyklus wurde für weitere 7 Minuten bei 72°C inkubiert und die Reaktion durch Extraktion mit Phenol-Chloroform gestoppt. Die DNA wurde mit Ethanol präzipitiert und anschließend mit BamHI und EcoRI doppelt geschnitten. Das entstandene 0,75 kb DNA Fragment wurde aus einem Agarosegel isoliert und in mit BamHI und EcoRI doppelt geschnittenes Plasmid pT7/T3α-19 (BRL) kloniert. Eines der erhaltenen Plasmide, von dem aufgrund der Sequenzierung des gesamten Inserts festgestellt worden war, daß es die gewünschte Sequenz aufwies, wurde pTNF-BP benannt.

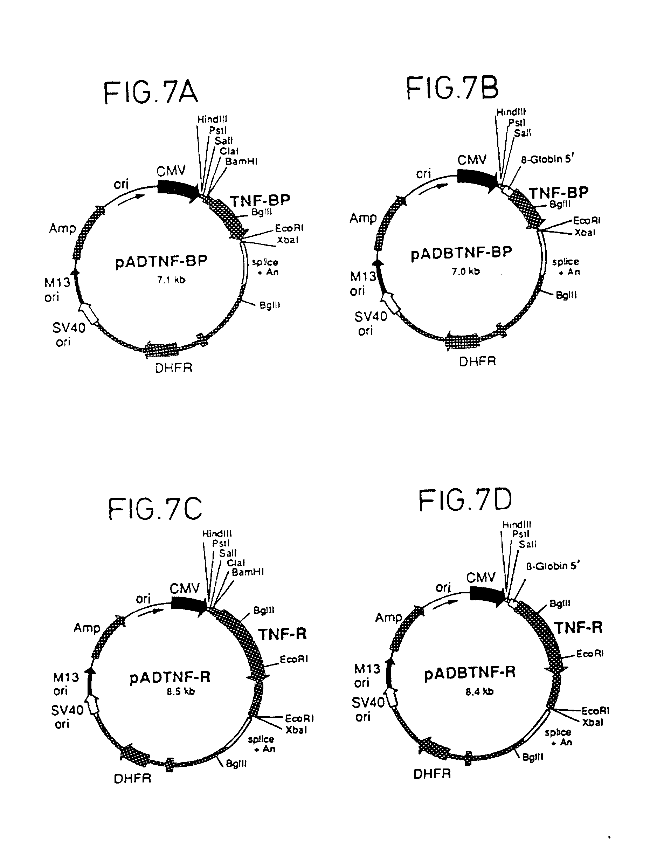

pTNF-BP wurde mit BamHI und EcoRI geschnitten und das 0,75 kb DNA Insert in das mit BamHI und EcoRI geschnittene Expressionsplasmid pAD-CMV1 kloniert. Ein erhaltenes Plasmid der gewünschten Zusammensetzung wurde pADTNF-BP benannt (Fig.7A).

### Beispiel 10

### Konstruktion des Plasmides pADBTNF-BP für die Expression der löslichen Form von TNF-BP

Für eine weitere Variante eines Expressionsplasmides für die Produktion von sekretiertem TNF-BP wurde die 5'-nicht kodierende Region der TNF-R cDNA gegen die 5'-nicht kodierende Region der humanen β-Globin mRNA ausgetauscht. Der Grund dafür war die Feststellung, daß die Nukleotidsequenz unmittelbar vor dem Translationsstartcodon der TNF-R Sequenz deutlich von der für effiziente Expression eukaryotischer Gene gefundenen Konsensussequenz (Kozak,1987) abweicht, wogegen die 5'-nicht kodierende Region der β-Globin mRNA sehr gut mit dieser Konsensussequenz übereinstimmt (Lawn et al., 1980). Mittels des Oligonukleotids EBI-2452 (5'-CACAGTCGACTTACATTTGCTTCTGACACAACTGTGTTCACTAGCAACCTCAAAC AGACACCATGGGCCTCTCCACCGTGC-3'), das nach einer SalI Restriktionsschnittstelle die authentische 5'-nicht kodierende Sequenz, entsprechend der humanen β-Globin mRNA-Sequenz, gefolgt von 20 Basen der kodierenden Region von TNF-BP enthielt, wurde in einer PCR die TNF-R Sequenz modifiziert. 100 ng mit EcoRI linearisiertes Plasmid pTNF-BP wurden in 100 µl Reaktionsansatz mit je 50 pmol der Oligonukleotide EBI-2452 und EBI-1922 (Antisense, 5'-GAGGCTGCAATTGAAGC-3';bindet an die huTNF-R Sequenz bei Pos. 656) in 20 PCR-Zyklen (40 sec bei 94°C, 45 sec bei 55°C, 90 sec bei 72°C) amplifiziert. Nach Reinigung des PCR-Produktes durch Extraktion mit Phenol-Chloroform und Ethanolfällung wurde die DNA mit SalI und BglII doppelt geschnitten und das entstandene 0,51 kb DNA Fragment aus einem Agarosegel isoliert. Der entsprechende Teil der TNF-R Sequenz wurde aus dem Plasmid pTNF-BP durch Schneiden mit SalI und BglII entfernt, der 3,1 kb lange Plasmidteil aus einem Agarosegel isoliert und mit dem 0,51 kb langen PCR-Produkt ligiert. Nach Transformation von E.coli wurden sieben der erhaltenen Plasmide sequenziert. Eines dieser Plasmide enthielt genau die gewünschte Sequenz. Dieses Plasmid wurde pBTNF-BP benannt.

Das gesamte SalI - EcoRI Insert von pBTNF-BP wurde in das ebenso geschnittene Expressionsplasmid pAD-CMV1 kloniert und das erhaltene Plasmid pADBTNF-BP benannt (Fig.7B).

### Beispiel 11

### Isolierung von Ratten TNF-R cDNA Klonen

Zunächst wurde eine Rattenhirn-cDNA analog der HS913T cDNA Bibliothek (vgl. Beispiel 4) aus der Ratten Glia Tumor Zellinie C6 (ATCC Nr. CCL107) in λ-gt11 hergestellt.

600.000 Phagen der Rattenhirn cDNA Bibliothek in λ-gt11 wurden, wie in Beispiel 6 beschrieben, durch Hybridisierung gescreent. Als Sonde wurde das gereinigte EcoRI Insert von pTNF-BP30 (vgl.Beispiel 6) verwendet. Etwa 100 ng DNA wurden mit 1 µg Random Hexamer Primer anstatt der spezifischen Oligonukleotide, wie in Beispiel 6 beschrieben, mit [α-³²P]dCTP radioaktiv markiert. Eingebaut wurden 25x10⁶ cpm. Die Hybridisierung der Filter erfolgte unter gleichen Bedingungen wie in Beispiel 6. Die Filter wurden zweimal 30 Minuten bei Raumtemperatur in 2xSSC/0,1% SDS und dreimal 30 Minuten bei 65°C in 2xSSC/0,1%SDS und zweimal 30 Minuten bei 65°C in 0,5xSSC/0,5%SDS gewaschen. Die luftgetrockneten Filter wurden anschließend an Kodak XAR Röntgenfilm 16 Stunden unter Verwendung einer Verstärkerfolie bei -70°C exponiert. Insgesamt wurden 10 hybridisierende Plaques identifiziert und durch Plaquereinigung vereinzelt. Nach dreimaliger Plaquereinigung wurden schließlich drei λ-Klone (λ-raTNF-R #3, 4, 8) vereinzelt und die Phagen DNA, wie beschrieben, dargestellt.

Die Länge der cDNA Inserts wurde nach Schneiden der λ-DNA mit EcoRI und Auftrennung in einem Agarosegel mit 2,2 kb für die Klone raTNF-R3 und raTNF-R8 und 2,1 kb für Klon raTNF-R4 bestimmt. Die EcoRI Inserts der Klone λraTNF-R3 und 8 wurden in ebenso geschnittenen M13mp19 kloniert und die DNA Sequenz mit universellen Sequenzierprimern und spezifisch synthetisierten Oligonukleotidprimern bestimmt.

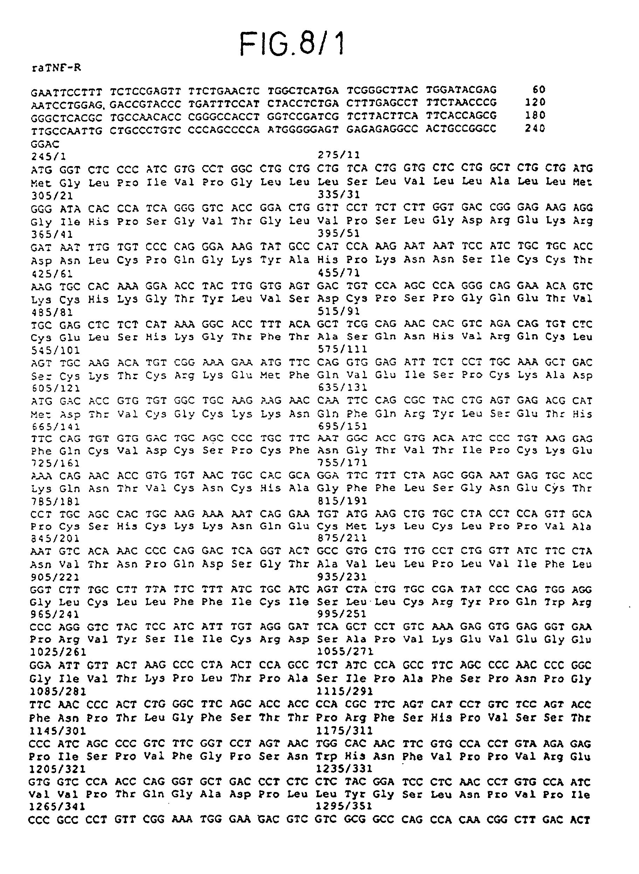

Die vollständige Nukleotidsequenz von raTNF-R8 ist in Fig.8 dargestellt. Die ersten und letzten sieben Basen entsprechen den EcoRI-Linkern, die bei der Herstellung der cDNA-Bibliothek angefügt worden waren.

### Beispiel 12

### Isolierung eines Klons, enthaltend die vollständige für den humanen TNF-Rezeptor kodierende cDNA

Die vollständige cDNA des Ratten TNF-R erleichterte die Suche nach dem noch fehlenden 3'-Teil der humanen TNF-R cDNA.

Als Sonde für die Hybridisierung wurde das 0,4 kb lange PCR-Produkt der Primer EBI-2316 (5'-ATTCGTGCGGCGCCTAG-3'; bindet an TNF-R mit 2.Base von EcoRI, an der TNF-R cDNA abbricht) und EBI-2467 (5'-GTCGGTAGCACCAAGGA-3'; bindet ca. 400 Basen vor poly-A an cDNA-Klon, entspricht Pos. 1775 in raTNF-R) mit λraTNF-R8 als Vorlage eingesetzt. Dieses DNA Fragment entspricht der Region der Ratten TNF-R cDNA, von der angenommen wurde, daß sie der, welche der internen EcoRI Stelle im humanen TNF-R folgt, entspricht.

2,5x10⁶ cpm der raTNF-R Sonde wurden eingesetzt, um 600.000 Plaques der HS913T cDNA-Bibliothek zu hybridisieren. Die Hybridisierbedingungen entsprachen den in Beispiel 6 angegebenen. Die Filter wurden zweimal 30 Minuten bei Raumtemperatur in 2xSSC/0,1%SDS und zweimal 30 Minuten bei 65°C in 2xSSC/0,1%SDS gewaschen, an der Luft getrocknet und an Kodak XAR Röntgenfilm unter Verwendung einer Verstärkerfolie 3 Tage bei -70°C exponiert. Sechs positive Plaques wurden identifiziert, in zwei weiteren Runden Plaques gereinigt und λ-DNA dargestellt (λ-TNF-R #2, 5, 6, 8, 11, 12). Nach Schneiden der λ-DNA mit EcoRI wiesen alle Klone eine DNA Bande mit etwa 0,8 kb Länge auf. λTNF-R2 und 11 enthielten zusätzlich ein EcoRI Fragment mit 1,3 kb. Die beiden EcoRI Inserts aus λTNF-R2 wurden in die EcoRI Stelle von Plasmid pUC218 (IBI) subkloniert und anschließend sequenziert. Die Sequenz des 1,3 kb EcoRI Fragments entsprach der von cDNA Klon pTNF-BP15, das 0,8 kb EcoRI Fragment entspricht dem 3'-Abschnitt der TNF-R mRNA und enthält vor der EcoRI-Linker Sequenz einen poly-A Schwanz mit 16 A Resten. λTNF-R2 enthält demnach die vollständige kodierende Region des humanen TNF-R, dargestellt in Fig.9.

### Beispiel 13

### Konstruktion der Plasmide pADTNF-R und pADBTNF-R für die Expression des gesamten humanen TNF-Rezeptors

Zunächst wurde, ähnlich wie im Beispiel 9 für pTNF-BP bzw. pADTNF-BP beschrieben, ein Plasmid konstruiert, in dem die 5'-nicht kodierende Region von pTNF-BP15 verkürzt, im Unterschied zu den im Beispiel 9 beschriebenen Plasmiden jedoch das 3'-Ende von pTNF-BP15 beibehalten wurde. Dazu wurde unter identischen Bedingungen wie in Beispiel 9 mit dem Oligonukleotid EBI-1986 und dem M13 -40 Universalprimer (5'-GTTTTCCCAGTCACGAC-3') pTNF-BP15 mit PCR amplifiziert. Das PCR Produkt wurde mit BamHI und EcoRI doppelt geschnitten und in das Plasmid pT7/T3α-19 kloniert. Eines der erhaltenen Plasmide wurde pTNF-BP15B benannt.

pTNF-BP15B wurde mit BamHI und EcoRI geschnitten und das 1,26 kb DNA Insert in mit BamHI und EcoRI geschnittenes Expressionsplasmid pAD-CMV1 kloniert. Ein erhaltenes Plasmid der gewünschten Zusammensetzung wurde pADTNF-BP15 benannt.

Dieses Plasmid wurde mit EcoRI linearisiert und in die Schnittstelle das 0,8 kb EcoRI Fragment, isoliert aus λTNF-R2, kloniert. Nach Transformation von E.coli wurden einige wahllos isolierte Plasmide durch Schneiden mit verschiedenen Restriktionsenzymen auf die korrekte Orientierung des eingesetzten EcoRI Fragments überprüft. Ein Plasmid, bezeichnet als pADTNF-R (Fig.7C), wurde noch genauer auf korrekte Orientierung untersucht, indem das Insert ausgehend vom 3'-Ende der insertierten cDNA mit dem Oligonukleotid EBI-2112 (5'-GTCCAATTATGTCACACC-3'), das nach der Multiklonierstelle an das Plasmid pAD-CMV1 und seine Derivate bindet, sequenziert wurde.

Ein weiteres Expressionsplasmid, in dem die 5'-nicht kodierende Region des TNF-R gegen die von β-Globin ausgetauscht ist, wurde konstruiert. Plasmid pADBTNF-BP wurde mit BglII vollständig geschnitten, um das 1,1 kb BglII Fragment zu entfernen, die DNA-Enden wurden nachfolgend mit alkalischer Phosphatase aus Kälberdarm dephosphoryliert und der Plasmidvektor (5,9 kb) mit der β-Globin 5'-nicht kodierenden Region des β-Globingens und dem 5'-Teil der TNF-R kodierenden Region aus einem Agarosegel isoliert. Plasmid pADTNF-R wurde mit BglII geschnitten und das 2,5 kb DNA Fragment, enthaltend den 3'-Abschnitt der TNF-R cDNA bis zur Promoterregion des nachfolgenden DHFR-Gens, aus einem Agarosegel isoliert und in den zuvor präparierten Plasmidvektor kloniert. Ein nach Transformation von E.coli erhaltenes Plasmid mit dem in korrekter Orientierung insertierten BglII-Fragment wurde pADBTNF-R benannt (Fig.7D).

### Beispiel 14

### Expression von löslichem TNF-BP in eukaryotischen Zellinien

### a) ELISA-Test

In diesem Beispiel wurde der Nachweis von TNF-BP mittels ELISA-Test wie folgt durchgeführt:

96-Napf Mikrotiterplatten wurden pro Napf mit 50 µl 1:3000 verdünntem polyklonalem Kaninchen-Serum (polyklonale Kaninchenantikörper, hergestellt durch Präzipitation von Antiserum mit Ammoniumsulfat, Endkonzentration 50 % Sättigung) gegen natürliches TNF-BP 18 Stunden bei 4°C beschichtet, einmal mit 0,05% Tween-20 in PBS gewaschen und freie Bindungsstellen mit 150-200 µl 0,5% Rinderserumalbumin, 0,05% Tween-20 in PBS (PBS/BSS/Tween) eine Stunde bei Raumtemperatur blockiert. Die Näpfe wurden einmal mit 0,05% Tween-20 in PBS gewaschen und 50 µl Zellüberstand oder bekannte Mengen von natürlichem TNF-BP (vgl. Tab.3 und 4) und 50 µl einer 1:10.000-fachen Verdünnung eines polyklonalen Mäuseserums gegen TNF-BP aufgetragen und zwei Stunden bei Raumtemperatur inkubiert. Anschließend wurden die Näpfe dreimal mit 0,05% Tween-20 in PBS gewaschen und 50 µl Kaninchen anti-Maus Ig-Peroxidase Konjugat (Dako P161; 1:5000 in PBS/BSA/Tween), zugegeben und weitere zwei Stunden bei Raumtemperatur inkubiert. Die Näpfe wurden dreimal mit Tween/PBS gewaschen und die Färbereaktion mit Orthophenylendiamin (3 mg/ml) und Na-Perborat (1 mg/ml) in 0,067M Kalium-Citrat pH 5,0, 100 µl/Napf, 20 Minuten bei Raumtemperatur unter Lichtschutz durchgeführt. Nach Zugabe von 100 µl 4N H₂SO₄ wurde die Farbintensität bei einer Wellenlänge von 492 nm in einer Mikrofilmplatten-Photometer photometrisch gemessen.

### b) Transiente Expression von löslichem TNF-BP in eukaryotischen Zellinien

Etwa 10⁶ Zellen (COS-7) pro 80 mm Petrischale wurden 24 Stunden vor der Transfektion in RPMI-1640 Medium mit 10% hitzeinaktiviertem fötalem Kälberserum angesetzt und bei 37°C in 5% CO₂ Atmosphäre inkubiert. Die Zellen wurden mit einem Gummischaber von der Petrischale gelöst und 5 Minuten bei 1200 UpM bei Raumtemperatur abzentrifugiert (Heraeus Minifuge, Ausschwing-Rotor 3360), einmal mit 5 ml serumfreiem Medium gewaschen, 5 Minuten bei 1200 UpM zentrifugiert und in 1 ml Medium, versetzt mit 250 µg/ml DEAE-Dextran und 10 µg Plasmid DNA (vgl. Tab.3, gereinigt durch zweimalige CsCl Dichtegradientenzentrifugation) suspendiert. Die Zellen wurden 40 Minuten bei 37°C inkubiert, einmal mit 5 ml Medium mit 10% Kälberserum gewaschen und in 5 ml Medium mit 100 µg/ml Chloroquin suspendiert. Die Zellen wurden eine Stunde bei 37°C inkubiert, einmal mit Medium gewaschen und mit 10 ml frischem Medium bei 37°C inkubiert. Nach 72 Stunden wurde der Zellüberstand geerntet und zum Nachweis des sekretierten TNF-BP verwendet.

**Tabelle 3:**

| Zellinie | COS-7 |
|---|---|
| ohne Plasmid | < 5 ng/ml |
| pADTNF-BP | 7,5 ng/ml |
| pADBTNF-BP | 146 ng/ml |

### c) Herstellung permanent TNF-BP produzierender Zellinien

Die Dihydrofolatreduktase(DHFR)-defiziente Hamster Ovarial Zellinie CHO DUKX BII (Urlaub und Chasin, 1980) wurde mit Plasmid pADBTNF-BP mittels Kalzium-Phosphat Präzipitation transfiziert (Current protocols in molecular biology, 1987). Vier dicht bewachsene Zellkultur-Fläschchen (25 cm², 5 ml Kulturmedium pro Fläschchen) wurden mit je 5 µg DNA transfiziert; nach vierstündiger Inkubation bei 37°C wurde das Medium entfernt und durch je 5 ml Selektionsmedium (MEM alpha Medium mit 10 % dialysiertem fötalem Rinderserum) ersetzt. Nach Inkubation über Nacht wurden die Zellen mittels Trypsin-Lösung abgelöst; die Zellen aus jedem Fläschchen wurden in zwei 96-Napf Gewebekulturplatten aufgeteilt (100 µl/Napf in Selektionsmedium). In etwa wöchentlichen Abständen wurde frisches Medium zugegeben. Nach etwa vier Wochen konnten in 79 Näpfen Zellklone beobachtet werden. Die Überstände wurden im ELISA auf TNF-BP Aktivität getestet. 37 Überstände zeigten Aktivität im ELISA. Die Ergebnisse des ELISA-Tests einiger posiver Klone ist in Tab.4 dargestellt.

**Tabelle 4:**

| Probe | | Absorption bei 492 nm |
|---|---|---|
| TNF-BP Standard | | |
| 1 ng/ml | | 0,390 |
| 10 ng/ml | | 1,233 |
| 100 ng/ml | | 1,875 |
| Kulturmedium (Negativkontrolle) 0,085 | | |
| Klon | A1G3 | 0,468 |
| | A2F5 | 0,931 |
| | A3A12 | 0,924 |
| | A4B8 | 0,356 |
| | A5A12 | 0,806 |
| | A5B10 | 0,915 |
| | A5C1 | 0,966 |

### Beispiel 15:

### RNA Analyse (Northern Blot) des humanen TNF-Rezeptors

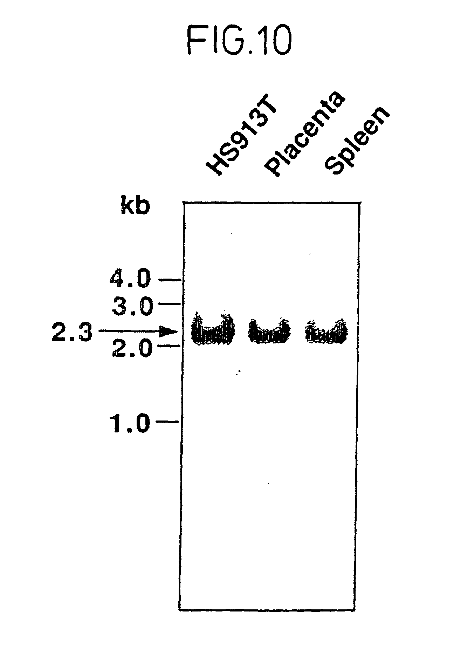

Je 1 µg poly-A⁺ RNA (isoliert aus HS913T (Fibrosarkom), Plazenta und Milz wurden in einem 2,5%igen vertikalen Agarosegel (10mM Na-Phosphatpuffer pH=7,0, 6,7 % Formaldehyd) elektrophoretisch aufgetrennt. Als GröBenmarker wurde eine durch Einfüllreaktion mit (α-³²P)dCTP und Klenow-Enzym radioaktiv markierte Kilobasenleiter (Bethesda Research Laboratories) verwendet. Das Formaldehyd wurde durch Wässern aus dem Gel entfernt und die RNA in 20x SSC auf eine Nylon-Membran (Genescreen plus, NEN-DuPont) transferiert. Die RNA wurde durch UV-Bestrahlung (100 Sekunden) auf der Membran kovalent gebunden. Die Membran wurde 2 Stunden bei 65°C in Church-Puffer (Church and Gilbert, 1984), (0,5 M Na-Phosphat pH=7,2, 7 % SDS, 1 mM EDTA) prähybridisiert und 19 Stunden bei 65°C in frischem Church-Puffer mit 3x10⁶ cpm P-32 markierter DNA Sonde (EcoRI Insert von pTNF-BP30) hybridisiert. Das Filter wurde dreimal 10 Minuten bei Raumtemperatur in Waschpuffer (40 mM Na-Phosphat pH=7,2, 1 % SDS) und anschließend viermal 30 Minuten bei 65°C in Waschpuffer gewaschen und 18 Stunden auf Kodak XAR Röntgenfilm unter Verwendung einer Verstärkerfolie bei -70°C exponiert.
Das Autoradiogramm (Fig.10) zeigt eine singuläre RNA Bande einer Länge von 2,3 kb für den humanen TNF-Rezeptor in den analysierten Geweben bzw. der Zellinie HS913T.

### Beispiel 16:

### Expression des TNF-Rezeptors

Für die transiente Expression wurden 5 - 10x10⁷ COS-7 Zellen 40 Minuten lang mit 10 µg pADTNF-R-Plasmid-DNA in einer Lösung enthaltend 250 µg/ml DEAE Dextran und 50 µg/ml Chloroquin inkubiert. Als Kontrolle wurde pADCMV-1-DNA verwendet. Nach der Transfektion wurden die Zellen gewaschen und anschließend 48 h lang gezüchtet. Die Expression des TNF-Rezeptors wurde durch Bindung von ¹²⁵I-TNF nachgewiesen. Für die Bindungstests wurden die Zellen gewaschen, 1 h lang bei 4°C mit 10 ng ¹²⁵I-TNF (spezifische Radioaktivität 38.000 cpm/ng) mit oder ohne 200fachen Überschuß an unmarkiertem TNF inkubiert, gewaschen, und die an die Zellen gebundene Radioaktivität in einem Gamma-Zähler gemessen. Die spezifische Bindung betrug bei der Kontrollprobe 2062 cpm, bei den mit TNF-Rezeptor DNA transformierten Proben 6150 cpm (die Werte sind ausgedrückt als mittlere gebundene cpm; die Standardabweichung, bestimmt aus Parallelversuchen, ist berücksichtigt. Unspezifischer Untergrund in Anwesenheit von unmarkiertem TNF wurde von den Werten abgezogen).

### Literatur:

Aggarwal, B.B., et al., 1985, Nature 318, 655-667
Beutler B., et al., 1988, Ann. Rev. Biochem. 57, 505-18
Beutler, B., et al., 1985, Nature 316, 552 - 554
Boshart, M., et al., 1985, Cell 41, 521-530
Carswell, E.A., et al., 1975, Proc.Natl.Acad.Sci.25, 3666-3670
Cerami, A., et al., 1988, Immunol. Today 9, 28-31
Church und Gilbert, 1984, Proc.Natl.Acad.Sci. 81, 1991-1995
Creasey, A.A., et al., 1987, Proc.Natl.Acad.Sci. 84, 3293-3297
Engelmann, H., et al., 1990, J.Biol.Chem.265, 1531-1536
Frohman, M.A., et al., 1988, Proc.Natl.Acad.Sci. 85, 8998-9002
Gullberg, U., et al., 1987, Eur. J. Haematol. 39, 241-251
Hsieng, S.L., et al., 1988, J. Chromatography 447, 351-364
Klebanoff, S.J., et al., 1986, J. Immunol. 136, 4220-4225
Kozak M., 1987,Nucleic Acids Res. 15, 8125-8148
Laemmli, U.K., 1970, Nature 227, 680-4
Lawn, et al., 1980, Cell 21, 647-651
Locksley, R.M., et al. 1987. J.Imunol. 139, 1891-1895
Mahoney, J.R., et al., 1985, J. Immunol. 134, 1673-1675
Maniatis, T., et al., 1982, Molecular Cloning A laboratory Manual. Cold Spring Harbor Laboratory, 474
McGrogan, M., et al., 1985, J.Biol.Chem. 260, 2307-2314
Mestan, J., et al., 1986, Nature 323, 816-819
Mitchell, P.J., et al., 1986, Mol.Cell.Biol. 6, 425-440
Oakley, B.R., et al., 1986, Analyt. Biochem. 105, 361-363
Old, L.J., 1987, Nature 326, 330-331
Oliff A., et al., 1987, Cell 555-63
Olsson I., et al., 1988, Eur.J. Haematol. 41, 414 - 420
Olsson I., et al., 1989, Eur.J. Haematol. 42, 270 - 275
Pieler Ch., 1987, Dissertation, Universität Wien
Piguet, P.F., et al., 1987, Immunobiol. 175, 27
Saiki, R.K., 1988, Science 239, 487-491
Sanger et al., 1977, Proc.Natl.Acad.Sci. 74, 5463-5467
Seckinger P., et al., 1988, J. Exp. Med., 1511-16
Seckinger, P., et al., 1987, J. Immunol. 139, 1546-1549
Seed und Aruffo, 1987, Proc.Natl.Acad.Sci. 84, 8573-8577
Seed, B., 1987, Nature 329, 840-842
Shalaby, M.R., et al., 1985, J. Immunol. 135, 2069-2073
Short, J.M., et al., 1988, Nucl.Acids Res.ll, 5521-5540
Staden, R., 1982, Nucleic Acid Res. 10, 4731-4751
Stauber, G.B., et al., 1988, J.Biolog.Chem. 35, Vol.263, 19098-19104
Stauber, G.B., et al., 1989, J.Biolog.Chem. 6, Vol.264, 3573-3576
Torti, F.M. et al., 1985, Nature 229:867-869
Tracey, K.J., et al., 1987, Nature 330, 662-666
Tracey, K.J., et al., 1986, Science 234, 470-474
Urlaub und Chasin, 1980, Proc.Natl.Acad.Sci. 77, 4216-4220
Waage, A., et al., 1987, Lancet. ii, 355-357
Wong, G.H.W., et al., 1986, Nature 323, 819-822

## Patentansprüche (Patentansprüche für folgende(n) Vertragsstaat(en): AT, BE, CH, DE, DK, FR, GB, IT, LI, LU, NL, SE)

1. DNA, kodierend für ein Polypeptid mit der Fähigkeit, TNF zu binden, wobei das Polypeptid eine Aminosäuresequenz mit der Formel 
asp ser val cys pro gln gly lys tyr ile his pro
gln asn asn ser ile cys cys thr lys cys his lys
gly thr tyr leu tyr asn asp cys pro gly pro gly
gln asp thr asp cys arg glu cys glu ser gly ser
phe thr ala ser glu asn his leu arg his cys leu
ser cys ser lys cys arg lys glu met gly gln val
glu ile ser ser cys thr val asp arg asp thr val
cys gly cys arg lys asn gln tyr arg his tyr trp
ser glu asn leu phe gln cys phe asn cys ser leu
cys leu asn gly thr val his leu ser cys gln glu
lys gln asn thr val cys thr cys his ala gly phe
phe leu arg glu asn glu cys val ser cys ser asn
cys lys lys ser leu glu cys thr lys leu cys leu
pro gln ile glu asn
 oder ein funktionelles Fragment oder eine Modifikation davon mit der Fähigkeit, TNF zu binden, umfasst.

2. DNA nach Anspruch 1, **dadurch gekennzeichnet, dass** das Polypeptid eine Aminosäuresequenz mit der Formel 
leu val pro his leu gly asp arg glu lys arg
asp ser val cys pro gln gly lys tyr ile his pro
gln asn asn ser ile cys cys thr lys cys his lys
gly thr tyr leu tyr asn asp cys pro gly pro gly
gln asp thr asp cys arg glu cys glu ser gly ser
phe thr ala ser glu asn his leu arg his cys leu
ser cys ser lys cys arg lys glu met gly gln val
glu ile ser ser cys thr val asp arg asp thr val
cys gly cys arg lys asn gln tyr arg his tyr trp
ser glu asn leu phe gln cys phe asn cys ser leu
cys leu asn gly thr val his leu ser cys gln glu
lys gln asn thr val cys thr cys his ala gly phe
phe leu arg glu asn glu cys val ser cys ser asn
cys lys lys ser leu glu cys thr lys leu cys leu
pro gln ile glu asn
 oder ein funkionelles Fragment oder eine Modifikation davon mit der Fähigkeit, TNF zu binden, umfasst.

3. DNA nach Anspruch 2, **dadurch gekennzeichnet, dass** das Polypeptid eine Aminosäuresequenz mit der Formel 
met gly leu ser thr val pro asp leu leu leu pro
leu val leu leu glu leu leu val gly ile tyr pro
ser gly val ile gly leu val pro his leu gly asp
arg glu lys arg asp ser val cys pro gln gly lys
tyr ile his pro gln asn asn ser ile cys cys thr
lys cys his lys gly thr tyr leu tyr asn asp cys
pro gly pro gly gln asp thr asp cys arg glu cys
glu ser gly ser phe thr ala ser glu asn his leu
arg his cys leu ser cys ser lys cys arg lys glu
met gly gln val glu ile ser ser cys thr val asp
arg asp thr val cys gly cys arg lys asn gln tyr
arg his tyr trp ser glu asn leu phe gln cys phe
asn cys ser leu cys leu asn gly thr val his leu
ser cys gln glu lys gln asn thr val cys thr cys
his ala gly phe phe leu arg glu asn glu cys val
ser cys ser asn cys lys lys ser leu glu cys thr
lys leu cys leu pro gln ile glu asn
 oder ein funktionelles Fragment oder eine Modifikation davon mit der Fähigkeit, TNF zu binden, umfasst.

4. DNA nach Anspruch 3, **dadurch gekennzeichnet, dass** das Polypeptid eine Aminosäuresequenz mit der Formel 
met gly leu ser thr val pro asp leu leu leu pro
leu val leu leu glu leu leu val gly ile tyr pro
ser gly val ile gly leu val pro his leu gly asp
arg glu lys arg asp ser val cys pro gln gly lys
tyr ile his pro gln asn asn ser ile cys cys thr
lys cys his lys gly thr tyr leu tyr asn asp cys
pro gly pro gly gln asp thr asp cys arg glu cys
glu ser gly ser phe thr ala ser glu asn his leu
arg his cys leu ser cys ser lys cys arg lys glu
met gly gln val glu ile ser ser cys thr val asp
arg asp thr val cys gly cys arg lys asn gln tyr
arg his tyr trp ser glu asn leu phe gln cys phe
asn cys ser leu cys leu asn gly thr val his leu
ser cys gln glu lys gln asn thr val cys thr cys
his ala gly phe phe leu arg glu asn glu cys val
ser cys ser asn cys lys lys ser leu glu cys thr
lys leu cys leu pro gln ile glu asn val lys gly
thr glu asp ser gly thr thr val leu leu pro leu
val ile phe phe gly leu cys leu leu ser leu leu
phe ile gly leu met tyr arg tyr gln arg trp lys
ser lys leu tyr ser ile val cys gly lys ser thr
pro glu lys glu gly glu leu glu gly thr thr thr
lys pro leu ala pro asn pro ser phe ser pro thr
pro gly phe thr pro thr leu gly phe ser pro val
pro ser ser thr phe thr ser ser ser thr tyr thr
pro gly asp cys pro asn phe ala ala pro arg arg
glu val ala pro pro tyr gln gly ala asp pro ile
leu ala thr ala leu ala ser asp pro ile pro asn
pro leu gln lys trp glu asp ser ala his lys pro
gln ser leu asp thr asp asp pro ala thr leu tyr
 ala val val glu asn val pro pro leu arg trp oder ein funktionelles Fragment oder eine Modifikation davon mit der Fähigkeit, TNF zu binden, umfasst.

5. DNA nach Anspruch 1, **dadurch gekennzeichnet, dass** das Polypeptid eine Aminosäuresequenz mit der Formel 
asp ser val cys pro gln gly lys
tyr ile his pro gln asn asn ser ile cys cys thr
lys cys his lys gly thr tyr leu tyr asn asp cys
pro gly pro gly gln asp thr asp cys arg glu cys
glu ser gly ser phe thr ala ser glu asn his leu
arg his cys leu ser cys ser lys cys arg lys glu
met gly gln val glu ile ser ser cys thr val asp
arg asp thr val cys gly cys arg lys asn gln tyr
arg his tyr trp ser glu asn leu phe gln cys phe
asn cys ser leu cys leu asn gly thr val his leu
ser cys gln glu lys gln asn thr val cys thr cys
his ala gly phe phe leu arg glu asn glu cys val
ser cys ser asn cys lys lys ser leu glu cys thr
lys leu cys leu pro gln ile glu asn val lys gly
thr glu asp ser gly thr thr
 oder ein funktionelles Fragment oder eine Modifikation davon mit der Fähigkeit, TNF zu binden, umfasst.

6. DNA nach Anspruch 5, **dadurch gekennzeichnet, dass** das Polypeptid eine Aminosäuresequenz mit der Formel 
leu val pro his leu gly asp
arg glu lys arg asp ser val cys pro gln gly lys
tyr ile his pro gln asn asn ser ile cys cys thr
lys cys his lys gly thr tyr leu tyr asn asp cys
pro gly pro gly gln asp thr asp cys arg glu cys
glu ser gly ser phe thr ala ser glu asn his leu
arg his cys leu ser cys ser lys cys arg lys glu
met gly gln val glu ile ser ser cys thr val asp
arg asp thr val cys gly cys arg lys asn gln tyr
arg his tyr trp ser glu asn leu phe gln cys phe
asn cys ser leu cys leu asn gly thr val his leu
ser cys gln glu lys gln asn thr val cys thr cys
his ala gly phe phe leu arg glu asn glu cys val
ser cys ser asn cys lys lys ser leu glu cys thr
lys leu cys leu pro gln ile glu asn val lys gly
thr glu asp ser gly thr thr
 oder ein funktionelles Fragment oder eine Modifikation davon mit der Fähigkeit, TNF zu binden, umfasst.

7. DNA nach Anspruch 1, **dadurch gekennzeichnet, dass** die Aminosäuresequenz des Polypeptids an einer oder mehreren Glykosylierungsstellen verändert ist.

8. DNA nach Anspruch 1, kodierend für sekretierbares TNF-bindendes Protein, **dadurch gekennzeichnet, dass** sie eine Nukleinsäuresequenz mit der Formel 
R= GAT AGT GTG TGT CCC CAA GGA AAA TAT ATC CAC
CCT CAA AAT AAT TCG ATT TGC TGT ACC AAG TGC CAC
AAA GGA ACC TAC TTG TAC AAT GAC TGT CCA GGC CCG
GGG CAG GAT ACG GAC TGC AGG GAG TGT GAG AGC GGC
TCC TTC ACC GCT TCA GAA AAC CAC CTC AGA CAC TGC
CTC AGC TGC TCC AAA TGC CGA AAG GAA ATG GGT CAG
GTG GAG ATC TCT TCT TGC ACA GTG GAC CGG GAC ACC
GTG TGT GGC TGC AGG AAG AAC CAG TAC CGG CAT TAT
TGG AGT GAA AAC CTT TTC CAG TGC TTC AAT TGC AGC
CTC TGC CTC AAT GGG ACC GTG CAC CTC TCC TGC CAG
GAG AAA CAG AAC ACC GTG TGC ACC TGC CAT GCA GGT
TTC TTT CTA AGA GAA AAC GAG TGT GTC TCC TGT AGT
AAC TGT AAG AAA AGC CTG GAG TGC ACG AAG TTG TGC
CTA CCC CAG ATT GAG AAT
 oder ein Fragment, eine Variante oder eine Modifikation dieser Nukleinsäuresequenz, die für ein Polypeptid mit der Fähigkeit, TNF zu binden, kodiert, umfast, wobei R² eine für ein *in vivo* abspaltbares (Poly)peptid kodierende DNA darstellt.

9. DNA nach Anspruch 8, kodierend für sekretierbares TNF-bindendes Protein,
**dadurch gekennzeichnet, dass** R² eine zur Gänze oder teilweise für eine Signalsequenz kodierende DNA darstellt.

10. DNA nach Anspruch 8, **dadurch gekennzeichnet, dass** R² eine Nukleinsäuresequenz mit der Formel CTG GTC CCT CAC CTA GGG GAC AGG GAG AAG AGA umfasst.

11. DNA nach Anspruch 9, **dadurch gekennzeichnet, dass** R² eine Nukleinsäure mit der Formel R^3 CTG GTC CCT CAC CTA GGG GAC AGG GAG AAG AGA umfasst, wobei R³ eine für ein Signalpeptid kodierende DNA darstellt.

12. DNA nach Anspruch 11, **dadurch gekennzeichnet, dass** R³ eine Nukleinsäuresequenz mit der Formel 
ATG GGC CTC TCC ACC GTG CCT GAC CTG CTG CTG CCA
CTG GTG CTC CTG GAG CTG TTG GTG GGA ATA TAC CCC
TCA GGG GTT ATT GGA
 umfasst.

13. DNA nach Anspruch 1, **dadurch gekennzeichnet, dass** sie DNA mit der Formel 
ATG GGC CTC TCC ACC GTG CCT GAC CTG CTG CTG CCA CTG GTG CTC
CTG GAG CTG TTG GTG GGA ATA TAC CCC TCA GGG GTT ATT GGA CTG
GTC CCT CAC CTA GGG GAC AGG GAG AAG AGA/GAT AGT GTG TGT CCC
CAA GGA AAA TAT ATC CAC CCT CAA AAT AAT TCG ATT TGC TGT ACC
AAG TGC CAC AAA GGA ACC TAC TTG TAC AAT GAC TGT CCA GGC CCG
GGG CAG GAT ACG GAC TGC AGG GAG TGT GAG AGC GGC TCC TTC ACC
GCT TCA GAA AAC CAC CTC AGA CAC TGC CTC AGC TGC TCC AAA TGC
CGA AAG GAA ATG GGT CAG GTG GAG ATC TCT TCT TGC ACA GTG GAC
CGG GAC ACC GTG TGT GGC TGC AGG AAG AAC CAG TAC CGG CAT TAT
TGG AGT GAA AAC CTT TTC CAG TGC TTC AAT TGC AGC CTC TGC CTC
 
AAT GGG ACC GTG CAC CTC TCC TGC CAG GAG AAA CAG AAC ACC GTG
TGC ACC TGC CAT GCA GGT TTC TTT CTA AGA GAA AAC GAG TGT GTC
TCC TGT AGT AAC TGT AAG AAA AGC CTG GAG TGC ACG AAG TTG TGC
CTA CCC CAG ATT GAG AAT GTT AAG GGC ACT GAG GAC TCA GGC ACC
ACA GTG CTG TTG CCC CTG GTC ATT TTC TTT GGT CTT TGC CTT TTA
TCC CTC CTC TTC ATT GGT TTA ATG TAT CGC TAC CAA CGG TGG AAG
TCC AAG CTC TAC TCC ATT GTT TGT GGG AAA TCG ACA CCT GAA AAA
GAG GGG GAG CTT GAA GGA ACT ACT ACT AAG CCC CTG GCC CCA AAC
CCA AGC TTC AGT CCC ACT CCA GGC TTC AGC CCC AGC GTG GGC TTG
AGT CCC GTG GCC AGT CCC ACC TTC AGC TCG AGC TCC AGC TAT ACC
GCC GGT GAC TGT CCG AAC TTT GCG GCT CCC CGC AGA GAG GTG GCA
CCA CCC TAT CAG GGG GCT GAC CCC ATC CTT GCG ACA GCC CTC GCC
TCC GAC CCC ATC CCC AAC CCC CTT CAG AAG TGG GAG GAC AGC GCC
CAC AAG CCA CAG AGC CTA GAC ACT GAT GAC CCC GCG ACG CTG TAC
GCC GTG GTG GAG AAC GTG CCC CCG TTG CGC TGG AA GGAATTC
 oder ein Fragment, eine Variante oder eine Modifikation dieser Nukleinsäuresequenz, die für ein Polypeptid mit der Fähigkeit, TNF zu binden, kodiert, umfasst.

14. Rekombinantes DNA-Molekül, **dadurch gekennzeichnet, dass** es die DNA nach einem der Ansprüche 1 bis 13 enthält.

15. Rekombinantes DNA-Molekül nach Anspruch 14, replizierbar in prokaryontischen oder eukaryontischen Wirtszellen, **dadurch gekennzeichnet, dass** es, funktionell verbunden mit der DNA nach einem der Ansprüche 1 bis 13, Expressionskontrollsequenzen enthält.

16. Rekombinantes DNA-Molekül nach Anspruch 15, replizierbar in Säugetierzellen.

17. Rekombinantes DNA-Molekül nach Anspruch 14, **dadurch gekennzeichnet, dass** es die in Anspruch 9 definierte DNA enthält.

18. Rekombinantes DNA-Molekül nach Anspruch 17 mit der Bezeichnung pADTNF-BP, gezeigt in Figur 7a.

19. Rekombinantes DNA-Molekül nach Anspruch 17 mit der Bezeichnung pADBTNF-BP, gezeigt in Figur 7b.

20. Rekombinantes DNA-Molekül nach Anspruch 17 mit der Bezeichnung pADTNF-R, gezeigt in Figur 7c.

21. Rekombinantes DNA-Molekül nach Anspruch 17 mit der Bezeichnung pADBTNF-R, gezeigt in Figur 7d.

22. Wirtszelle, enthaltend mindestens eines der in den Ansprüchen 14 bis 21 definierten rekombinanten DNA-Moleküle, das eine Teilsequenz der für das TNF-bindende Protein kodierenden DNA darstellt.

23. Wirtszelle nach Anspruch 22, **dadurch gekennzeichnet, dass** es eine eukaryontische Wirtszelle, bevorzugt eine COS-Zelle oder CHO-Zelle ist.

24. Verfahren zum Herstellen eines rekombinanten TNF-bindenden Proteines oder eines funktionellen Derivates davon, umfassend das Kultivieren eines Wirtes gemäß Anspruch 22 und Exprimieren des Proteines.

25. Verfahren nach Anspruch 24, umfassend weiter den Schritt des Isolierens des exprimierten Proteines.

26. Rekombinantes Polypeptid, **dadurch gekennzeichnet, dass** es von einer der DNA kodiert wird, die aus der Gruppe ausgewählt ist, die für eines der folgenden Polypeptide kodiert:

(i) leu val pro his leu gly asp arg glu lys arg
asp ser val cys pro gln gly lys tyr ile his pro
gln asn asn ser ile cys cys thr lys cys his lys
gly thr tyr leu tyr asn asp cys pro gly pro gly
gln asp thr asp cys arg glu cys glu ser gly ser
phe thr ala ser glu asn his leu arg his cys leu
ser cys ser lys cys arg lys glu met gly gln val
glu ile ser ser cys thr val asp arg asp thr val
cys gly cys arg lys asn gln tyr arg his tyr trp

ser glu asn leu phe gln cys phe asn cys ser leu
cys leu asn gly thr val his leu ser cys gln glu
lys gln asn thr val cys thr cys his ala gly phe
phe leu arg glu asn glu cys val ser cys ser asn
cys lys lys ser leu glu cys thr lys leu cys leu
pro gln ile glu asn ,

(ii)

met gly leu ser thr val pro asp leu leu leu pro
leu val leu leu glu leu leu val gly ile tyr pro
ser gly val ile gly leu val pro his leu gly asp
arg glu lys arg asp ser val cys pro gln gly lys
tyr ile his pro gln asn asn ser ile cys cys thr
lys cys his lys gly thr tyr leu tyr asn asp cys
pro gly pro gly gln asp thr asp cys arg glu cys
glu ser gly ser phe thr ala ser glu asn his leu
arg his cys leu ser cys ser lys cys arg lys glu
met gly gln val glu ile ser ser cys thr val asp
arg asp thr val cys gly cys arg lys asn gln tyr
arg his tyr trp ser glu asn leu phe gln cys phe
asn cys ser leu cys leu asn gly thr val his leu
ser cys gln glu lys gln asn thr val cys thr cys
his ala gly phe phe leu arg glu asn glu cys val
ser cys ser asn cys lys lys ser leu glu cys thr
lys leu cys leu pro gln ile glu asn

(iii) leu val pro his leu gly asp
arg glu lys arg asp ser val cys pro gln gly lys
tyr ile his pro gln asn asn ser ile cys cys thr
lys cys his lys gly thr tyr leu tyr asn asp cys
pro gly pro gly gln asp thr asp cys arg glu cys
glu ser gly ser phe thr ala ser glu asn his leu
arg his cys leu ser cys ser lys cys arg lys glu
met gly gln val glu ile ser ser cys thr val asp
arg asp thr val cys gly cys arg lys asn gln tyr
arg his tyr trp ser glu asn leu phe gln cys phe
asn cys ser leu cys leu asn gly thr val his leu
ser cys gln glu lys gln asn thr val cys thr cys
his ala gly phe phe leu arg glu asn glu cys val
ser cys ser asn cys lys lys ser leu glu cys thr
lys leu cys leu pro gln ile glu asn val lys gly
thr glu asp ser gly thr thr

27. Polypeptid, **dadurch gekennzeichnet, dass** es ein TNF-bindendes Protein der Formel: 
asp ser val cys pro gln gly lys tyr ile his pro
gln asn asn ser ile cys cys thr lys cys his lys

gly thr tyr leu tyr asn asp cys pro gly pro gly
gln asp thr asp cys arg glu cys glu ser gly ser
phe thr ala ser glu asn his leu arg his cys leu
ser cys ser lys cys arg lys glu met gly gln val
glu ile ser ser cys thr val asp arg asp thr val
cys gly cys arg lys asn gln tyr arg his tyr trp
ser glu asn leu phe gln cys phe asn cys ser leu
cys leu asn gly thr val his leu ser cys gln glu
lys gln asn thr val cys thr cys his ala gly phe
phe leu arg glu asn glu cys val ser cys ser asn
cys lys lys ser leu glu cys thr lys leu cys leu
pro gln ile glu asn
 umfasst.

28. Polypeptid nach Anspruch 27, **dadurch gekennzeichnet, dass** es den TNF-Rezeptor der Formel 
met gly leu ser thr val pro asp leu leu leu pro
leu val leu leu glu leu leu val gly ile tyr pro
ser gly val ile gly leu val pro his leu gly asp
arg glu lys arg asp ser val cys pro gln gly lys
tyr ile his pro gln asn asn ser ile cys cys thr
lys cys his lys gly thr tyr leu tyr asn asp cys
pro gly pro gly gln asp thr asp cys arg glu cys
glu ser gly ser phe thr ala ser glu asn his leu
arg his cys leu ser cys ser lys cys arg lys glu
met gly gln val glu ile ser ser cys thr val asp
arg asp thr val cys gly cys arg lys asn gln tyr
arg his tyr trp ser glu asn leu phe gln cys phe
asn cys ser leu cys leu asn gly thr val his leu
ser cys gln glu lys gln asn thr val cys thr cys
his ala gly phe phe leu arg glu asn glu cys val
ser cys ser asn cys lys lys ser leu glu cys thr
lys leu cys leu pro gln ile glu asn val lys gly
thr glu asp ser gly thr thr val leu leu pro leu
val ile phe phe gly leu cys leu leu ser leu leu
phe ile gly leu met tyr arg tyr gln arg trp lys
ser lys leu tyr ser ile val cys gly lys ser thr
pro glu lys glu gly glu leu glu gly thr thr thr
lys pro leu ala pro asn pro ser phe ser pro thr
pro gly phe thr pro thr leu gly phe ser pro val
pro ser ser thr phe thr ser ser ser thr tyr thr
pro gly asp cys pro asn phe ala ala pro arg arg
glu val ala pro pro tyr gln gly ala asp pro ile
leu ala thr ala leu ala ser asp pro ile pro asn
pro leu gln lys trp glu asp ser ala his lys pro
gln ser leu asp thr asp asp pro ala thr leu tyr
ala val val glu asn val pro pro leu arg trp
 umfasst.

29. Verwendung eines in den Ansprüchen 26 bis 28 definierten Polypeptids zum Untersuchen von Substanzen auf ihre Wechselwirkung mit diesem Polypeptid und/oder mit TNF-α und/oder mit TNF-β und/oder auf ihre Beeinflussung der biologischen Wirkung von TNF-α und/oder TNF-β *in vitro*.

30. Verwendung einer in Anspruch 24 definierten Wirtszelle zum Untersuchen von Substanzen auf ihre Wechselwirkung mit dem TNF-Rezeptor und/oder ihre Beeinflussung der biologischen Wirkung von TNF-α und/oder TNF-β.

31. Verwendung eines Polypeptides nach einem der Ansprüche 26 bis 28 zum Herstellen eines Medikaments zum Behandeln von Autoimmunerkrankungen.

32. Verwendung eines Polypeptides nach einem der Ansprüche 26 bis 28 zum Herstellen eines Medikaments zum Behandeln von rheumatoider Arthritis.

33. Verwendung eines Polypeptides nach einem der Ansprüche 26 bis 28 zum Herstellen eines Medikaments zum Behandeln von ARDS.

34. Verwendung eines Polypeptides nach einem der Ansprüche 26 bis 28 zum Herstellen eines Medikaments zum Behandeln von Anorexia/Cachexie.

35. Verwendung eines Polypeptides nach einem der Ansprüche 26 bis 28 zum Herstellen eines Medikaments zum Behandeln von GVHR.

36. Verwendung eines Polypeptides nach einem der Ansprüche 26 bis 28 zum Herstellen eines Medikaments zum Behandeln von Schock.

37. Verwendung eines Polypeptides nach einem der Ansprüche 26 bis 28 als Diagnostikum zur Bestimmung von TNF-α und/oder TNF-β.

38. Pharmazeutische Zubereitung, **dadurch gekennzeichnet, dass** sie als therapeutisch wirksame Komponente ein in einem der Ansprüche 26 bis 28 definiertes Polypeptid enthält.

## Patentansprüche (Patentansprüche für folgende(n) Vertragsstaat(en): ES)

1. Verfahren zur Herstellung einer DNA, umfassend die Amplifikation einer DNA, die für ein Polypeptid mit der Fähigkeit, TNF zu binden, kodiert, wobei das Polypeptid eine Aminosäuresequenz mit der Formel 
asp ser val cys pro gln gly lys tyr ile his pro
gln asn asn ser ile cys cys thr lys cys his lys
gly thr tyr leu tyr asn asp cys pro gly pro gly
gln asp thr asp cys arg glu cys glu ser gly ser
phe thr ala ser glu asn his leu arg his cys leu
ser cys ser lys cys arg lys glu met gly gln val
glu ile ser ser cys thr val asp arg asp thr val
cys gly cys arg lys asn gln tyr arg his tyr trp
ser glu asn leu phe gln cys phe asn cys ser leu
cys leu asn gly thr val his leu ser cys gln glu
lys gln asn thr val cys thr cys his ala gly phe
phe leu arg glu asn glu cys val ser cys ser asn
cys lys lys ser leu glu cys thr lys leu cys leu
pro gln ile glu asn
 oder ein funktionelles Fragment oder eine Modifikation davon mit der Fähigkeit, TNF zu binden, umfasst.

2. Verfahren nach Anspruch 1, **dadurch gekennzeichnet, dass** das Polypeptid eine Aminosäuresequenz mit der Formel 
leu val pro his leu gly asp arg glu lys arg
asp ser val cys pro gln gly lys tyr ile his pro
gln asn asn ser ile cys cys thr lys cys his lys
gly thr tyr leu tyr asn asp cys pro gly pro gly
gln asp thr asp cys arg glu cys glu ser gly ser
phe thr ala ser glu asn his leu arg his cys leu
ser cys ser lys cys arg lys glu met gly gln val
glu ile ser ser cys thr val asp arg asp thr val
cys gly cys arg lys asn gln tyr arg his tyr trp
ser glu asn leu phe gln cys phe asn cys ser leu
cys leu asn gly thr val his leu ser cys gln glu
lys gln asn thr val cys thr cys his ala gly phe
phe leu arg glu asn glu cys val ser cys ser asn
cys lys lys ser leu glu cys thr lys leu cys leu
pro gln ile glu asn
 oder ein funktionelles Fragment oder eine Modifikation davon mit der Fähigkeit, TNF zu binden, umfasst.

3. Verfahren nach Anspruch 2, **dadurch gekennzeichnet, dass** das Polypeptid eine Aminosäuresequenz mit der Formel 
met gly leu ser thr val pro asp leu leu leu pro
leu val leu leu glu leu leu val gly ile tyr pro
ser gly val ile gly leu val pro his leu gly asp
arg glu lys arg asp ser val cys pro gln gly lys
tyr ile his pro gln asn asn ser ile cys cys thr
lys cys his lys gly thr tyr leu tyr asn asp cys
pro gly pro gly gln asp thr asp cys arg glu cys
glu ser gly ser phe thr ala ser glu asn his leu
arg his cys leu ser cys ser lys cys arg lys glu
met gly gln val glu ile ser ser cys thr val asp
arg asp thr val cys gly cys arg lys asn gln tyr
arg his tyr trp ser glu asn leu phe gln cys phe
asn cys ser leu cys leu asn gly thr val his leu
ser cys gln glu lys gln asn thr val cys thr cys
his ala gly phe phe leu arg glu asn glu cys val
ser cys ser asn cys lys lys ser leu glu cys thr
lys leu cys leu pro gln ile glu asn
 oder ein funktionelles Fragment oder eine Modifikation davon mit der Fähigkeit, TNF zu binden, umfasst.

4. Verfahren nach Anspruch 3, **dadurch gekennzeichnet, dass** das Polypeptid eine Aminosäuresequenz mit der Formel 
met gly leu ser thr val pro asp leu leu leu pro
leu val leu leu glu leu leu val gly ile tyr pro
ser gly val ile gly leu val pro his leu gly asp
arg glu lys arg asp ser val cys pro gln gly lys
tyr ile his pro gln asn asn ser ile cys cys thr
lys cys his lys gly thr tyr leu tyr asn asp cys
pro gly pro gly gln asp thr asp cys arg glu cys
glu ser gly ser phe thr ala ser glu asn his leu
arg his cys leu ser cys ser lys cys arg lys glu
met gly gln val glu ile ser ser cys thr val asp
arg asp thr val cys gly cys arg lys asn gln tyr
arg his tyr trp ser glu asn leu phe gln cys phe
asn cys ser leu cys leu asn gly thr val his leu
ser cys gln glu lys gln asn thr val cys thr cys
his ala gly phe phe leu arg glu asn glu cys val
ser cys ser asn cys lys lys ser leu glu cys thr
lys leu cys leu pro gln ile glu asn val lys gly
thr glu asp ser gly thr thr val leu leu pro leu
val ile phe phe gly leu cys leu leu ser leu leu
phe ile gly leu met tyr arg tyr gln arg trp lys
ser lys leu tyr ser ile val cys gly lys ser thr
pro glu lys glu gly glu leu glu gly thr thr thr
lys pro leu ala pro asn pro ser phe ser pro thr
pro gly phe thr pro thr leu gly phe ser pro val
pro ser ser thr phe thr ser ser ser thr tyr thr
pro gly asp cys pro asn phe ala ala pro arg arg
glu val ala pro pro tyr gln gly ala asp pro ile
leu ala thr ala leu ala ser asp pro ile pro asn
pro leu gln lys trp glu asp ser ala his lys pro
gln ser leu asp thr asp asp pro ala thr leu tyr

ala val val glu asn val pro pro leu arg trp
 oder ein funktionelles Fragment oder eine Modifikation davon mit der Fähigkeit, TNF zu binden, umfasst.

5. Verfahren nach Anspruch 1, **dadurch gekennzeichnet, dass** das Polypeptid eine Aminosäuresequenz mit der Formel 
asp ser val cys pro gln gly lys
tyr ile his pro gln asn asn ser ile cys cys thr
lys cys his lys gly thr tyr leu tyr asn asp cys
pro gly pro gly gln asp thr asp cys arg glu cys
glu ser gly ser phe thr ala ser glu asn his leu
arg his cys leu ser cys ser lys cys arg lys glu
met gly gln val glu ile ser ser cys thr val asp
arg asp thr val cys gly cys arg lys asn gln tyr
arg his tyr trp ser glu asn leu phe gln cys phe
asn cys ser leu cys leu asn gly thr val his leu
ser cys gln glu lys gln asn thr val cys thr cys
his ala gly phe phe leu arg glu asn glu cys val
ser cys ser asn cys lys lys ser leu glu cys thr
lys leu cys leu pro gln ile glu asn val lys gly
thr glu asp ser gly thr thr
 oder ein funktionelles Fragment oder eine Modifikation davon mit der Fähigkeit, TNF zu binden, umfasst.

6. Verfahren nach Anspruch 5, **dadurch gekennzeichnet, dass** das Polypeptid eine Aminosäuresequenz mit der Formel 
leu val pro his leu gly asp
arg glu lys arg asp ser val cys pro gln gly lys
tyr ile his pro gln asn asn ser ile cys cys thr
lys cys his lys gly thr tyr leu tyr asn asp cys
pro gly pro gly gln asp thr asp cys arg glu cys
glu ser gly ser phe thr ala ser glu asn his leu
arg his cys leu ser cys ser lys cys arg lys glu
met gly gln val glu ile ser ser cys thr val asp
arg asp thr val cys gly cys arg lys asn gln tyr
arg his tyr trp ser glu asn leu phe gln cys phe
asn cys ser leu cys leu asn gly thr val his leu
ser cys gln glu lys gln asn thr val cys thr cys
his ala gly phe phe leu arg glu asn glu cys val
ser cys ser asn cys lys lys ser leu glu cys thr
lys leu cys leu pro gln ile glu asn val lys gly
thr glu asp ser gly thr thr
 oder ein funktionelles Fragment oder eine Modifikation davon mit der Fähigkeit, TNF zu binden, umfasst.

7. Verfahren nach Anspruch 1, **dadurch gekennzeichnet, dass** die Aminosäuresequenz des Polypeptids an einer oder mehreren Glykosylierungsstellen verändert ist.

8. Verfahren nach Anspruch 1, wobei die DNA für ein sekretierbares TNF-bindendes Protein kodiert, **dadurch gekennzeichnet, dass** die DNA eine Nukleinsäuresequenz mit der Formel 
R= GAT AGT GTG TGT CCC CAA GGA AAA TAT ATC CAC
CCT CAA AAT AAT TCG ATT TGC TGT ACC AAG TGC CAC
AAA GGA ACC TAC TTG TAC AAT GAC TGT CCA GGC CCG
GGG CAG GAT ACG GAC TGC AGG GAG TGT GAG AGC GGC
TCC TTC ACC GCT TCA GAA AAC CAC CTC AGA CAC TGC
CTC AGC TGC TCC AAA TGC CGA AAG GAA ATG GGT CAG
GTG GAG ATC TCT TCT TGC ACA GTG GAC CGG GAC ACC
GTG TGT GGC TGC AGG AAG AAC CAG TAC CGG CAT TAT
TGG AGT GAA AAC CTT TTC CAG TGC TTC AAT TGC AGC
CTC TGC CTC AAT GGG ACC GTG CAC CTC TCC TGC CAG
GAG AAA CAG AAC ACC GTG TGC ACC TGC CAT GCA GGT
TTC TTT CTA AGA GAA AAC GAG TGT GTC TCC TGT AGT
AAC TGT AAG AAA AGC CTG GAG TGC ACG AAG TTG TGC
CTA CCC CAG ATT GAG AAT
 oder ein Fragment, eine Variante oder eine Modifikation dieser Nukleinsäuresequenz, die für ein Polypeptid mit der Fähigkeit, TNF zu binden, kodiert, umfasst, wobei R² eine für ein *in vivo* abspaltbares (Poly)peptid kodierende DNA darstellt.

9. Verfahren nach Anspruch 8, wobei die DNA für ein sekretierbares TNF-bindendes Protein kodiert, **dadurch gekennzeichnet, dass** R² eine zur Gänze oder teilweise für eine Signalsequenz kodierende DNA darstellt.

10. Verfahren nach Anspruch 8, **dadurch gekennzeichnet, dass** R² eine Nukleinsäuresequenz mit der Formel CTG GTC CCT CAC CTA GGG GAC AGG GAG AAG AGA umfasst.

11. Verfahren nach Anspruch 9, **dadurch gekennzeichnet, dass** R² eine Nukleinsäure mit der Formel R^3 CTG GTC CCT CAC CTA GGG GAC AGG GAG AAG AGA umfasst, wobei R³ eine für ein Signalpeptid kodierende DNA darstellt.

12. Verfahren nach Anspruch 11, **dadurch gekennzeichnet, dass** R³ eine Nukleinsäuresequenz mit der Formel 
ATG GGC CTC TCC ACC GTG CCT GAC CTG CTG CTG CCA
CTG GTG CTC CTG GAG CTG TTG GTG GGA ATA TAC CCC
TCA GGG GTT ATT GGA
 umfasst.

13. Verfahren nach Anspruch 1, **dadurch gekennzeichnet, dass** die DNA eine DNA mit der Formel 
ATG GGC CTC TCC ACC GTG CCT GAC CTG CTG CTG CCA CTG GTG CTC
CTG GAG CTG TTG GTG GGA ATA TAC CCC TCA GGG GTT ATT GGA CTG
GTC CCT CAC CTA GGG GAC AGG GAG AAG AGA GAT AGT GTG TGT CCC
CAA GGA AAA TAT ATC CAC CCT CAA AAT AAT TCG ATT TGC TGT ACC
AAG TGC CAC AAA GGA ACC TAC TTG TAC AAT GAC TGT CCA GGC CCG
GGG CAG GAT ACG GAC TGC AGG GAG TGT GAG AGC GGC TCC TTC ACC
GCT TCA GAA AAC CAC CTC AGA CAC TGC CTC AGC TGC TCC AAA TGC
CGA AAG GAA ATG GGT CAG GTG GAG ATC TCT TCT TGC ACA GTG GAC
CGG GAC ACC GTG TGT GGC TGC AGG AAG AAC CAG TAC CGG CAT TAT
TGG AGT GAA AAC CTT TTC CAG TGC TTC AAT TGC AGC CTC TGC CTC
 
AAT GGG ACC GTG CAC CTC TCC TGC CAG GAG AAA CAG AAC ACC GTG
TGC ACC TGC CAT GCA GGT TTC TTT CTA AGA GAA AAC GAG TGT GTC
TCC TGT AGT AAC TGT AAG AAA AGC CTG GAG TGC ACG AAG TTG TGC
CTA CCC CAG ATT GAG AAT GTT AAG GGC ACT GAG GAC TCA GGC ACC
ACA GTG CTG TTG CCC CTG GTC ATT TTC TTT GGT CTT TGC CTT TTA
TCC CTC CTC TTC ATT GGT TTA ATG TAT CGC TAC CAA CGG TGG AAG
TCC AAG CTC TAC TCC ATT GTT TGT GGG AAA TCG ACA CCT GAA AAA
GAG GGG GAG CTT GAA GGA ACT ACT ACT AAG CCC CTG GCC CCA AAC
CCA AGC TTC AGT CCC ACT CCA GGC TTC ACC CCC ACC CTG GGC TTC
AGT CCC GTG CCC AGT TCC ACC TTC ACC TCC AGC TCC ACC TAT ACC
CCC GGT GAC TGT CCC AAC TTT GCG GCT CCC CGC AGA GAG GTG GCA
CCA CCC TAT CAG GGG GCT GAC CCC ATC CTT GCG ACA GCC CTC GCC
TCC GAC CCC ATC CCC AAC CCC CTT CAG AAG TGG GAG GAC AGC GCC
CAC AAG CCA CAG AGC CTA GAC ACT GAT GAC CCC GCG ACG CTG TAC
GCC GTG GTG GAG AAC GTG CCC CCG TTG CGC TGG AA GGAATTC
 oder ein Fragment, eine Variante oder eine Modifikation dieser Nukleinsäuresequenz, die für ein Polypeptid mit der Fähigkeit, TNF zu binden, kodiert, umfasst.

14. Verfahren zur Herstellung eines rekombinanten DNA-Moleküls, umfassend die Amplifikation einer DNA, wobei das DNA-Molekül die nach einem Verfahren gemäß einem der Ansprüche 1 bis 13 erhältliche DNA enthält.

15. Verfahren nach Anspruch 14, wobei das DNA Molekül in prokaryotischen oder eukaryotischen Wirtszellen replizierbar ist, und, funktionell verbunden mit der nach einem Verfahren gemäß einem der Ansprüche 1 bis 13 erhältlichen DNA, Expressionskontrollsequenzen enthält.

16. Verfahren nach Anspruch 15, wobei das rekombinante DNA-Molekül in Säugetierzellen replizierbar ist.

17. Verfahren nach Anspruch 14, **dadurch gekennzeichnet, dass** das rekombinante DNA-Molekül die in Anspruch 9 definierte DNA enthält.

18. Verfahren nach Anspruch 17, **dadurch gekennzeichnet, dass** das rekombinante DNA Molekül pADTNF-BP, gezeigt in Figur 7a, ist.

19. Verfahren nach Anspruch 17, **dadurch gekennzeichnet, dass** das rekombinante DNA Molekül pADBTNF-BP, gezeigt in Figur 7b, ist.

20. Verfahren nach Anspruch 17, **dadurch gekennzeichnet, dass** das rekombinante DNA Molekül pADTNF-R, gezeigt in Figur 7c, ist.

21. Verfahren nach Anspruch 17, **dadurch gekennzeichnet, dass** das rekombinante DNA Molekül pADBTNF-R, gezeigt in Figur 7d, ist.

22. Verfahren zur Herstellung einer Wirtszelle, umfassend die Transformation einer Zelle mit mindestens einem der rekombinanten DNA-Moleküle, erhältlich nach einem Verfahren gemäß einem der Ansprüche 14 bis 21, wobei die rekombinanten DNA-Moleküle eine Teilsequenz der für das TNF-bindende Protein kodierenden DNA darstellen.

23. Verfahren nach Anspruch 22, **dadurch gekennzeichnet, dass** die Wirtszelle eine eukaryotische Wirtszelle, bevorzugt eine COS-Zelle oder CHO-Zelle ist.

24. Verfahren zum Herstellen eines rekombinanten TNF-bindenden Proteines oder eines funktionellen Derivates davon, umfassend das Kultivieren einer Wirtszelle erhältlich nach einem Verfahren gemäß Anspruch 22 und Exprimieren des Proteines.

25. Verfahren nach Anspruch 24, umfassend weiter den Schritt des Isolierens des exprimierten Proteines.

26. Verfahren zur Herstellung eines rekombinanten Polypeptids, umfassend die Expression einer DNA, die aus der Gruppe ausgewählt ist, die für eines der folgenden Polypeptide kodiert:

(i) leu val pro his leu gly asp arg glu lys arg
asp ser val cys pro gln gly lys tyr ile his pro
gln asn asn ser ile cys cys thr lys cys his lys
gly thr tyr leu tyr asn asp cys pro gly pro gly
gln asp thr asp cys arg glu cys glu ser gly ser
phe thr ala ser glu asn his leu arg his cys leu
ser cys ser lys cys arg lys glu met gly gln val
glu ile ser ser cys thr val asp arg asp thr val
cys gly cys arg lys asn gln tyr arg his tyr trp

ser glu asn leu phe gln cys phe asn cys ser leu
cys leu asn gly thr val his leu ser cys gln glu
lys gln asn thr val cys thr cys his ala gly phe
phe leu arg glu asn glu cys val ser cys ser asn
cys lys lys ser leu glu cys thr lys leu cys leu
pro gln ile glu asn ,

(ii)

met gly leu ser thr val pro asp leu leu leu pro
leu val leu leu glu leu leu val gly ile tyr pro
ser gly val ile gly leu val pro his leu gly asp
arg glu lys arg asp ser val cys pro gln gly lys
tyr ile his pro gln asn asn ser ile cys cys thr
lys cys his lys gly thr tyr leu tyr asn asp cys
pro gly pro gly gln asp thr asp cys arg glu cys
glu ser gly ser phe thr ala ser glu asn his leu
arg his cys leu ser cys ser lys cys arg lys glu
met gly gln val glu ile ser ser cys thr val asp
arg asp thr val cys gly cys arg lys asn gln tyr
arg his tyr trp ser glu asn leu phe gln cys phe
asn cys ser leu cys leu asn gly thr val his leu
ser cys gln glu lys gln asn thr val cys thr cys
his ala gly phe phe leu arg glu asn glu cys val
ser cys ser asn cys lys lys ser leu glu cys thr
lys leu cys leu pro gln ile glu asn

(iii)
leu val pro his leu gly asp
arg glu lys arg asp ser val cys pro gln gly lys
tyr ile his pro gln asn asn ser ile cys cys thr
lys cys his lys gly thr tyr leu tyr asn asp cys
pro gly pro gly gln asp thr asp cys arg glu cys
glu ser gly ser phe thr ala ser glu asn his leu
arg his cys leu ser cys ser lys cys arg lys glu
met gly gln val glu ile ser ser cys thr val asp
arg asp thr val cys gly cys arg lys asn gln tyr
arg his tyr trp ser glu asn leu phe gln cys phe
asn cys ser leu cys leu asn gly thr val his leu
ser cys gln glu lys gln asn thr val cys thr cys
his ala gly phe phe leu arg glu asn glu cys val
ser cys ser asn cys lys lys ser leu glu cys thr
lys leu cys leu pro gln ile glu asn val lys gly
thr glu asp ser gly thr thr

27. Verfahren zur Herstellung eines Polypeptids, **dadurch gekennzeichnet, dass** ein TNF-bindendes Protein der folgenden Formel rekombinant exprimiert wird:

asp ser val cys pro gln gly lys tyr ile his pro
gln asn asn ser ile cys cys thr lys cys his lys

gly thr tyr leu tyr asn asp cys pro gly pro gly
gln asp thr asp cys arg glu cys glu ser gly ser
phe thr ala ser glu asn his leu arg his cys leu
ser cys ser lys cys arg lys glu met gly gln val
glu ile ser ser cys thr val asp arg asp thr val
cys gly cys arg lys asn gln tyr arg his tyr trp
ser glu asn leu phe gln cys phe asn cys ser leu
cys leu asn gly thr val his leu ser cys gln glu
lys gln asn thr val cys thr cys his ala gly phe
phe leu arg glu asn glu cys val ser cys ser asn
cys lys lys ser leu glu cys thr lys leu cys leu
pro gln ile glu asn

28. Verfahren nach Anspruch 27, **dadurch gekennzeichnet, dass** das Polypeptid den TNF-Rezeptor der Formel 
met gly leu ser thr val pro asp leu leu leu pro
leu val leu leu glu leu leu val gly ile tyr pro
ser gly val ile gly leu val pro his leu gly asp
arg glu lys arg asp ser val cys pro gln gly lys
tyr ile his pro gln asn asn ser ile cys cys thr
lys cys his lys gly thr tyr leu tyr asn asp cys
pro gly pro gly gln asp thr asp cys arg glu cys
glu ser gly ser phe thr ala ser glu asn his leu
arg his cys leu ser cys ser lys cys arg lys glu
met gly gln val glu ile ser ser cys thr val asp
arg asp thr val cys gly cys arg lys asn gln tyr
arg his tyr trp ser glu asn leu phe gln cys phe
asn cys ser leu cys leu asn gly thr val his leu
ser cys gln glu lys gln asn thr val cys thr cys
his ala gly phe phe leu arg glu asn glu cys val
ser cys ser asn cys lys lys ser leu glu cys thr
lys leu cys leu pro gln ile glu asn val lys gly
thr glu asp ser gly thr thr val leu leu pro leu
val ile phe phe gly leu cys leu leu ser leu leu
phe ile gly leu met tyr arg tyr gln arg trp lys
ser lys leu tyr ser ile val cys gly lys ser thr
pro glu lys glu gly glu leu glu gly thr thr thr
lys pro leu ala pro asn pro ser phe ser pro thr
pro gly phe thr pro thr leu gly phe ser pro val
pro ser ser thr phe thr ser ser ser thr tyr thr
pro gly asp cys pro asn phe ala ala pro arg arg
glu val ala pro pro tyr gln gly ala asp pro ile
leu ala thr ala leu ala ser asp pro ile pro asn
pro leu gln lys trp glu asp ser ala his lys pro
gln ser leu asp thr asp asp pro ala thr leu tyr
ala val val glu asn val pro pro leu arg trp
 umfasst.

29. Verfahren zum Untersuchen von Substanzen, **dadurch gekennzeichnet, dass** ein Polypeptid, erhältlich nach einem der Ansprüche 26 bis 28, verwendet wird, um die Wechselwirkung dieser Substanzen mit diesem Polypeptid und/oder mit TNF-α und/oder mit TNF-β und/oder den Einfluss dieser Substanzen auf die biologische Wirkung von TNF-α und/oder TNF-β *in vitro* zu bestimmen.

30. Verfahren zum Untersuchen von Substanzen, **dadurch gekennzeichnet, dass** die Wechselwirkung dieser Substanzen mit dem TNF-Rezeptor und/oder ihre Beeinflussung der biologischen Wirkung von TNF-α und/oder TNF-β mit einer nach Anspruch 24 erhältlichen Wirtszelle bestimmt wird.

31. Verfahren zum Herstellen eines Medikaments zum Behandeln von Autoimmunerkrankungen, **dadurch gekennzeichnet, dass** ein Polypeptid, erhältlich nach einem Verfahren gemäß einem der Ansprüche 26 bis 28, mit pharmazeutisch verträglichen Zusatzstoffen kombiniert wird

32. Verfahren zum Herstellen eines Medikaments zum Behandeln von rheumatoider Arthritis **dadurch gekennzeichnet, dass** ein Polypeptid, erhältlich nach einem Verfahren gemäß einem der Ansprüche 26 bis 28, mit pharmazeutisch verträglichen Zusatzstoffen kombiniert wird.

33. Verfahren zum Herstellen eines Medikaments zum Behandeln von ARDS **dadurch gekennzeichnet, dass** ein Polypeptid, erhältlich nach einem Verfahren gemäß einem der Ansprüche 26 bis 28, mit pharmazeutisch verträglichen Zusatzstoffen kombiniert wird.

34. Verfahren zum Herstellen eines Medikaments zum Behandeln von Anorexia/Cachexie, **dadurch gekennzeichnet, dass** ein Polypeptid, erhältlich nach einem Verfahren gemäß einem der Ansprüche 26 bis 28, mit pharmazeutisch verträglichen Zusatzstoffen kombiniert wird.

35. Verfahren zum Herstellen eines Medikaments zum Behandeln von GVHR, **dadurch gekennzeichnet, dass** ein Polypeptid, erhältlich nach einem Verfahren gemäß einem der Ansprüche 26 bis 28, mit pharmazeutisch verträglichen Zusatzstoffen kombiniert wird.

36. Verfahren zum Herstellen eines Medikaments zum Behandeln von Schock, **dadurch gekennzeichnet, dass** ein Polypeptid, erhältlich nach einem Verfahren gemäß einem der Ansprüche 26 bis 28, mit pharmazeutisch verträglichen Zusatzstoffen kombiniert wird.

37. Verfahren zur Herstellung eines Diagnostikums zur Bestimmung von TNF-α und/oder TNF-β, **dadurch gekennzeichnet, dass** ein Polypeptid, erhältlich nach einem Verfahren gemäß einem der Ansprüche 26 bis 28, zusammen mit Antikörpern gegen TNF bereitgestellt wird.

38. Verfahren zum Herstellen einer pharmazeutischen Zubereitung, **dadurch gekennzeichnet, dass** ein Polypeptid, erhältlich nach einem Verfahren gemäß einem der Ansprüche 26 bis 28, mit pharmazeutisch verträglichen Zusatzstoffen kombiniert wird.

## Patentansprüche (Patentansprüche für folgende(n) Vertragsstaat(en): GR)

1. DNA, kodierend für ein Polypeptid mit der Fähigkeit, TNF zu binden, wobei das Polypeptid eine Aminosäuresequenz mit der Formel 
asp ser val cys pro gln gly lys tyr ile his pro
gln asn asn ser ile cys cys thr lys cys his lys
gly thr tyr leu tyr asn asp cys pro gly pro gly
gln asp thr asp cys arg glu cys glu ser gly ser
phe thr ala ser glu asn his leu arg his cys leu
ser cys ser lys cys arg lys glu met gly gln val
glu ile ser ser cys thr val asp arg asp thr val
cys gly cys arg lys asn gln tyr arg his tyr trp
ser glu asn leu phe gln cys phe asn cys ser leu
cys leu asn gly thr val his leu ser cys gln glu
lys gln asn thr val cys thr cys his ala gly phe
phe leu arg glu asn glu cys val ser cys ser asn
cys lys lys ser leu glu cys thr lys leu cys leu
pro gln ile glu asn
 oder ein funktionelles Fragment oder eine Modifikation davon mit der Fähigkeit, TNF zu binden, umfasst.

2. DNA nach Anspruch 1, **dadurch gekennzeichnet, dass** das Polypeptid eine Aminosäuresequenz mit der Formel 
leu val pro his leu gly asp arg glu lys arg
asp ser val cys pro gln gly lys tyr ile his pro
gln asn asn ser ile cys cys thr lys cys his lys
gly thr tyr leu tyr asn asp cys pro gly pro gly
gln asp thr asp cys arg glu cys glu ser gly ser
phe thr ala ser glu asn his leu arg his cys leu
ser cys ser lys cys arg lys glu met gly gln val
glu ile ser ser cys thr val asp arg asp thr val
cys gly cys arg lys asn gln tyr arg his tyr trp
ser glu asn leu phe gln cys phe asn cys ser leu
cys leu asn gly thr val his leu ser cys gln glu
lys gln asn thr val cys thr cys his ala gly phe
phe leu arg glu asn glu cys val ser cys ser asn
cys lys lys ser leu glu cys thr lys leu cys leu
pro gln ile glu asn
 oder ein funkionelles Fragment oder eine Modifikation davon mit der Fähigkeit, TNF zu binden, umfasst.

3. DNA nach Anspruch 2, **dadurch gekennzeichnet, dass** das Polypeptid eine Aminosäuresequenz mit der Formel 
met gly leu ser thr val pro asp leu leu leu pro
leu val leu leu glu leu leu val gly ile tyr pro
ser gly val ile gly leu val pro his leu gly asp
arg glu lys arg asp ser val cys pro gln gly lys
tyr ile his pro gln asn asn ser ile cys cys thr
lys cys his lys gly thr tyr leu tyr asn asp cys
pro gly pro gly gln asp thr asp cys arg glu cys
glu ser gly ser phe thr ala ser glu asn his leu
arg his cys leu ser cys ser lys cys arg lys glu
met gly gln val glu ile ser ser cys thr val asp
arg asp thr val cys gly cys arg lys asn gln tyr
arg his tyr trp ser glu asn leu phe gln cys phe
asn cys ser leu cys leu asn gly thr val his leu
ser cys gln glu lys gln asn thr val cys thr cys
his ala gly phe phe leu arg glu asn glu cys val
ser cys ser asn cys lys lys ser leu glu cys thr
lys leu cys leu pro gln ile glu asn
 oder ein funktionelles Fragment oder eine Modifikation davon mit der Fähigkeit, TNF zu binden, umfasst.

4. DNA nach Anspruch 3, **dadurch gekennzeichnet, dass** das Polypeptid eine Aminosäuresequenz mit der Formel 
met gly leu ser thr val pro asp leu leu leu pro
leu val leu leu glu leu leu val gly ile tyr pro
ser gly val ile gly leu val pro his leu gly asp
arg glu lys arg asp ser val cys pro gln gly lys
tyr ile his pro gln asn asn ser ile cys cys thr
lys cys his lys gly thr tyr leu tyr asn asp cys
pro gly pro gly gln asp thr asp cys arg glu cys
glu ser gly ser phe thr ala ser glu asn his leu
arg his cys leu ser cys ser lys cys arg lys glu
met gly gln val glu ile ser ser cys thr val asp
arg asp thr val cys gly cys arg lys asn gln tyr
arg his tyr trp ser glu asn leu phe gln cys phe
asn cys ser leu cys leu asn gly thr val his leu
ser cys gln glu lys gln asn thr val cys thr cys
his ala gly phe phe leu arg glu asn glu cys val
ser cys ser asn cys lys lys ser leu glu cys thr
lys leu cys leu pro gln ile glu asn val lys gly
thr glu asp ser gly thr thr, val leu leu pro leu
val ile phe phe gly leu cys leu leu ser leu leu
phe ile gly leu met tyr arg tyr gln arg trp lys
ser lys leu tyr ser ile val cys gly lys ser thr
pro glu lys glu gly glu leu glu gly thr thr thr
lys pro leu ala pro asn pro ser phe ser pro thr
pro gly phe thr pro thr leu gly phe ser pro val
pro ser ser thr phe thr ser ser ser thr tyr thr
pro gly asp cys pro asn phe ala ala pro arg arg
glu val ala pro pro tyr gln gly ala asp pro ile
leu ala thr ala leu ala ser asp pro ile pro asn
pro leu gln lys trp glu asp ser ala his lys pro
gln ser leu asp thr asp asp pro ala thr leu tyr

ala val val glu asn val pro pro leu arg trp
 oder ein funktionelles Fragment oder eine Modifikation davon mit der Fähigkeit, TNF zu binden, umfasst.

5. DNA nach Anspruch 1, **dadurch gekennzeichnet, dass** das Polypeptid eine Aminosäuresequenz mit der Formel 
asp ser val cys pro gln gly lys
tyr ile his pro gln asn asn ser ile cys cys thr
lys cys his lys gly thr tyr leu tyr asn asp cys
pro gly pro gly gln asp thr asp cys arg glu cys
glu ser gly ser phe thr ala ser glu asn his leu
arg his cys leu ser cys ser lys cys arg lys glu
met gly gln val glu ile ser ser cys thr val asp
arg asp thr val cys gly cys arg lys asn gln tyr
arg his tyr trp ser glu asn leu phe gln cys phe
asn cys ser leu cys leu asn gly thr val his leu
ser cys gln glu lys gln asn thr val cys thr cys
his ala gly phe phe leu arg glu asn glu cys val
ser cys ser asn cys lys lys ser leu glu cys thr
lys leu cys leu pro gln ile glu asn val lys gly
thr glu asp ser gly thr thr
 oder ein funktionelles Fragment oder eine Modifikation davon mit der Fähigkeit, TNF zu binden, umfasst.

6. DNA nach Anspruch 5, **dadurch gekennzeichnet, dass** das Polypeptid eine Aminosäuresequenz mit der Formel 
leu val pro his leu gly asp
arg glu lys arg asp ser val cys pro gln gly lys
tyr ile his pro gln asn asn ser ile cys cys thr
lys cys his lys gly thr tyr leu tyr asn asp cys
pro gly pro gly gln asp thr asp cys arg glu cys
glu ser gly ser phe thr ala ser glu asn his leu
arg his cys leu ser cys ser lys cys arg lys glu
met gly gln val glu ile ser ser cys thr val asp
arg asp thr val cys gly cys arg lys asn gln tyr
arg his tyr trp ser glu asn leu phe gln cys phe
asn cys ser leu cys leu asn gly thr val his leu
ser cys gln glu lys gln asn thr val cys thr cys
his ala gly phe phe leu arg glu asn glu cys val
ser cys ser asn cys lys lys ser leu glu cys thr
lys leu cys leu pro gln ile glu asn val lys gly
thr glu asp ser gly thr thr
 oder ein funktionelles Fragment oder eine Modifikation davon mit der Fähigkeit, TNF zu binden, umfasst.

7. DNA nach Anspruch 1, **dadurch gekennzeichnet, dass** die Aminosäuresequenz des Polypeptids an einer oder mehreren Glykosylierungsstellen verändert ist.

8. DNA nach Anspruch 1, kodierend für sekretierbares TNF-bindendes Protein, **dadurch gekennzeichnet, dass** sie eine Nukleinsäuresequenz mit der Formel 
R= GAT AGT GTG TGT CCC CAA GGA AAA TAT ATC CAC
CCT CAA AAT AAT TCG ATT TGC TGT ACC AAG TGC CAC
AAA GGA ACC TAC TTG TAC AAT GAC TGT CCA GGC CCG
GGG CAG GAT ACG GAC TGC AGG GAG TGT GAG AGC GGC
TCC TTC ACC GCT TCA GAA AAC CAC CTC AGA CAC TGC
CTC AGC TGC TCC AAA TGC CGA AAG GAA ATG GGT CAG
GTG GAG ATC TCT TCT TGC ACA GTG GAC CGG GAC ACC
GTG TGT GGC TGC AGG AAG AAC CAG TAC CGG CAT TAT
TGG AGT GAA AAC CTT TTC CAG TGC TTC AAT TGC AGC
CTC TGC CTC AAT GGG ACC GTG CAC CTC TCC TGC CAG
GAG AAA CAG AAC ACC GTG TGC ACC TGC CAT GCA GGT
TTC TTT CTA AGA GAA AAC GAG TGT GTC TCC TGT AGT
AAC TGT AAG AAA AGC CTG GAG TGC ACG AAG TTG TGC
CTA CCC CAG ATT GAG AAT
 oder ein Fragment, eine Variante oder eine Modifikation dieser Nukleinsäuresequenz, die für ein Polypeptid mit der Fähigkeit, TNF zu binden, kodiert, umfasst, wobei R² eine für ein in *vivo* abspaltbares (Poly)peptid kodierende DNA darstellt.

9. DNA nach Anspruch 8, kodierend für sekretierbares TNF-bindendes Protein, **dadurch gekennzeichnet, dass** R² eine zur Gänze oder teilweise für eine Signalsequenz kodierende DNA darstellt.

10. DNA nach Anspruch 8, **dadurch gekennzeichnet, dass** R² eine Nukleinsäuresequenz mit der Formel CTG GTC CCT CAC CTA GGG GAC AGG GAG AAG AGA umfasst.

11. DNA nach Anspruch 9, **dadurch gekennzeichnet, dass** R² eine Nukleinsäure mit der Formel R3 CTG GTC CCT CAC CTA GGG GAC AGG GAG AAG AGA umfasst, wobei R³ eine für ein Signalpeptid kodierende DNA darstellt.

12. DNA nach Anspruch 11, **dadurch gekennzeichnet, dass** R³ eine Nukleinsäuresequenz mit der Formel 
ATG GGC CTC TCC ACC GTG CCT GAC CTG CTG CTG CGA
CTG GTG CTC CTG GAG CTG TTG GTG GGA ATA TAC CCC
TCA GGG GTT ATT GGA
 umfasst.

13. DNA nach Anspruch 1, **dadurch gekennzeichnet, dass** sie DNA mit der Formel 
ATG GGC CTC TCC ACC GTG CCT GAC CTG CTG CTG CCA CTG GTG CTC
CTG GAG CTG TTG GTG GGA ATA TAC CCC TCA GGG GTT ATT GGA CTG
GTC CCT CAC CTA GGG GAC AGG GAG AAG AGA|GAT AGT GTG TGT CCC
CAA GGA AAA TAT ATC CAC CCT CAA AAT AAT TCG ATT TGC TGT ACC
AAG TGC CAC AAA GGA ACC TAC TTG TAC AAT GAC TGT CCA GGC CCG
GGG CAG GAT ACG GAC TGC AGG GAG TGT GAG AGC GGC TCC TTC ACC
GCT TCA GAA AAC CAC CTC AGA CAC TGC CTC AGC TGC TCC AAA TGC
CGA AAG GAA ATG GGT CAG GTG GAG ATC TCT TCT TGC ACA GTG GAC
CGG GAC ACC GTG TGT GGC TGC AGG AAG AAC CAG TAC CGG CAT TAT
TGG AGT GAA AAC CTT TTC CAG TGC TTC AAT TGC AGC CTC TGC CTC
 
AAT GGG ACC GTG CAC CTC TCC TGC CAG GAG AAA CAG AAC ACC GTG
TGC ACC TGC CAT GCA GGT TTC TTT CTA AGA GAA AAC GAG TGT GTC
TCC TGT AGT AAC TGT AAG AAA AGC CTG GAG TGC ACG AAG TTG TGC
CTA CCC CAG ATT GAG AAT GTT AAG GGC ACT GAG GAC TCA GGC ACC
ACA GTG CTG TTG CCC CTG GTC ATT TTC TTT GGT CTT TGC CTT TTA
TCC CTC CTC TTC ATT GGT TTA ATG TAT CGC TAC CAA CGG TGG AAG
TCC AAG CTC TAC TCC ATT GTT TGT GGG AAA TCG ACA CCT GAA AAA
GAG GGG GAG CTT GAA GGA ACT ACT ACT AAG CCC CTG GCC CCA AAC
CCA AGC TTC AGT CCC ACT CCA GGC TTC ACC CCC ACC CTG GGC TTC
AGT CCC GTG CCC AGT TCC ACC TTC ACC TCC AGC TCC ACC TAT ACC
CCC GGT GAC TGT CCC AAC TTT GCG GCT CCC CGC AGA GAG GTG GCA
CCA CCC TAT CAG GGG GCT GAC CCC ATC CTT GCG ACA GCC CTC GCC
TCC GAC CCC ATC CCC AAC CCC CTT CAG AAG TGG GAG GAC AGC GCC
CAC AAG CCA CAG AGC CTA GAC ACT GAT GAC CCC GCG ACG CTG TAC
GCC GTG GTG GAG AAC GTG CCC CCG TTG CGC TGG AA GGAATTC
 oder ein Fragment, eine Variante oder eine Modifikation dieser Nukleinsäuresequenz, die für ein Polypeptid mit der Fähigkeit, TNF zu binden, kodiert, umfasst.

14. Rekombinantes DNA-Molekül, **dadurch gekennzeichnet, dass** es die DNA nach einem der Ansprüche 1 bis 13 enthält.

15. Rekombinantes DNA-Molekül nach Anspruch 14, replizierbar in prokaryontischen oder eukaryontischen Wirtszellen, **dadurch gekennzeichnet, dass** es, funktionell verbunden mit der DNA nach einem der Ansprüche 1 bis 13, Expressionskontrollsequenzen enthält.

16. Rekombinantes DNA-Molekül nach Anspruch 15, replizierbar in Säugetierzellen.

17. Rekombinantes DNA-Molekül. nach Anspruch 14, **dadurch gekennzeichnet, dass** es die in Anspruch 9 definierte DNA enthält.

18. Rekombinantes DNA-Molekül nach Anspruch 17 mit der Bezeichnung pADTNF-BP, gezeigt in Figur 7a.

19. Rekombinantes DNA-Molekül nach Anspruch 17 mit der Bezeichnung pADBTNF-BP, gezeigt in Figur 7b.

20. Rekombinantes DNA-Molekül nach Anspruch 17 mit der Bezeichnung pADTNF-R, gezeigt in Figur 7c.

21. Rekombinantes DNA-Molekül nach Anspruch 17 mit der Bezeichnung pADBTNF-R, gezeigt in Figur 7d.

22. Wirtszelle, enthaltend mindestens eines der in den Ansprüchen 14 bis 21 definierten rekombinanten DNA-Moleküle, das eine Teilsequenz der für das TNF-bindende Protein kodierenden DNA darstellt.

23. Wirtszelle nach Anspruch 22, **dadurch gekennzeichnet, dass** es eine eukaryontische Wirtszelle, bevorzugt eine COS-Zelle oder CHO-Zelle ist.

24. Verfahren zum Herstellen eines rekombinanten TNF-bindenden Proteines oder eines funktionellen Derivates davon, umfassend das Kultivieren eines Wirtes gemäß Anspruch 22 und Exprimieren des Proteines.

25. Verfahren nach Anspruch 24, umfassend weiter den Schritt des Isolierens des exprimierten Proteines.

26. Rekombinantes Polypeptid, **dadurch gekennzeichnet, dass** es von einer der DNA kodiert wird, die aus der Gruppe ausgewählt ist, die für eines der folgenden Polypeptide kodiert:

(i) leu val pro his leu gly asp arg glu lys arg
asp ser val cys pro gln gly lys tyr ile his pro
gln asn asn ser ile cys cys thr lys cys his lys
gly thr tyr leu tyr asn asp cys pro gly pro gly
gln asp thr asp cys arg glu cys glu ser gly ser
phe thr ala ser glu asn his leu arg his cys leu
ser cys ser lys cys arg lys glu met gly gln val
glu ile ser ser cys thr val asp arg asp thr val
cys gly cys arg lys asn gln tyr arg his tyr trp
ser glu asn leu phe gln cys phe asn cys ser leu
cys leu asn gly thr val his leu ser cys gln glu
lys gln asn thr val cys thr cys his ala gly phe
phe leu arg glu asn glu cys val ser cys ser asn
cys lys lys ser leu glu cys thr lys leu cys leu
pro gln ile glu asn ,

(ii)

met gly leu ser thr val pro asp leu leu leu pro
leu val leu leu glu leu leu val gly ile tyr pro
ser gly val ile gly leu val pro his leu gly asp
arg glu lys arg asp ser val cys pro gln gly lys
tyr ile his pro gln asn asn ser ile cys cys thr
lys cys his lys gly thr tyr leu tyr asn asp cys
pro gly pro gly gln asp thr asp cys arg glu cys
glu ser gly ser phe thr ala ser glu asn his leu
arg his cys leu ser cys ser lys cys arg lys glu
met gly gln val glu ile ser ser cys thr val asp
arg asp thr val cys gly cys arg lys asn gln tyr
arg his tyr trp ser glu asn leu phe gln cys phe
asn cys ser leu cys leu asn gly thr val his leu
ser cys gln glu lys gln asn thr val cys thr cys
his ala gly phe phe leu arg glu asn glu cys val
ser cys ser asn cys lys lys ser leu glu cys thr
lys leu cys leu pro gln ile glu asn

(iii)
leu val pro his leu gly asp
arg glu lys arg asp ser val cys pro gln gly lys
tyr ile his pro gln asn asn ser ile cys cys thr
lys cys his lys gly thr tyr leu tyr asn asp cys
pro gly pro gly gln asp thr asp cys arg glu cys
glu ser gly ser phe thr ala ser glu asn his leu
arg his cys leu ser cys ser lys cys arg lys glu
met gly gln val glu ile ser ser cys thr val asp
arg asp thr val cys gly cys arg lys asn gln tyr
arg his tyr trp ser glu asn leu phe gln cys phe
asn cys ser leu cys leu asn gly thr val his leu
ser cys gln glu lys gln asn thr val cys thr cys
his ala gly phe phe leu arg glu asn glu cys val
ser cys ser asn cys lys lys ser leu glu cys thr
lys leu cys leu pro gln ile glu asn val lys gly
thr glu asp ser gly thr thr

27. Polypeptid, **dadurch gekennzeichnet, dass** es ein TNF-bindendes Protein der Formel: 
asp ser val cys pro gln gly lys tyr ile his pro
gln asn asn ser ile cys cys thr lys cys his lys

gly thr tyr leu tyr asn asp cys pro gly pro gly
gln asp thr asp cys arg glu cys glu ser gly ser
phe thr ala ser glu asn his leu arg his cys leu
ser cys ser lys cys arg lys glu met gly gln val
glu ile ser ser cys thr val asp arg asp thr val
cys gly cys arg lys asn gln tyr arg his tyr trp
ser glu asn leu phe gln cys phe asn cys ser leu
cys leu asn gly thr val his leu ser cys gln glu
lys gln asn thr val cys thr cys his ala gly phe
phe leu arg glu asn glu cys val ser cys ser asn
cys lys lys ser leu glu cys thr lys leu cys leu
pro gln ile glu asn
 umfasst.

28. Polypeptid nach Anspruch 27, **dadurch gekennzeichnet, dass** es den TNF-Rezeptor der Formel 
met gly leu ser thr val pro asp leu leu leu pro
leu val leu leu glu leu leu val gly ile tyr pro
ser gly val ile gly leu val pro his leu gly asp
arg glu lys arg asp ser val cys pro gln gly lys
tyr ile his pro gln asn asn ser ile cys cys thr
lys cys his lys gly thr tyr leu tyr asn asp cys
pro gly pro gly gln asp thr asp cys arg glu cys
glu ser gly ser phe thr ala ser glu asn his leu
arg his cys leu ser cys ser lys cys arg lys glu
met gly gln val glu ile ser ser cys thr val asp
arg asp thr val cys gly cys arg lys asn gln tyr
arg his tyr trp ser glu asn leu phe gln cys phe
asn cys ser leu cys leu asn gly thr val his leu
ser cys gln glu lys gln asn thr val cys thr cys
his ala gly phe phe leu arg glu asn glu cys val
ser cys ser asn cys lys lys ser leu glu cys thr
lys leu cys leu pro gln ile glu asn val lys gly
thr glu asp ser gly thr thr val leu leu pro leu
val ile phe phe gly leu cys leu leu ser leu leu
phe ile gly leu met tyr arg tyr gln arg trp lys
ser lys leu tyr ser ile val cys gly lys ser thr
pro glu lys glu gly glu leu glu gly thr thr thr
lys pro leu ala pro asn pro ser phe ser pro thr
pro gly phe thr pro thr leu gly phe ser pro val
pro ser ser thr phe thr ser ser ser thr tyr thr
pro gly asp cys pro asn phe ala ala pro arg arg
glu val ala pro pro tyr gln gly ala asp pro ile
leu ala thr ala leu ala ser asp pro ile pro asn
pro leu gln lys trp glu asp ser ala his lys pro
gln ser leu asp thr asp asp pro ala thr leu tyr
ala val val glu asn val pro pro leu arg trp
 umfasst.

29. Verwendung eines in den Ansprüchen 26 bis 28 definierten Polypeptids zum Untersuchen von Substanzen auf ihre Wechselwirkung mit diesem Polypeptid und/oder mit TNF-α und/oder mit TNF-β und/oder auf ihre Beeinflussung der biologischen Wirkung von TNF-α und/oder TNF-β *in vitro*.

30. Verwendung einer in Anspruch 24 definierten Wirtszelle zum Untersuchen von Substanzen auf ihre Wechselwirkung mit dem TNF-Rezeptor und/oder ihre Beeinflussung der biologischen Wirkung von TNF-α und/oder TNF-β.

31. Verwendung eines Polypeptides nach einem der Ansprüche 26 bis 28 zum Herstellen eines Medikaments zum Behandeln von Autoimmunerkrankungen.

32. Verwendung eines Polypeptides nach einem der Ansprüche 26 bis 28 zum Herstellen eines Medikaments zum Behandeln von rheumatoider Arthritis.

33. Verwendung eines Polypeptides nach einem der Ansprüche 26 bis 28 zum Herstellen eines Medikaments zum Behandeln von ARDS.

34. Verwendung eines Polypeptides nach einem der Ansprüche 26 bis 28 zum Herstellen eines Medikaments zum Behandeln von Anorexia/Cachexie.

35. Verwendung eines Polypeptides nach einem der Ansprüche 26 bis 28 zum Herstellen eines Medikaments zum Behandeln von GVHR.

36. Verwendung eines Polypeptides nach einem der Ansprüche 26 bis 28 zum Herstellen eines Medikaments zum Behandeln von Schock.

37. Verwendung eines Polypeptides nach einem der Ansprüche 26 bis 28 als Diagnostikum zur Bestimmung von TNF-α und/oder TNF-β.

38. Verfahren zum Herstellen einer pharmazeutischen Zubereitung, **dadurch gekennzeichnet, dass** ein Polypeptid gemäß einem der Ansprüche 26 bis 28 mit pharmazeutisch verträglichen Zusatzstoffen kombiniert wird.

## Claims (Claims for the following Contracting State(s): AT, BE, CH, DE, DK, FR, GB, IT, LI, LU, NL, SE)

1. DNA coding for a polypeptide having the ability to bind TNF, wherein the polypeptide comprises an amino acid sequence having the formula 
asp ser val cys pro gln gly lys tyr ile his pro
gln asn asn ser ile cys cys thr lys cys his lys
gly thr tyr leu tyr asn asp cys pro gly pro gly
gln asp thr asp cys arg glu cys glu ser gly ser
phe thr ala ser glu asn his leu arg his cys leu
ser cys ser lys cys arg lys glu met gly gln val
glu ile ser ser cys thr val asp arg asp thr val
cys gly cys arg lys asn gln tyr arg his tyr trp
ser glu asn leu phe gln cys phe asn cys ser leu
cys leu asn gly thr val his leu ser cys gln glu
lys gln asn thr val cys thr cys his ala gly phe
phe leu arg glu asn glu cys val ser cys ser asn
cys lys lys ser leu glu cys thr lys leu cys leu
pro gln ile glu asn
 or a functional fragment or a modification thereof having the ability to bind TNF.

2. DNA according to claim 1, **characterised in that** the polypeptide comprises an amino acid sequence having the formula 
leu val pro his leu gly asp arg glu lys arg
asp ser val cys pro gln gly lys tyr ile his pro
gln asn asn ser ile cys cys thr lys cys his lys
gly thr tyr leu tyr asn asp cys pro gly pro gly
gln asp thr asp cys arg glu cys glu ser gly ser
phe thr ala ser glu asn his leu arg his cys leu
ser cys ser lys cys arg lys glu met gly gln val
glu ile ser ser cys thr val asp arg asp thr val
cys gly cys arg lys asn gln tyr arg his tyr trp
ser glu asn leu phe gln cys phe asn cys ser leu
cys leu asn gly thr val his leu ser cys gln glu
lys gln asn thr val cys thr cys his ala gly phe
phe leu arg glu asn glu cys val ser cys ser asn
cys lys lys ser leu glu cys thr lys leu cys leu
pro gln ile glu asn
 or a functional fragment or a modification thereof having the ability to bind TNF.

3. DNA according to claim 2, **characterised in that** the polypeptide comprises an amino acid sequence having the formula 
met gly leu ser thr val pro asp leu leu leu pro
leu val leu leu glu leu leu val gly ile tyr pro
ser gly val ile gly leu val pro his leu gly asp
arg glu lys arg asp ser val cys pro gln gly lys
tyr ile his pro gln asn asn ser ile cys cys thr
lys cys his lys gly thr tyr leu tyr asn asp cys
pro gly pro gly gln asp thr asp cys arg glu cys
glu ser gly ser phe thr ala ser glu asn his leu
arg his cys leu ser cys ser lys cys arg lys glu
met gly gln val glu ile ser ser cys thr val asp
arg asp thr val cys gly cys arg lys asn gln tyr
arg his tyr trp ser glu asn leu phe gln cys phe
asn cys ser leu cys leu asn gly thr val his leu
ser cys gln glu lys gln asn thr val cys thr cys
his ala gly phe phe leu arg glu asn glu cys val
ser cys ser asn cys lys lys ser leu glu cys thr
lys leu cys leu pro gln ile glu asn
 or a functional fragment or a modification thereof having the ability to bind TNF.

4. DNA according to claim 3, **characterised in that** the polypeptide comprises an amino acid sequence having the formula 
met gly leu ser thr val pro asp leu leu leu pro
leu val leu leu glu leu leu val gly ile tyr pro
ser gly val ile gly leu val pro his leu gly asp
arg glu lys arg asp ser val cys pro gln gly lys
tyr ile his pro gln asn asn ser ile cys cys thr
lys cys his lys gly thr tyr leu tyr asn asp cys
pro gly pro gly gln asp thr asp cys arg glu cys
glu ser gly ser phe thr ala ser glu asn his leu
arg his cys leu ser cys ser lys cys arg lys glu
met gly gln val glu ile ser ser cys thr val asp
arg asp thr val cys gly cys arg lys asn gln tyr
arg his tyr trp ser glu asn leu phe gln cys phe
asn cys ser leu cys leu asn gly thr val his leu
ser cys gln glu lys gln asn thr val cys thr cys
his ala gly phe phe leu arg glu asn glu cys val
ser cys ser asn cys lys lys ser leu glu cys thr
lys leu cys leu pro gln ile glu asn val lys gly
thr glu asp ser gly thr thr val leu leu pro leu
val ile phe phe gly leu cys leu leu ser leu leu
phe ile gly leu met tyr arg tyr gln arg trp lys
ser lys leu tyr ser ile val cys gly lys ser thr
pro glu lys glu gly glu leu glu gly thr thr thr
lys pro leu ala pro asn pro ser phe ser pro thr
pro gly phe thr pro thr leu gly phe ser pro val
pro ser ser thr phe thr ser ser ser thr tyr thr
pro gly asp cys pro asn phe ala ala pro arg arg
glu val ala pro pro tyr gln gly ala asp pro ile
leu ala thr ala leu ala ser asp pro ile pro asn
pro leu gln lys trp glu asp ser ala his lys pro
gln ser leu asp thr asp asp pro ala thr leu tyr

ala val val glu asn val pro pro leu arg trp
 or a functional fragment or a modification thereof having the ability to bind TNF.

5. DNA according to claim 1, **characterised in that** the polypeptide comprises an amino acid sequence having the formula 
asp ser val cys pro gln gly lys
tyr ile his pro gln asn asn ser ile cys cys thr
lys cys his lys gly thr tyr leu tyr asn asp cys
pro gly pro gly gln asp thr asp cys arg glu cys
glu ser gly ser phe thr ala ser glu asn his leu
arg his cys leu ser cys ser lys cys arg lys glu
met gly gln val glu ile ser ser cys thr val asp
arg asp thr val cys gly cys arg lys asn gln tyr
arg his tyr trp ser glu asn leu phe gln cys phe
asn cys ser leu cys leu asn gly thr val his leu
ser cys gln glu lys gln asn thr val cys thr cys
his ala gly phe phe leu arg glu asn glu cys val
ser cys ser asn cys lys lys ser leu glu cys thr
lys leu cys leu pro gln ile glu asn val lys gly
thr glu asp ser gly thr thr
 or a functional fragment or a modification thereof having the ability to bind TNF.

6. DNA according to claim 5, **characterised in that** the polypeptide comprises an amino acid sequence having the formula 
leu val pro his leu gly asp
arg glu lys arg asp ser val cys pro gln gly lys
tyr ile his pro gln asn asn ser ile cys cys thr
lys cys his lys gly thr tyr leu tyr asn asp cys
pro gly pro gly gln asp thr asp cys arg glu cys
glu ser gly ser phe thr ala ser glu asn his leu
arg his cys leu ser cys ser lys cys arg lys glu
met gly gln val glu ile ser ser cys thr val asp
arg asp thr val cys gly cys arg lys asn gln tyr
arg his tyr trp ser glu asn leu phe gln cys phe
asn cys ser leu cys leu asn gly thr val his leu
ser cys gln glu lys gln asn thr val cys thr cys
his ala gly phe phe leu arg glu asn glu cys val
ser cys ser asn cys lys lys ser leu glu cys thr
lys leu cys leu pro gln ile glu asn val lys gly
thr glu asp ser gly thr thr
 or a functional fragment or a modification thereof having the ability to bind TNF.

7. DNA according to claim 1, **characterised in that** the amino acid sequence of the polypeptide is altered at one or more glycosylation sites.

8. DNA according to claim 1, coding for secretable TNF-binding protein, **characterised in that** it comprises a nucleic acid sequence having the formula 
R= GAT AGT GTG TGT CCC CAA GGA AAA TAT ATC CAC
CCT CAA AAT AAT TCG ATT TGC TGT ACC AAG TGC CAC
AAA GGA ACC TAC TTG TAC AAT GAC TGT CCA GGC CCG
GGG CAG GAT ACG GAC TGC AGG GAG TGT GAG AGC GGC
TCC TTC ACC GCT TCA GAA AAC CAC CTC AGA CAC TGC
CTC AGC TGC TCC AAA TGC CGA AAG GAA ATG GGT CAG
GTG GAG ATC TCT TCT TGC ACA GTG GAC CGG GAC ACC
GTG TGT GGC TGC AGG AAG AAC CAG TAC CGG CAT TAT
TGG AGT GAA AAC CTT TTC CAG TGC TTC AAT TGC AGC
CTC TGC CTC AAT GGG ACC GTG CAC CTC TCC TGC CAG
GAG AAA CAG AAC ACC GTG TGC ACC TGC CAT GCA GGT
TTC TTT CTA AGA GAA AAC GAG TGT GTC TCC TGT AGT
AAC TGT AAG AAA AGC CTG GAG TGC ACG AAG TTG TGC
CTA CCC CAG ATT GAG AAT
 or a fragment, a variant or a modification of this nucleic acid sequence which codes for a polypeptide having the ability to bind TNF, wherein R² represents a DNA coding for a (poly)peptide which can be cleaved *in vivo.*

9. DNA according to claim 8, coding for secretable TNF-binding protein, **characterised in that** R² represents a DNA coding completely or partly for a signal sequence.

10. DNA according to claim 8, **characterised in that** R² comprises a nucleic acid sequence having the formula

CTG GTC CCT CAC CTA GGG GAC AGG GAG AAG AGA

11. DNA according to claim 9, **characterised in that** R² comprises a nucleic acid having the formula R3 CTG GTC CCT GAC CTA GGG GAC AGG GAG AAG AGA wherein R³ represents a DNA coding for a signal peptide.

12. DNA according to claim 11, **characterised in that** R³ comprises a nucleic acid sequence having the formula

ATG GGC CTC TCC ACC GTG CCT GAC CTG CTG CTG CGA
CTG GTG CTC CTG GAG CTG TTG GTG GGA ATA TAC CCC
TCA GGG GTT ATT GGA

13. DNA according to claim 1, **characterised in that** it comprises DNA having the formula 
ATG GGC CTC TCC ACC GTG CCT GAC CTG CTG CTG CCA CTG GTG CTC
CTG GAG CTG TTG GTG GGA ATA TAC CCC TCA GGG GTT ATT GGA CTC
GTC CCT CAC CTA GGG GAC AGG GAG AAG AGA/GAT AGT GTG TGT CCC
CAA GGA AAA TAT ATC CAC CCT CAA AAT AAT TCG ATT TGC TGT ACC
AAG TGC CAC AAA GGA ACC TAC TTG TAC AAT GAC TGT CCA GGC CCG
GGG CAG GAT ACG GAC TGC AGG GAG TGT GAG AGC GGC TCC TTC ACC
GCT TCA GAA AAC CAC CTC AGA CAC TGC CTC AGC TGC TCC AAA TGC
CGA AAG GAA ATG GGT CAG GTG GAG ATC TCT TCT TGC ACA GTG GAC
CGG GAC ACC GTG TGT GGC TGC AGG AAG AAC CAG TAC CGG CAT TAT
TGG AGT GAA AAC CTT TTC CAG TGC TTC AAT TGC AGC CTC TGC CTC

AAT GGG ACC GTG CAC CTC TCC TGC CAG GAG AAA CAG AAC ACC G[?]
TGC ACC TGC CAT GCA GGT TTC TTT CTA AGA GAA AAC GAG TGT G[?]
TCC TGT AGT AAC TGT AAG AAA AGC CTG GAG TGC ACG AAG TTG TC
CTA CCC CAG ATT GAG AAT GTT AAG GGC ACT GAG GAC TCA GGC AC
ACA GTG CTG TTG CCC CTG GTC ATT TTC TTT GGT CTT TGC CTT T[?]
TCC CTC CTC TTC ATT GGT TTA ATG TAT CGC TAC CAA CGG TGG AA
TCC AAG CTC TAC TCC ATT GTT TGT GGG AAA TCG ACA CCT GAA AA
GAG GGG GAG CTT GAA GGA ACT ACT ACT AAG CCC CTG GCC CCA AA[?]
CCA AGC TTC AGT CCC ACT CCA GGC TTC ACC CCC ACC CTG GGC TT[?]
AGT CCC GTG CCC AGT [?]CC ACC TTC ACC TCC AGC [?]CC ACC TAT AC[?]
CCC GGT GAC TGT CCC AAC TTT GCG GCT CCC CGC AGA GAG GTG GC
CCA CCC TAT CAG GGG GCT GAC CCC ATC CTT GCG ACA GCC CTC GC[?]
TCC GAC CCC ATC CCC AAC CCC CTT CAG AAG TGG GAG GAC AGC GCC
CAC AAG CCA CAG AGC CTA GAC ACT GAT GAC CCC GCG ACG CTG TAC
GCC GTG GTG GAG AAC GTG CCC CCG TTG CGC TGG AA GGAATTC
 or a fragment, a variant or a modification of this nucleic acid sequence which codes for a polypeptide having the ability to bind TNF.

14. Recombinant DNA molecule, **characterised in that** it contains the DNA according to one of claims 1 to 13.

15. Recombinant DNA molecule according to claim 14, which can be replicated in prokaryotic or eukaryotic host cells, **characterised in that**, functionally bound to the DNA according to one of claims 1 to 13, it contains expression control sequences.

16. Recombinant DNA molecule according to claim 15, which can be replicated in mammalian cells.

17. Recombinant DNA molecule according to claim 14, **characterised in that** it contains the DNA defined in claim 9.

18. Recombinant DNA molecule according to claim 17 having the designation pADTNF-BP, shown in Figure 7a.

19. Recombinant DNA molecule according to claim 17 having the designation pADBTNF-BP, shown in Figure 7b.

20. Recombinant DNA molecule according to claim 17 having the designation pADTNF-R, shown in Figure 7c.

21. Recombinant DNA molecule according to claim 17 having the designation pADBTNF-R, shown in Figure 7d.

22. Host cell, containing at least one of the recombinant DNA molecules defined in claims 14 to 21, which represents a partial sequence of the DNA coding for the TNF-binding protein.

23. Host cell according to claim 22, **characterised in that** it is a eukaryotic host cell, preferably a COS cell or CHO cell.

24. Process for producing a recombinant TNF-binding protein or a functional derivative thereof, comprising the cultivation of a host according to claim 22 and expressing of the protein.

25. Process according to claim 24, comprising further the step of isolating the expressed protein.

26. Recombinant polypeptide, **characterised in that** it is coded by one of the DNAs which is selected from the group which codes for one of the following polypeptides:

(i) leu val pro his leu gly asp arg glu lys arg
asp ser val cys pro gln gly lys tyr ile his pro
gln asn asn ser ile cys cys thr lys cys his lys
gly thr tyr leu tyr asn asp cys pro gly pro gly
gln asp thr asp cys arg glu cys glu ser gly ser
phe thr ala ser glu asn his leu arg his cys leu
ser cys ser lys cys arg lys glu met gly gln val
glu ile ser ser cys thr val asp arg asp thr val
cys gly cys arg lys asn gln tyr arg his tyr trp
ser glu asn leu phe gln cys phe asn cys ser leu
cys leu asn gly thr val his leu ser cys gln glu
lys gln asn thr val cys thr cys his ala gly phe
phe leu arg glu asn glu cys val ser cys ser asn
cys lys lys ser leu glu cys thr lys leu cys leu
pro gln ile glu asn,

(ii)

met gly leu ser thr val pro asp leu leu leu pro
leu val leu leu glu leu leu val gly ile tyr pro
ser gly val ile gly leu val pro his leu gly asp
arg glu lys arg asp ser val cys pro gln gly lys
tyr ile his pro gln asn asn ser ile cys cys thr
lys cys his lys gly thr tyr leu tyr asn asp cys
pro gly pro gly gln asp thr asp cys arg glu cys
glu ser gly ser phe thr ala ser glu asn his leu
arg his cys leu ser cys ser lys cys arg lys glu
met gly gln val glu ile ser ser cys thr val asp
arg asp thr val cys gly cys arg lys asn gln tyr
arg his tyr trp ser glu asn leu phe gln cys phe
asn cys ser leu cys leu asn gly thr val his leu
ser cys gln glu lys gln asn thr val cys thr cys
his ala gly phe phe leu arg glu asn glu cys val
ser cys ser asn cys lys lys ser leu glu cys thr
lys leu cys leu pro gln ile glu asn

(iii) leu val pro his leu gly asp
arg glu lys arg asp ser val cys pro gln gly lys
tyr ile his pro gln asn asn ser ile cys cys thr
lys cys his lys gly thr tyr leu tyr asn asp cys
pro gly pro gly gln asp thr asp cys arg glu cys
glu ser gly ser phe thr ala ser glu asn his leu
arg his cys leu ser cys ser lys cys arg lys glu
met gly gln val glu ile ser ser cys thr val asp
arg asp thr val cys gly cys arg lys asn gln tyr
arg his tyr trp ser glu asn leu phe gln cys phe
asn cys ser leu cys leu asn gly thr val his leu
ser cys gln glu lys gln asn thr val cys thr cys
his ala gly phe phe leu arg glu asn glu cys val
ser cys ser asn cys lys lys ser leu glu cys thr
lys leu cys leu pro gln ile glu asn val lys gly
thr glu asp ser gly thr thr

27. Polypeptide, **characterised in that** it comprises a TNF-binding protein of the formula:

asp ser val cys pro gln gly lys tyr ile his pro
gln asn asn ser ile cys cys thr lys cys his lys

gly thr tyr leu tyr asn asp cys pro gly pro gly
gln asp thr asp cys arg glu cys glu ser gly ser
phe thr ala ser glu asn his leu arg his cys leu
ser cys ser lys cys arg lys glu met gly gln val
glu ile ser ser cys thr val asp arg asp thr val
cys gly cys arg lys asn gln tyr arg his tyr trp
ser glu asn leu phe gln cys phe asn cys ser leu
cys leu asn gly thr val his leu ser cys gln glu
lys gln asn thr val cys thr cys his ala gly phe
phe leu arg glu asn glu cys val ser cys ser asn
cys lys lys ser leu glu cys thr lys leu cys leu
pro gln ile glu asn

28. Polypeptide according to claim 27, **characterised in that** it comprises the TNF receptor of the formula

met gly leu ser thr val pro asp leu leu leu pro
leu val leu leu glu leu leu val gly ile tyr pro
ser gly val ile gly leu val pro his leu gly asp
arg glu lys arg asp ser val cys pro gln gly lys
tyr ile his pro gln asn asn ser ile cys cys thr
lys cys his lys gly thr tyr leu tyr asn asp cys
pro gly pro gly gln asp thr asp cys arg glu cys
glu ser gly ser phe thr ala ser glu asn his leu
arg his cys leu ser cys ser lys cys arg lys glu
met gly gln val glu ile ser ser cys thr val asp
arg asp thr val cys gly cys arg lys asn gln tyr
arg his tyr trp ser glu asn leu phe gln cys phe
asn cys ser leu cys leu asn gly thr val his leu
ser cys gln glu lys gln asn thr val cys thr cys
his ala gly phe phe leu arg glu asn glu cys val
ser cys ser asn cys lys lys ser leu glu cys thr
lys leu cys leu pro gln ile glu asn val lys gly
thr glu asp ser gly thr thr val leu leu pro leu
val ile phe phe gly leu cys leu leu ser leu leu
phe ile gly leu met tyr arg tyr gln arg trp lys
ser lys leu tyr ser ile val cys gly lys ser thr
pro glu lys glu gly glu leu glu gly thr thr thr
lys pro leu ala pro asn pro ser phe ser pro thr
pro gly phe thr pro thr leu gly phe ser pro val
pro ser ser thr phe thr ser ser ser thr tyr thr
pro gly asp cys pro asn phe ala ala pro arg arg
glu val ala pro pro tyr gln gly ala asp pro ile
leu ala thr ala leu ala ser asp pro ile pro asn
pro leu gln lys trp glu asp ser ala his lys pro
gln ser leu asp thr asp asp pro ala thr leu tyr
ala val val glu asn val pro pro leu arg trp

29. Use of a polypeptide defined in claims 26 to 28 for investigating substances for their interaction with this polypeptide and/or with TNF-α and/or with TNF-β and/or for their influence of the biological effect of TNF-α and/or TNF-β *in vitro*.

30. Use of a host cell defined in claim 24 for investigating substances for their interaction with the TNF receptor and/or their influence of the biological effect of TNF-α and/or TNF-β.

31. Use of a polypeptide according to one of claims 26 to 28 for producing a medicament for treating autoimmune diseases.

32. Use of a polypeptide according to one of claims 26 to 28 for producing a medicament for treating rheumatoid arthritis.

33. Use of a polypeptide according to one of claims 26 to 28 for producing a medicament for treating ARDS.

34. Use of a polypeptide according to one of claims 26 to 28 for producing a medicament for treating anorexia/cachexia.

35. Use of a polypeptide according to one of claims 26 to 28 for producing a medicament for treating GVHR.

36. Use of a polypeptide according to one of claims 26 to 28 for producing a medicament for treating shock.

37. Use of a polypeptide according to one of claims 26 to 28 as a diagnostic agent for determining TNF-α and/or TNF-β.

38. Pharmaceutical preparation, **characterised in that** it contains as a therapeutically active component, a polypeptide defined in one of claims 26 to 28.

## Claims (Claims for the following Contracting State(s): ES)

1. Process for producing a DNA, comprising the amplification of a DNA which codes for a polypeptide having the ability to bind TNF, wherein the polypeptide comprises an amino acid sequence having the formula 
asp ser val cys pro gln gly lys tyr ile his pro
gln asn asn ser ile cys cys thr lys cys his lys
gly thr tyr leu tyr asn asp cys pro gly pro gly
gln asp thr asp cys arg glu cys glu ser gly ser
phe thr ala ser glu asn his leu arg his cys leu
ser cys ser lys cys arg lys glu met gly gln val
glu ile ser ser cys thr val asp arg asp thr val
cys gly cys arg lys asn gln tyr arg his tyr trp
ser glu asn leu phe gln cys phe asn cys ser leu
cys leu asn gly thr val his leu ser cys gln glu
lys gln asn thr val cys thr cys his ala gly phe
phe leu arg glu asn glu cys val ser cys ser asn
cys lys lys ser leu glu cys thr lys leu cys leu
pro gln ile glu asn
 or a functional fragment or a modification thereof having the ability to bind TNF.

2. Process according to claim 1, **characterised in that** the polypeptide comprises an amino acid sequence having the formula 
leu val pro his leu gly asp arg glu lys arg
asp ser val cys pro gln gly lys tyr ile his pro
gln asn asn ser ile cys cys thr lys cys his lys
gly thr tyr leu tyr asn asp cys pro gly pro gly
gln asp thr asp cys arg glu cys glu ser gly ser
phe thr ala ser glu asn his leu arg his cys leu
ser cys ser lys cys arg lys glu met gly gln val
glu ile ser ser cys thr val asp arg asp thr val
cys gly cys arg lys asn gln tyr arg his tyr trp
ser glu asn leu phe gln cys phe asn cys ser leu
cys leu asn gly thr val his leu ser cys gln glu
lys gln asn thr val cys thr cys his ala gly phe
phe leu arg glu asn glu cys val ser cys ser asn
cys lys lys ser leu glu cys thr lys leu cys leu
pro gln ile glu asn
 or a functional fragment or a modification thereof having the ability to bind TNF.

3. Process according to claim 2, **characterised in that** the polypeptide comprises an amino acid sequence having the formula 
met gly leu ser thr val pro asp leu leu leu pro
leu val leu leu glu leu leu val gly ile tyr pro
ser gly val ile gly leu val pro his leu gly asp
arg glu lys arg asp ser val cys pro gln gly lys
tyr ile his pro gln asn asn ser ile cys cys thr
lys cys his lys gly thr tyr leu tyr asn asp cys
pro gly pro gly gln asp thr asp cys arg glu cys
glu ser gly ser phe thr ala ser glu asn his leu
arg his cys leu ser cys ser lys cys arg lys glu
met gly gln val glu ile ser ser cys thr val asp
arg asp thr val cys gly cys arg lys asn gln tyr
arg his tyr trp ser glu asn leu phe gln cys phe
asn cys ser leu cys leu asn gly thr val his leu
ser cys gln glu lys gln asn thr val cys thr cys
his ala gly phe phe leu arg glu asn glu cys val
ser cys ser asn cys lys lys ser leu glu cys thr
lys leu cys leu pro gln ile glu asn
 or a functional fragment or a modification thereof having the ability to bind TNF.

4. Process according to claim 3, **characterised in that** the polypeptide comprises an amino acid sequence having the formula 
met gly leu ser thr val pro asp leu leu leu pro
leu val leu leu glu leu leu val gly ile tyr pro
ser gly val ile gly leu val pro his leu gly asp
arg glu lys arg asp ser val cys pro gln gly lys
tyr ile his pro gln asn asn ser ile cys cys thr
lys cys his lys gly thr tyr leu tyr asn asp cys
pro gly pro gly gln asp thr asp cys arg glu cys
glu ser gly ser phe thr ala ser glu asn his leu
arg his cys leu ser cys ser lys cys arg lys glu
met gly gln val glu ile ser ser cys thr val asp
arg asp thr val cys gly cys arg lys asn gln tyr
arg his tyr trp ser glu asn leu phe gln cys phe
asn cys ser leu cys leu asn gly thr val his leu
ser cys gln glu lys gln asn thr val cys thr cys
his ala gly phe phe leu arg glu asn glu cys val
ser cys ser asn cys lys lys ser leu glu cys thr
lys leu cys leu pro gln ile glu asn val lys gly
thr glu asp ser gly thr thr val leu leu pro leu
val ile phe phe gly leu cys leu leu ser leu leu
phe ile gly leu met tyr arg tyr gln arg trp lys
ser lys leu tyr ser ile val cys gly lys ser thr
pro glu lys glu gly glu leu glu gly thr thr thr
lys pro leu ala pro asn pro ser phe ser pro thr
pro gly phe thr pro thr leu gly phe ser pro val
pro ser ser thr phe thr ser ser ser thr tyr thr
pro gly asp cys pro asn phe ala ala pro arg arg
glu val ala pro pro tyr gln gly ala asp pro ile
leu ala thr ala leu ala ser asp pro ile pro asn
pro leu gln lys trp glu asp ser ala his lys pro
gln ser leu asp thr asp asp pro ala thr leu tyr

ala val val glu asn val pro pro leu arg trp
 or a functional fragment or a modification thereof having the ability to bind TNF.

5. Process according to claim 1, **characterised in that** the polypeptide comprises an amino acid sequence having the formula 
asp ser val cys pro gln gly lys
tyr ile his pro gln asn asn ser ile cys cys thr
lys cys his lys gly thr tyr leu tyr asn asp cys
pro gly pro gly gln asp thr asp cys arg glu cys
glu ser gly ser phe thr ala ser glu asn his leu
arg his cys leu ser cys ser lys cys arg lys glu
met gly gln val glu ile ser ser cys thr val asp
arg asp thr val cys gly cys arg lys asn gln tyr
arg his tyr trp ser glu asn leu phe gln cys phe
asn cys ser leu cys leu asn gly thr val his leu
ser cys gln glu lys gln asn thr val cys thr cys
his ala gly phe phe leu arg glu asn glu cys val
ser cys ser asn cys lys lys ser leu glu cys thr
lys leu cys leu pro gln ile glu asn val lys gly
thr glu asp ser gly thr thr
 or a functional fragment or a modification thereof having the ability to bind TNF.

6. Process according to claim 5, **characterised in that** the polypeptide comprises an amino acid sequence having the formula 
leu val pro his leu gly asp
arg glu lys arg asp ser val cys pro gln gly lys
tyr ile his pro gln asn asn ser ile cys cys thr
lys cys his lys gly thr tyr leu tyr asn asp cys
pro gly pro gly gln asp thr asp cys arg glu cys
glu ser gly ser phe thr ala ser glu asn his leu
arg his cys leu ser cys ser lys cys arg lys glu
met gly gln val glu ile ser ser cys thr val asp
arg asp thr val cys gly cys arg lys asn gln tyr
arg his tyr trp ser glu asn leu phe gln cys phe
asn cys ser leu cys leu asn gly thr val his leu
ser cys gln glu lys gln asn thr val cys thr cys
his ala gly phe phe leu arg glu asn glu cys val
ser cys ser asn cys lys lys ser leu glu cys thr
lys leu cys leu pro gln ile glu asn val lys gly
thr glu asp ser gly thr thr
 or a functional fragment or a modification thereof having the ability to bind TNF.

7. Process according to claim 1, **characterised in that** the amino acid sequence of the polypeptide is altered at one or more glycosylation sites.

8. Process according to claim 1, wherein the DNA codes for a secretable TNF-binding protein, **characterised in that** the DNA comprises a nucleic acid sequence having the formula 
R= GAT AGT GTG TGT CCC CAA GGA AAA TAT ATC CAC
CCT CAA AAT AAT TCG ATT TGC TGT ACC AAG TGC CAC
AAA GGA ACC TAC TTG TAC AAT GAC TGT CCA GGC CCG
GGG CAG GAT ACG GAC TGC AGG GAG TGT GAG AGC GGC
TCC TTC ACC GCT TCA GAA AAC CAC CTC AGA CAC TGC
CTC AGC TGC TCC AAA TGC CGA AAG GAA ATG GGT CAG
GTG GAG ATC TCT TCT TGC ACA GTG GAC CGG GAC ACC
GTG TGT GGC TGC AGG AAG AAC CAG TAC CGG CAT TAT
TGG AGT GAA AAC CTT TTC CAG TGC TTC AAT TGC AGC
CTC TGC CTC AAT GGG ACC GTG CAC CTC TCC TGC CAG
GAG AAA CAG AAC ACC GTG TGC ACC TGC CAT GCA GGT
TTC TTT CTA AGA GAA AAC GAG TGT GTC TCC TGT AGT
AAC TGT AAG AAA AGC CTG GAG TGC ACG AAG TTG TGC
CTA CCC CAG ATT GAG AAT
 or a fragment, a variant or a modification of this nucleic acid sequence which codes for a polypeptide having the ability to bind TNF, wherein R² represents a DNA coding for a (poly)peptide which can be cleaved *in vivo.*

9. Process according to claim 8, wherein the DNA codes for a secretable TNF-binding protein, **characterised in that** R² represents a DNA coding completely or partly for a signal sequence.

10. Process according to claim 8, **characterised in that** R² comprises a nucleic acid sequence having the formula

CTG GTC CCT CAC CTA GGG GAC AGG GAG AAG AGA

11. Process according to claim 9, **characterised in that** R² comprises a nucleic acid having the formula R3 CTG GTC CCT GAC CTA GGG GAC AGG GAG AAG AGA wherein R³ represents a DNA coding for a signal peptide.

12. Process according to claim 11, **characterised in that** R³ comprises a nucleic acid sequence having the formula

ATG GGC CTC TCC ACC GTG CCT GAC CTG CTG CTG CGA
CTG GTG CTC CTG GAG CTG TTG GTG GGA ATA TAC CCC
TCA GGG GTT ATT GGA

13. Process according to claim 1, **characterised in that** the DNA comprises a DNA having the formula 
ATG GGC CTC TCC ACC GTG CCT GAC CTG CTG CTG CCA CTG GTG CTC
CTG GAG CTG TTG GTG GGA ATA TAC CCC TCA GGG GTT ATT GGA CTG
GTC CCT CAC CTA GGG GAC AGG GAG AAG AGA GAT AGT GTG TGT CCC
CAA GGA AAA TAT ATC CAC CCT CAA AAT AAT TCG ATT TGC TGT ACC
AAG TGC CAC AAA GGA ACC TAC TTG TAC AAT GAC TGT CCA GGC CCG
GGG CAG GAT ACG GAC TGC AGG GAG TGT GAG AGC GGC TCC TTC ACC
GCT TCA GAA AAC CAC CTC AGA CAC TGC CTC AGC TGC TCC AAA TGC
CGA AAG GAA ATG GGT CAG GTG GAG ATC TCT TCT TGC ACA GTG GAC
CGG GAC ACC GTG TGT GGC TGC AGG AAG AAC CAG TAC CGG CAT TAT
TGG AGT GAA AAC CTT TTC CAG TGC TTC AAT TGC AGC CTC TGC CTC
 
AAT GGG ACC GTG CAC CTC TCC TGC CAG GAG AAA CAG AAC ACC GTG
TGC ACC TGC CAT GCA GGT TTC TTT CTA AGA GAA AAC GAG TGT GTC
TCC TGT AGT AAC TGT AAG AAA AGC CTG GAG TGC ACG AAG TTG TGC
CTA CCC CAG ATT GAG AAT GTT AAG GGC ACT GAG GAC TCA GGC ACC
ACA GTG CTG TTG CCC CTG GTC ATT TTC TTT GGT CTT TGC CTT TTA
TCC CTC CTC TTC ATT GGT TTA ATG TAT CGC TAC CAA CGG TGG AAG
TCC AAG CTC TAC TCC ATT GTT TGT GGG AAA TCG ACA CCT GAA AAA
GAG GGG GAG CTT GAA GGA ACT ACT ACT AAG CCC CTG GCC CCA AAC
CCA AGC TTC AGT CCC ACT CCA GGC TTC AGC CCC ACC CTG GGC TTC
AGT CCC GTG CCC AGT TCC ACC TTC ACC TCC AGC TCC ACC TAT ACC
CCC GGT GAC TGT CCC AAC TTT GCG GCT CCC CGC AGA GAG GTG GCA
CCA CCC TAT CAG GGG GCT GAC CCC ATC CTT GCG ACA GCC CTC GCC
TCC GAC CCC ATC CCC AAC CCC CTT CAG AAG TGG GAG GAC AGC GCC
CAC AAG CCA CAG AGC CTA GAC ACT GAT GAC CCC GCG ACG CTG TAC
GCC GTG GTG GAG AAC GTG CCC CCG TTG CGC TGG AA GGAATTC
 or a fragment, a variant or a modification of this nucleic acid sequence which codes for a polypeptide having the ability to bind TNF.

14. Process for producing a recombinant DNA molecule, comprising the amplification of a DNA, wherein the DNA molecule contains the DNA which can be obtained by a process according to one of claims 1 to 13.

15. Process according to claim 14, wherein the DNA molecule can be replicated in prokaryotic or eukaryotic host cells, and, functionally bound to the DNA which can be obtained by a process according to one of claims 1 to 13, contains expression control sequences.

16. Process according to claim 15, wherein the recombinant DNA molecule can be replicated in mammalian cells.

17. Process according to claim 14, **characterised in that** the recombinant DNA molecule contains the DNA defined in claim 9.

18. Process according to claim 17, **characterised in that** the recombinant DNA molecule is pADTNF-BP, shown in Figure 7a.

19. Process according to claim 17, **characterised in that** the recombinant DNA molecule is pADBTNF-BP, shown in Figure 7b.

20. Process according to claim 17, **characterised in that** the recombinant DNA molecule is pADTNF-R, shown in Figure 7c.

21. Process according to claim 17, **characterised in that** the recombinant DNA molecule is pADBTNF-R, shown in Figure 7d.

22. Process for producing a host cell, comprising the transformation of a cell having at least one of the recombinant DNA molecules which can be obtained by a process according to one of claims 14 to 21, wherein the recombinant DNA molecules represent a partial sequence of the DNA coding for the TNF-binding protein.

23. Process according to claim 22, **characterised in that** the host cell is a eukaryotic host cell, preferably a COS cell or CHO cell.

24. Process for producing a recombinant TNF-binding protein or a functional derivative thereof, comprising the cultivation of a host cell which can be obtained by a process according to claim 22 and expressing of the protein.

25. Process according to claim 24, comprising further the step of isolating the expressed protein.

26. Process for producing a recombinant polypeptide, comprising the expression of a DNA which is selected from the group which codes for one of the following polypeptides:

(i) leu val pro his leu gly asp arg glu lys arg
asp ser val cys pro gln gly lys tyr ile his pro
gln asn asn ser ile cys cys thr lys cys his lys
gly thr tyr leu tyr asn asp cys pro gly pro gly
gln asp thr asp cys arg glu cys glu ser gly ser
phe thr ala ser glu asn his leu arg his cys leu
ser cys ser lys cys arg lys glu met gly gln val
glu ile ser ser cys thr val asp arg asp thr val
cys gly cys arg lys asn gln tyr arg his tyr trp

ser glu asn leu phe gln cys phe asn cys ser leu
cys leu asn gly thr val his leu ser cys gln glu
lys gln asn thr val cys thr cys his ala gly phe
phe leu arg glu asn glu cys val ser cys ser asn
cys lys lys ser leu glu cys thr lys leu cys leu
pro gln ile glu asn ,

(ii)

met gly leu ser thr val pro asp leu leu leu pro
leu val leu leu glu leu leu val gly ile tyr pro
ser gly val ile gly leu val pro his leu gly asp
arg glu lys arg asp ser val cys pro gln gly lys
tyr ile his pro gln asn asn ser ile cys cys thr
lys cys his lys gly thr tyr leu tyr asn asp cys
pro gly pro gly gln asp thr asp cys arg glu cys
glu ser gly ser phe thr ala ser glu asn his leu
arg his cys leu ser cys ser lys cys arg lys glu
met gly gln val glu ile ser ser cys thr val asp
arg asp thr val cys gly cys arg lys asn gln tyr
arg his tyr trp ser glu asn leu phe gln cys phe
asn cys ser leu cys leu asn gly thr val his leu
ser cys gln glu lys gln asn thr val cys thr cys
his ala gly phe phe leu arg glu asn glu cys val
ser cys ser asn cys lys lys ser leu glu cys thr
lys leu cys leu pro gln ile glu asn

(iii)
leu val pro his leu gly asp
arg glu lys arg asp ser val cys pro gln gly lys
tyr ile his pro gln asn asn ser ile cys cys thr
lys cys his lys gly thr tyr leu tyr asn asp cys
pro gly pro gly gln asp thr asp cys arg glu cys
glu ser gly ser phe thr ala ser glu asn his leu
arg his cys leu ser cys ser lys cys arg lys glu
met gly gln val glu ile ser ser cys thr val asp
arg asp thr val cys gly cys arg lys asn gln tyr
arg his tyr trp ser glu asn leu phe gln cys phe
asn cys ser leu cys leu asn gly thr val his leu
ser cys gln glu lys gln asn thr val cys thr cys
his ala gly phe phe leu arg glu asn glu cys val
ser cys ser asn cys lys lys ser leu glu cys thr
lys leu cys leu pro gln ile glu asn val lys gly
thr glu asp ser gly thr thr

27. Process for producing a polypeptide, **characterised in that** a TNF-binding protein of the following formula is expressed in recombinant manner:

asp ser val cys pro gln gly lys tyr ile his pro
gln asn asn ser ile cys cys thr lys cys his lys

gly thr tyr leu tyr asn asp cys pro gly pro gly
gln asp thr asp cys arg glu cys glu ser gly ser
phe thr ala ser glu asn his leu arg his cys leu
ser cys ser lys cys arg lys glu met gly gln val
glu ile ser ser cys thr val asp arg asp thr val
cys gly cys arg lys asn gln tyr arg his tyr trp
ser glu asn leu phe gln cys phe asn cys ser leu
cys leu asn gly thr val his leu ser cys gln glu
lys gln asn thr val cys thr cys his ala gly phe
phe leu arg glu asn glu cys val ser cys ser asn
cys lys lys ser leu glu cys thr lys leu cys leu
pro gln ile glu asn

28. Process according to claim 27, **characterised in that** the polypeptide comprises the TNF receptor of the formula

met gly leu ser thr val pro asp leu leu leu pro
leu val leu leu glu leu leu val gly ile tyr pro
ser gly val ile gly leu val pro his leu gly asp
arg glu lys arg asp ser val cys pro gln gly lys
tyr ile his pro gln asn asn ser ile cys cys thr
lys cys his lys gly thr tyr leu tyr asn asp cys
pro gly pro gly gln asp thr asp cys arg glu cys
glu ser gly ser phe thr ala ser glu asn his leu
arg his cys leu ser cys ser lys cys arg lys glu
met gly gln val glu ile ser ser cys thr val asp
arg asp thr val cys gly cys arg lys asn gln tyr
arg his tyr trp ser glu asn leu phe gln cys phe
asn cys ser leu cys leu asn gly thr val his leu
ser cys gln glu lys gln asn thr val cys thr cys
his ala gly phe phe leu arg glu asn glu cys val
ser cys ser asn cys lys lys ser leu glu cys thr
lys leu cys leu pro gln ile glu asn val lys gly
thr glu asp ser gly thr thr val leu leu pro leu
val ile phe phe gly leu cys leu leu ser leu leu
phe ile gly leu met tyr arg tyr gln arg trp lys
ser lys leu tyr ser ile val cys gly lys ser thr
pro glu lys glu gly glu leu glu gly thr thr thr
lys pro leu ala pro asn pro ser phe ser pro thr
pro gly phe thr pro thr leu gly phe ser pro val
pro ser ser thr phe thr ser ser ser thr tyr thr
pro gly asp cys pro asn phe ala ala pro arg arg
glu val ala pro pro tyr gln gly ala asp pro ile
leu ala thr ala leu ala ser asp pro ile pro asn
pro leu gln lys trp glu asp ser ala his lys pro
gln ser leu asp thr asp asp pro ala thr leu tyr
ala val val glu asn val pro pro leu arg trp

29. Process for investigating substances, **characterised in that** a polypeptide which can be obtained according to one of claims 26 to 28 is used for determining the interaction of these substances with said polypeptide and/or with TNF-α and/or with TNF-β and/or the influence of these substances on the biological effect of TNF-α and/or TNF-β *in vitro*.

30. Process for investigating substances, **characterised in that** the interaction of these substances with the TNF receptor and/or its influence of the biological effect of TNF-α and/or TNF-β is determined using a host cell which can be obtained according to claim 24.

31. Process for producing a medicament for treating autoimmune diseases, **characterised in that** a polypeptide, which can be obtained by a process according to one of claims 26 to 28, is combined with pharmaceutically acceptable additives.

32. Process for producing a medicament for treating rheumatoid arthritis, **characterised in that** a polypeptide, which can be obtained by a process according to one of claims 26 to 28, is combined with pharmaceutically acceptable additives.

33. Process for producing a medicament for treating ARDS, **characterised in that** a polypeptide, which can be obtained by a process according to one of claims 26 to 28, is combined with pharmaceutically acceptable additives.

34. Process for producing a medicament for treating anorexia/cachexia, **characterised in that** a polypeptide, which can be obtained by a process according to one of claims 26 to 28, is combined with pharmaceutically acceptable additives.

35. Process for producing a medicament for treating GVHR, **characterised in that** a polypeptide, which can be obtained by a process according to one of claims 26 to 28, is combined with pharmaceutically acceptable additives.

36. Process for producing a medicament for treating shock, **characterised in that** a polypeptide, which can be obtained by a process according to one of claims 26 to 28, is combined with pharmaceutically acceptable additives.

37. Process for producing a diagnostic agent for determining TNF-α and/or TNF-β, **characterised in that** a polypeptide, which can be obtained by a process according to one of claims 26 to 28, is provided together with antibodies against TNF.

38. Process for producing a pharmaceutical preparation, **characterised in that** a polypeptide, which can be obtained by a process according to one of claims 26 to 28, is combined with pharmaceutically acceptable additives.

## Claims (Claims for the following Contracting State(s): GR)

1. DNA coding for a polypeptide having the ability to bind TNF, wherein the polypeptide comprises an amino acid sequence having the formula 
asp ser val cys pro gln gly lys tyr ile his pro
gln asn asn ser ile cys cys thr lys cys his lys
gly thr tyr leu tyr asn asp cys pro gly pro gly
gln asp thr asp cys arg glu cys glu ser gly ser
phe thr ala ser glu asn his leu arg his cys leu
ser cys ser lys cys arg lys glu met gly gln val
glu ile ser ser cys thr val asp arg asp thr val
cys gly cys arg lys asn gln tyr arg his tyr trp
ser glu asn leu phe gln cys phe asn cys ser leu
cys leu asn gly thr val his leu ser cys gln glu
lys gln asn thr val cys thr cys his ala gly phe
phe leu arg glu asn glu cys val ser cys ser asn
cys lys lys ser leu glu cys thr lys leu cys leu
pro gln ile glu asn
 or a functional fragment or a modification thereof having the ability to bind TNF.

2. DNA according to claim 1, **characterised in that** the polypeptide comprises an amino acid sequence having the formula 
leu val pro his leu gly asp arg glu lys arg
asp ser val cys pro gln gly lys tyr ile his pro
gln asn asn ser ile cys cys thr lys cys his lys
gly thr tyr leu tyr asn asp cys pro gly pro gly
gln asp thr asp cys arg glu cys glu ser gly ser
phe thr ala ser glu asn his leu arg his cys leu
ser cys ser lys cys arg lys glu met gly gln val
glu ile ser ser cys thr val asp arg asp thr val
cys gly cys arg lys asn gln tyr arg his tyr trp
ser glu asn leu phe gln cys phe asn cys ser leu
cys leu asn gly thr val his leu ser cys gln glu
lys gln asn thr val cys thr cys his ala gly phe
phe leu arg glu asn glu cys val ser cys ser asn
cys lys lys ser leu glu cys thr lys leu cys leu
pro gln ile glu asn
 or a functional fragment or a modification thereof having the ability to bind TNF.

3. DNA according to claim 2, **characterised in that** the polypeptide comprises an amino acid sequence having the formula 
met gly leu ser thr val pro asp leu leu leu pro
leu val leu leu glu leu leu val gly ile tyr pro
ser gly val ile gly leu val pro his leu gly asp
arg glu lys arg asp ser val cys pro gln gly lys
tyr ile his pro gln asn asn ser ile cys cys thr
lys cys his lys gly thr tyr leu tyr asn asp cys
pro gly pro gly gln asp thr asp cys arg glu cys
glu ser gly ser phe thr ala ser glu asn his leu
arg his cys leu ser cys ser lys cys arg lys glu
met gly gln val glu ile ser ser cys thr val asp
arg asp thr val cys gly cys arg lys asn gln tyr
arg his tyr trp ser glu asn leu phe gln cys phe
asn cys ser leu cys leu asn gly thr val his leu
ser cys gln glu lys gln asn thr val cys thr cys
his ala gly phe phe leu arg glu asn glu cys val
ser cys ser asn cys lys lys ser leu glu cys thr
lys leu cys leu pro gln ile glu asn
 or a functional fragment or a modification thereof having the ability to bind TNF.

4. DNA according to claim 3, **characterised in that** the polypeptide comprises an amino acid sequence having the formula 
met gly leu ser thr val pro asp leu leu leu pro
leu val leu leu glu leu leu val gly ile tyr pro
ser gly val ile gly leu val pro his leu gly asp
arg glu lys arg asp ser val cys pro gln gly lys
tyr ile his pro gln asn asn ser ile cys cys thr
lys cys his lys gly thr tyr leu tyr asn asp cys
pro gly pro gly gln asp thr asp cys arg glu cys
glu ser gly ser phe thr ala ser glu asn his leu
arg his cys leu ser cys ser lys cys arg lys glu
met gly gln val glu ile ser ser cys thr val asp
arg asp thr val cys gly cys arg lys asn gln tyr
arg his tyr trp ser glu asn leu phe gln cys phe
asn cys ser leu cys leu asn gly thr val his leu
ser cys gln glu lys gln asn thr val cys thr cys
his ala gly phe phe leu arg glu asn glu cys val
ser cys ser asn cys lys lys ser leu glu cys thr

lys leu cys leu pro gln ile glu asn val lys gly
thr glu asp ser gly thr thr val leu leu pro leu
val ile phe phe gly leu cys leu leu ser leu leu
phe ile gly leu met tyr arg tyr gln arg trp lys
ser lys leu tyr ser ile val cys gly lys ser thr
pro glu lys glu gly glu leu glu gly thr thr thr
lys pro leu ala pro asn pro ser phe ser pro thr
pro gly phe thr pro thr leu gly phe ser pro val
pro ser ser thr phe thr ser ser ser thr tyr thr
pro gly asp cys pro asn phe ala ala pro arg arg
glu val ala pro pro tyr gln gly ala asp pro ile
leu ala thr ala leu ala ser asp pro ile pro asn
pro leu gln lys trp glu asp ser ala his lys pro
gln ser leu asp thr asp asp pro ala thr leu tyr

ala val val glu asn val pro pro leu arg trp
 or a functional fragment or a modification thereof having the ability to bind TNF.

5. DNA according to claim 1, **characterised in that** the polypeptide comprises an amino acid sequence having the formula 
asp ser val cys pro gln gly lys
tyr ile his pro gln asn asn ser ile cys cys thr
lys cys his lys gly thr tyr leu tyr asn asp cys
pro gly pro gly gln asp thr asp cys arg glu cys
glu ser gly ser phe thr ala ser glu asn his leu
arg his cys leu ser cys ser lys cys arg lys glu
met gly gln val glu ile ser ser cys thr val asp
arg asp thr val cys gly cys arg lys asn gln tyr
arg his tyr trp ser glu asn leu phe gln cys phe
asn cys ser leu cys leu asn gly thr val his leu
ser cys gln glu lys gln asn thr val cys thr cys
his ala gly phe phe leu arg glu asn glu cys val
ser cys ser asn cys lys lys ser leu glu cys thr
lys leu cys leu pro gln ile glu asn val lys gly
thr glu asp ser gly thr thr
 or a functional fragment or a modification thereof having the ability to bind TNF.

6. DNA according to claim 5. **characterised in that** the polypeptide comprises an amino acid sequence having the formula 
leu val pro his leu gly asp
arg glu lys arg asp ser val cys pro gln gly lys
tyr ile his pro gln asn asn ser ile cys cys thr
lys cys his lys gly thr tyr leu tyr asn asp cys
pro gly pro gly gln asp thr asp cys arg glu cys
glu ser gly ser phe thr ala ser glu asn his leu
arg his cys leu ser cys ser lys cys arg lys glu
met gly gln val glu ile ser ser cys thr val asp
arg asp thr val cys gly cys arg lys asn gln tyr
arg his tyr trp ser glu asn leu phe gln cys phe
asn cys ser leu cys leu asn gly thr val his leu
ser cys gln glu lys gln asn thr val cys thr cys
his ala gly phe phe leu arg glu asn glu cys val
ser cys ser asn cys lys lys ser leu glu cys thr
lys leu cys leu pro gln ile glu asn val lys gly
thr glu asp ser gly thr thr
 or a functional fragment or a modification thereof having the ability to bind TNF.

7. DNA according to claim 1, **characterised in that** the amino acid sequence of the polypeptide is altered at one or more glycosylation sites.

8. DNA according to claim 1, coding for secretable TNF-binding protein, **characterised in that** it comprises a nucleic acid sequence having the formula 
R= GAT AGT GTG TGT CCC CAA GGA AAA TAT ATC CAC
CCT CAA AAT AAT TCG ATT TGC TGT ACC AAG TGC CAC
AAA GGA ACC TAC TTG TAC AAT GAC TGT CCA GGC CCG
GGG CAG GAT ACG GAC TGC AGG GAG TGT GAG AGC GGC
TCC TTC ACC GCT TCA GAA AAC CAC CTC AGA CAC TGC
CTC AGC TGC TCC AAA TGC CGA AAG GAA ATG GGT CAG
GTG GAG ATC TCT TCT TGC ACA GTG GAC CGG GAC ACC
GTG TGT GGC TGC AGG AAG AAC CAG TAC CGG CAT TAT
TGG AGT GAA AAC CTT TTC CAG TGC TTC AAT TGC AGC
CTC TGC CTC AAT GGG ACC GTG CAC CTC TCC TGC CAG
GAG AAA CAG AAC ACC GTG TGC ACC TGC CAT GCA GGT
TTC TTT CTA AGA GAA AAC GAG TGT GTC TCC TGT AGT
AAC TGT AAG AAA AGC CTG GAG TGC ACG AAG TTG TGC
CTA CCC CAG ATT GAG AAT
 or a fragment, a variant or a modification of this nucleic acid sequence which codes for a polypeptide having the ability to bind TNF, wherein R² represents a DNA coding for a (poly)peptide which can be cleaved *in vivo*.

9. DNA according to claim 8, coding for secretable TNF-binding protein, **characterised in that** R² represents a DNA coding completely or partly for a signal sequence.

10. DNA according to claim 8, **characterised in that** R² comprises a nucleic acid sequence having the formula

CTG GTC CCT CAC CTA GGG GAC AGG GAG AAG AGA

11. DNA according to claim 9, **characterised in that** R² comprises a nucleic acid having the formula R3 CTG GTC CCT CAC CTA GGG GAC AGG GAG AAG AGA wherein R³ represents a DNA coding for a signal peptide.

12. DNA according to claim 11, **characterised in that** R³ comprises a nucleic acid sequence having the formula

ATG GGC CTC TCC ACC GTG CCT GAC CTG CTG CTG CGA
CTG GTG CTC CTG GAG CTG TTG GTG GGA ATA TAC CCC
TCA GGG GTT ATT GGA

13. DNA according to claim 1, **characterised in that** it comprises DNA having the formula 
ATG GGC CTC TCC ACC GTG CCT GAC CTG CTG CTG CCA CTG GTG CTC
CTG GAG CTG TTG GTG GGA ATA TAC CCC TCA GGG GTT ATT GGA CTG
GTC CCT CAC CTA GGG GAC AGG GAG AAG AGA|GAT AGT GTG TGT CCC
CAA GGA AAA TAT ATC CAC CCT CAA AAT AAT TCG ATT TGC TGT ACC
AAG TGC CAC AAA GGA ACC TAC TTG TAC AAT GAC TGT CCA GGC CCG
GGG CAG GAT ACG GAC TGC AGG GAG TGT GAG AGC GGC TCC TTC ACC
GCT TCA GAA AAC CAC CTC AGA CAC TGC CTC AGC TGC TCC AAA TGC
CGA AAG GAA ATG GGT CAG GTG GAG ATC TCT TCT TGC ACA GTG GAC
CGG GAC ACC GTG TGT GGC TGC AGG AAG AAC CAG TAC CGG CAT TAT
TGG AGT GAA AAC CTT TTC CAG TGC TTC AAT TGC AGC CTC TGC CTC
 
AAT GGG ACC GTG CAC CTC TCC TGC CAG GAG AAA CAG AAC ACC GTG
TGC ACC TGC CAT GCA GGT TTG TTT CTA AGA GAA AAC GAG TGT GTC
TCC TGT AGT AAC TGT AAG AAA AGC CTG GAG TGC ACG AAG TTG TGC
CTA CCC CAG ATT GAG AAT GTT AAG GGC ACT GAG GAC TCA GGC ACC
ACA GTG CTG TTG CCC CTG GTC ATT TTC TTT GGT CTT TGC CTT TTA
TCC CTC CTC TTC ATT GGT TTA ATG TAT CGC TAC CAA CGG TGG AAG
TCC AAG CTC TAC TCC ATT GTT TGT GGG AAA TCG ACA CCT GAA AAA
GAG GGG GAG CTT GAA GGA ACT ACT ACT AAG CCC CTG GCC CCA AAC
CCA AGC TTC AGT CCC ACT CCA GGC TTC ACC CCC ACC CTG GGC TTC
AGT CCC GTG CCC AGT TCC ACC TTC ACC TCC AGC TCC ACC TAT ACC
CCC GGT GAC TGT CCC AAC TTT GCG GCT CCC CGC AGA GAG GTG GCA
CCA CCC TAT CAG GGG GCT GAC CCC ATC CTT GCG ACA GCC CTC GCC
TCC GAC CCC ATC CCC AAC CCC CTT CAG AAG TGG GAG GAC AGC GCC
CAC AAG CCA CAG AGC CTA GAC ACT GAT GAC CCC GCG ACG CTG TAC
GCC GTG GTG GAG AAC GTG CCC CCG TTG CGC TGG AA GGAATTC
 or a fragment, a variant or a modification of this nucleic acid sequence which codes for a polypeptide having the ability to bind TNF.

14. Recombinant DNA molecule, **characterised in that** it contains the DNA according to one of claims 1 to 13.

15. Recombinant DNA molecule according to claim 14, which can be replicated in prokaryotic or eukaryotic host cells, **characterised in that**, functionally bound to the DNA according to one of claims 1 to 13, it contains expression control sequences.

16. Recombinant DNA molecule according to claim 15, which can be replicated in mammalian cells.

17. Recombinant DNA molecule according to claim 14, **characterised in that** it contains the DNA defined in claim 9.

18. Recombinant DNA molecule according to claim 17 having the designation pADTNF-BP, shown in Figure 7a.

19. Recombinant DNA molecule according to claim 17 having the designation pADBTNF-BP, shown in Figure 7b.

20. Recombinant DNA molecule according to claim 17 having the designation pADTNF-R, shown in Figure 7c.

21. Recombinant DNA molecule according to claim 17 having the designation pADBTNF-R, shown in Figure 7d.

22. Host cell, containing at least one of the recombinant DNA molecules defined in claims 14 to 21, which represents a partial sequence of the DNA coding for the TNF-binding protein.

23. Host cell according to claim 22, **characterised in that** it is a eukaryotic host cell, preferably a COS cell or CHO cell.

24. Process for producing a recombinant TNF-binding protein or a functional derivative thereof, comprising the cultivation of a host according to claim 22 and expressing of the protein.

25. Process according to claim 24, comprising further the step of isolating the expressed protein.

26. Recombinant polypeptide, **characterised in that** it is coded by one of the DNAs which is selected from the group which codes for one of the following polypeptides:

(i) leu val pro his leu gly asp arg glu lys arg
asp ser val cys pro gln gly lys tyr ile his pro
gln asn asn ser ile cys cys thr lys cys his lys
gly thr tyr leu tyr asn asp cys pro gly pro gly
gln asp thr asp cys arg glu cys glu ser gly ser
phe thr ala ser glu asn his leu arg his cys leu
ser cys ser lys cys arg lys glu met gly gln val
glu ile ser ser cys thr val asp arg asp thr val
cys gly cys arg lys asn gln tyr arg his tyr trp
ser glu asn leu phe gln cys phe asn cys ser leu
cys leu asn gly thr val his leu ser cys gln glu
lys gln asn thr val cys thr cys his ala gly phe
phe leu arg glu asn glu cys val ser cys ser asn
cys lys lys ser leu glu cys thr lys leu cys leu
pro gln ile glu asn ,

(II)

met gly leu ser thr val pro asp leu leu leu pro
leu val leu leu glu leu leu val gly ile tyr pro
ser gly val ile gly leu val pro his leu gly asp
arg glu lys arg asp ser val cys pro gln gly lys
tyr ile his pro gln asn asn ser ile cys cys thr
lys cys his lys gly thr tyr leu tyr asn asp cys
pro gly pro gly gln asp thr asp cys arg glu cys
glu ser gly ser phe thr ala ser glu asn his leu
arg his cys leu ser cys ser lys cys arg lys glu
met gly gln val glu ile ser ser cys thr val asp
arg asp thr val cys gly cys arg lys asn gln tyr
arg his tyr trp ser glu asn leu phe gln cys phe
asn cys ser leu cys leu asn gly thr val his leu
ser cys gln glu lys gln asn thr val cys thr cys
his ala gly phe phe leu arg glu asn glu cys val
ser cys ser asn cys lys lys ser leu glu cys thr
lys leu cys leu pro gln ile glu asn

(iii) leu val pro his leu gly asp
arg glu lys arg asp ser val cys pro gln gly lys
tyr ile his pro gln asn asn ser ile cys cys thr
lys cys his lys gly thr tyr leu tyr asn asp cys
pro gly pro gly gln asp thr asp cys arg glu cys
glu ser gly ser phe thr ala ser glu asn his leu
arg his cys leu ser cys ser lys cys arg lys glu
met gly gln val glu ile ser ser cys thr val asp
arg asp thr val cys gly cys arg lys asn gln tyr
arg his tyr trp ser glu asn leu phe gln cys phe
asn cys ser leu cys leu asn gly thr val his leu
ser cys gln glu lys gln asn thr val cys thr cys
his ala gly phe phe leu arg glu asn glu cys val
ser cys ser asn cys lys lys ser leu glu cys thr
lys leu cys leu pro gln ile glu asn val lys gly
thr glu asp ser gly thr thr

27. Polypeptide, **characterised in that** it comprises a TNF-binding protein of the formula:

asp ser val cys pro gln gly lys tyr ile his pro
gln asn asn ser ile cys cys thr lys cys his lys

gly thr tyr leu tyr asn asp cys pro gly pro gly
gln asp thr asp cys arg glu cys glu ser gly ser
phe thr ala ser glu asn his leu arg his cys leu
ser cys ser lys cys arg lys glu met gly gln val
glu ile ser ser cys thr val asp arg asp thr val
cys gly cys arg lys asn gln tyr arg his tyr trp
ser glu asn leu phe gln cys phe asn cys ser leu
cys leu asn gly thr val his leu ser cys gln glu
lys gln asn thr val cys thr cys his ala gly phe
phe leu arg glu asn glu cys val ser cys ser asn
cys lys lys ser leu glu cys thr lys leu cys leu
pro gln ile glu asn

28. Polypeptide according to claim 27, **characterised in that** it comprises the TNF receptor of the formula

met gly leu ser thr val pro asp leu leu leu pro
leu val leu leu glu leu leu val gly ile tyr pro
ser gly val ile gly leu val pro his leu gly asp
arg glu lys arg asp ser val cys pro gln gly lys
tyr ile his pro gln asn asn ser ile cys cys thr
lys cys his lys gly thr tyr leu tyr asn asp cys
pro gly pro gly gln asp thr asp cys arg glu cys
glu ser gly ser phe thr ala ser glu asn his leu
arg his cys leu ser cys ser lys cys arg lys glu
met gly gln val glu ile ser ser cys thr val asp
arg asp thr val cys gly cys arg lys asn gln tyr
arg his tyr trp ser glu asn leu phe gln cys phe
asn cys ser leu cys leu asn gly thr val his leu
ser cys gln glu lys gln asn thr val cys thr cys
his ala gly phe phe leu arg glu asn glu cys val
ser cys ser asn cys lys lys ser leu glu cys thr
lys leu cys leu pro gln ile glu asn val lys gly
thr glu asp ser gly thr thr val leu leu pro leu
val ile phe phe gly leu cys leu leu ser leu leu
phe ile gly leu met tyr arg tyr gln arg trp lys
ser lys leu tyr ser ile val cys gly lys ser thr
pro glu lys glu gly glu leu glu gly thr thr thr
lys pro leu ala pro asn pro ser phe ser pro thr
pro gly phe thr pro thr leu gly phe ser pro val
pro ser ser thr phe thr ser ser ser thr tyr thr
pro gly asp cys pro asn phe ala ala pro arg arg
glu val ala pro pro tyr gln gly ala asp pro ile
leu ala thr ala leu ala ser asp pro ile pro asn
pro leu gln lys trp glu asp ser ala his lys pro
gln ser leu asp thr asp asp pro ala thr leu tyr
ala val val glu asn val pro pro leu arg trp

29. Use of a polypeptide defined in claims 26 to 28 for investigating substances for their interaction with this polypeptide and/or with TNF-α and/or with TNF-β and/or for their influence of the biological effect of TNF-α and/or TNF-β *in vitro.*

30. Use of a host cell defined in claim 24 for investigating substances for their interaction with the TNF receptor and/or their influence of the biological effect of TNF-α and/or TNF-β.

31. Use of a polypeptide according to one of claims 26 to 28 for producing a medicament for treating autoimmune diseases.

32. Use of a polypeptide according to one of claims 26 to 28 for producing a medicament for treating rheumatoid arthritis.

33. Use of a polypeptide according to one of claims 26 to 28 for producing a medicament for treating ARDS.

34. Use of a polypeptide according to one of claims 26 to 28 for producing a medicament for treating anorexia/cachexia.

35. Use of a polypeptide according to one of claims 26 to 28 for producing a medicament for treating GVHR.

36. Use of a polypeptide according to one of claims 26 to 28 for producing a medicament for treating shock.

37. Use of a polypeptide according to one of claims 26 to 28 as a diagnostic agent for determining TNF-α and/or TNF-β.

38. Process for producing a pharmaceutical preparation, **characterised in that** a polypeptide defined in one of claims 26 to 28 is combined with pharmaceutically acceptable additives.

## Revendications (Revendications pour l'(les) Etat(s) contractant(s) suivant(s): AT, BE, CH, DE, DK, FR, GB, IT, LI, LU, NL, SE)

1. ADN, codant pour un polypeptide ayant la capacité de lier le TNF, dans lequel le polypeptide comprend une séquence d'acides aminés de formule : 
asp ser val cys pro gln gly lys tyr ile his pro
gln asn asn ser ile cys cys thr lys cys his lys
gly thr tyr leu tyr asn asp cys pro gly pro gly
gln asp thr asp cys arg glu cys glu ser gly ser
phe thr ala ser glu asn his leu arg his cys leu
ser cys ser lys cys arg lys glu met gly gln val
glu ile ser ser cys thr val asp arg asp thr val
cys gly cys arg lys asn gln tyr arg his tyr trp
ser glu asn leu phe gln cys phe asn cys ser leu
cys leu asn gly thr val his leu ser cys gln glu
lys gln asn thr val cys thr cys his ala gly phe
phe leu arg glu asn glu cys val ser cys ser asn
cys lys lys ser leu glu cys thr lys leu cys leu
pro gln ile glu asn
 ou un fragment fonctionnel ou une modification de celui-ci ayant la capacité de lier le TNF.

2. ADN selon la revendication 1, **caractérisé en ce que** le polypeptide comprend une séquence d'acides aminés de formule : 
leu val pro his leu gly asp arg glu lys arg
asp ser val cys pro gln gly lys tyr ile his pro
gln asn asn ser ile cys cys thr lys cys his lys
gly thr tyr leu tyr asn asp cys pro gly pro gly
gln asp thr asp cys arg glu cys glu ser gly ser
phe thr ala ser glu asn his leu arg his cys leu
ser cys ser lys cys arg lys glu met gly gln val
glu ile ser ser cys thr val asp arg asp thr val
cys gly cys arg lys asn gln tyr arg his tyr trp
ser glu asn leu phe gln cys phe asn cys ser leu
cys leu asn gly thr val his leu ser cys gln glu
lys gln asn thr val cys thr cys his ala gly phe
phe leu arg glu asn glu cys val ser cys ser asn
cys lys lys ser leu glu cys thr lys leu cys leu
pro gln ile glu asn
 ou un fragment fonctionnel ou une modification de celui-ci ayant la capacité de lier le TNF.

3. ADN selon la revendication 2, **caractérisé en ce que** le polypeptide comprend une séquence d'acides aminés de formule : 
met gly leu ser thr val pro asp leu leu leu pro
leu val leu leu glu leu leu val gly ile tyr pro
ser gly val ile gly leu val pro his leu gly asp
arg glu lys arg asp ser val cys pro gln gly lys
tyr ile his pro gln asn asn ser ile cys cys thr
lys cys his lys gly thr tyr leu tyr asn asp cys
pro gly pro gly gln asp thr asp cys arg glu cys
glu ser gly ser phe thr ala ser glu asn his leu
arg his cys leu ser cys ser lys cys arg lys glu
met gly gln val glu ile ser ser cys thr val asp
arg asp thr val cys gly cys arg lys asn gln tyr
arg his tyr trp ser glu asn leu phe gln cys phe
asn cys ser leu cys leu asn gly thr val his leu
ser cys gln glu lys gln asn thr val cys thr cys
his ala gly phe phe leu arg glu asn glu cys val
ser cys ser asn cys lys lys ser leu glu cys thr
lys leu cys leu pro gln ile glu asn
 ou un fragment fonctionnel ou une modification de celui-ci ayant la capacité de lier le TNF.

4. ADN selon la revendication 3, **caractérisé en ce que** le polypeptide comprend une séquence d'acides aminés de formule : 
met gly leu ser thr val pro asp leu leu leu pro
leu val leu leu glu leu leu val gly ile tyr pro
ser gly val ile gly leu val pro his leu gly asp
arg glu lys arg asp ser val cys pro gln gly lys
tyr ile his pro gln asn asn ser ile cys cys thr
lys cys his lys gly thr tyr leu tyr asn asp cys
pro gly pro gly gln asp thr asp cys arg glu cys
glu ser gly ser phe thr ala ser glu asn his leu
arg his cys leu ser cys ser lys cys arg lys glu
met gly gln val glu ile ser ser cys thr val asp
arg asp thr val cys gly cys arg lys asn gln tyr
arg his tyr trp ser glu asn leu phe gln cys phe
asn cys ser leu cys leu asn gly thr val his leu
ser cys gln glu lys gln asn thr val cys thr cys
his ala gly phe phe leu arg glu asn glu cys val
ser cys ser asn cys lys lys ser leu glu cys thr
lys leu cys leu pro gln ile glu asn val lys gly
thr glu asp ser gly thr thr val leu leu pro leu
val ile phe phe gly leu cys leu leu ser leu leu
phe ile gly leu met tyr arg tyr gln arg trp lys
ser lys leu tyr ser ile val cys gly lys ser thr
pro glu lys glu gly glu leu glu gly thr thr thr
lys pro leu ala pro asn pro ser phe ser pro thr
pro gly phe thr pro thr leu gly phe ser pro val
pro ser ser thr phe thr ser ser ser thr tyr thr
pro gly asp cys pro asn phe ala ala pro arg arg
glu val ala pro pro tyr gln gly ala asp pro ile
leu ala thr ala leu ala ser asp pro ile pro asn
pro leu gln lys trp glu asp ser ala his lys pro
gln ser leu asp thr asp asp pro ala thr leu tyr

ala val val glu asn val pro pro leu arg trp
 ou un fragment fonctionnel ou une modification de celui-ci ayant la capacité de lier le TNF.

5. ADN selon la revendication 1, **caractérisé en ce que** le polypeptide comprend une séquence d'acides aminés de formule : 
asp ser val cys pro gln gly lys
tyr ile his pro gln asn asn ser ile cys cys thr
lys cys his lys gly thr tyr leu tyr asn asp cys
pro gly pro gly gln asp thr asp cys arg glu cys
glu ser gly ser phe thr ala ser glu asn his leu
arg his cys leu ser cys ser lys cys arg lys glu
met gly gln val glu ile ser ser cys thr val asp
arg asp thr val cys gly cys arg lys asn gln tyr
arg his tyr trp ser glu asn leu phe gln cys phe
asn cys ser leu cys leu asn gly thr val his leu
ser cys gln glu lys gln asn thr val cys thr cys
his ala gly phe phe leu arg glu asn glu cys val
ser cys ser asn cys lys lys ser leu glu cys thr
lys leu cys leu pro gln ile glu asn val lys gly
thr glu asp ser gly thr thr
 ou un fragment fonctionnel ou une modification de celui-ci ayant la capacité de lier le TNF.

6. ADN selon la revendication 5, **caractérisé en ce que** le polypeptide comprend une séquence d'acides aminés de formule : 
leu val pro his leu gly asp
arg glu lys arg asp ser val cys pro gln gly lys
tyr ile his pro gln asn asn ser ile cys cys thr
lys cys his lys gly thr tyr leu tyr asn asp cys
pro gly pro gly gln asp thr asp cys arg glu cys
glu ser gly ser phe thr ala ser glu asn his leu
arg his cys leu ser cys ser lys cys arg lys glu
met gly gln val glu ile ser ser cys thr val asp
arg asp thr val cys gly cys arg lys asn gln tyr
arg his tyr trp ser glu asn leu phe gln cys phe
asn cys ser leu cys leu asn gly thr val his leu
ser cys gln glu lys gln asn thr val cys thr cys
his ala gly phe phe leu arg glu asn glu cys val
ser cys ser asn cys lys lys ser leu glu cys thr
lys leu cys leu pro gln ile glu asn val lys gly
thr glu asp ser gly thr thr
 ou un fragment fonctionnel ou une modification de celui-ci ayant la capacité de lier le TNF.

7. ADN selon la revendication 1, **caractérisé en ce que** la séquence d'acides aminés du polypeptide est modifiée en un ou plusieurs sites de glycosylation.

8. ADN selon la revendication 1, codant pour une protéine pouvant être sécrétée liant le TNF, **caractérisé en ce qu'**elle comprend la séquence d'acides nucléiques de formule 
R= GAT AGT GTG TGT CCC CAA GGA AAA TAT ATC CAC
CCT CAA AAT AAT TCG ATT TGC TGT ACC AAG TGC CAC
AAA GGA ACC TAC TTG TAC AAT GAC TGT CCA GGC CCG
GGG CAG GAT ACG GAC TGC AGG GAG TGT GAG AGC GGC
TCC TTC ACC GCT TCA GAA AAC CAC CTC AGA CAC TGC
CTC AGC TGC TCC AAA TGC CGA AAG GAA ATG GGT CAG
GTG GAG ATC TCT TCT TGC ACA GTG GAC CGG GAC ACC
GTG TGT GGC TGC AGG AAG AAC CAG TAC CGG CAT TAT
TGG AGT GAA AAC CTT TTC CAG TGC TTC AAT TGC AGC
CTC TGC CTC AAT GGG ACC GTG CAC CTC TCC TGC CAG
GAG AAA CAG AAC ACC GTG TGC ACC TGC CAT GCA GGT
TTC TTT CTA AGA GAA AAC GAG TGT GTC TCC TGT AGT
AAC TGT AAG AAA AGC CTG GAG TGC ACG AAG TTG TGC
CTA CCC CAG ATT GAG AAT
 ou un fragment, une variante ou modification de cette séquence d'acides nucléiques, qui code pour un polypeptide ayant la capacité de lier le TNF, dans lequel R² représente un ADN codant pour un (poly)peptide pouvant être excisé in vivo.

9. ADN selon la revendication 8, codant pour une protéine pouvant être sécrétée liant le TNF, **caractérisé en ce que** R² est un ADN codant pour tout ou partie d'une séquence signal.

10. ADN selon la revendication 8, caractérisé en que R² comprend une séquence d'acides nucléiques de formule

CTG GTC CCT CAC CTA GGG GAC AGG GAG AAG AGA

11. ADN selon la revendication 9, caractérisé en que R² comprend une séquence d'acides nucléiques de formule R3 CTG GTC CCT CAC-CTA GGG GAC AGG GAG AAG AGA , dans laquelle R³ représente un ADN codant pour un peptide signal.

12. ADN selon la revendication 11, caractérisé en que R³ comprend une séquence d'acides nucléiques de formule

ATG GGC CTC TCC ACC GTG CCT GAC CTG CTG CTG CGA
CTG GTG CTC CTG GAG CTG TTG GTG GGA ATA TAC CCC
TCA GGG GTT ATT GGA

13. ADN selon la revendication 1, **caractérisé en ce que** l'ADN comprend un ADN de formule 
ATG GGC CTC TCC ACC GTG CCT GAC CTG CTG CTG CCA CTG GTG CTC
CTG GAG CTG TTG GTG GGA ATA TAC CCC TCA GGG GTT ATT GGA CTG
GTC CCT CAC CTA GGG GAC AGG GAG AAG AGA GAT AGT GTG TGT CCC
CAA GGA AAA TAT ATC CAC CCT CAA AAT AAT TCG ATT TGC TGT ACC
AAG TGC CAC AAA GGA ACC TAC TTG TAC AAT GAC TGT CCA GGC CCG
GGG CAG GAT ACG GAC TGC AGG GAG TGT GAG AGC GGC TCC TTC ACC
GCT TCA GAA AAC CAC CTC AGA CAC TGC CTC AGC TGC TCC AAA TGC
CGA AAG GAA ATG GGT CAG GTG GAG ATC TCT TCT TGC ACA GTG GAC
CGG GAC ACC GTG TGT GGC TGC AGG AAG AAC CAG TAC CGG CAT TAT
TGG AGT GAA AAC CTT TTC CAG TGC TTC AAT TGC AGC CTC TGC CTC

AAT GGG ACC GTG CAC CTC TCC TGC CAG GAG AAA CAG AAC ACC GTG
TGC ACC TGC CAT GCA GGT TTC TTT CTA AGA GAA AAC GAG TGT GTC
TCC TGT AGT AAC TGT AAG AAA AGC CTG GAG TGC ACG AAG TTG TGC
CTA CCC CAG ATT GAG AAT GTT AAG GGC ACT GAG GAC TCA GGC ACC
ACA GTG CTG TTG CCC CTG GTC ATT TTC TTT GGT CTT TGC CTT TTA
TCC CTC CTC TTC ATT GGT TTA ATG TAT CGC TAC CAA CGG TGG AAG
TCC AAG CTC TAC TCC ATT GTT TGT GGG AAA TCG ACA CCT GAA AAA
GAG GGG GAG CTT GAA GGA ACT ACT ACT AAG CCC CTG GCC CCA AAC
CCA AGC TTC AGT CCC ACT CCA GGC TTC ACC CCC ACC CTG GGC TTC
AGT CCC GTG CCC AGT TCC ACC TTC ACC TCC AGC TCC ACC TAT ACC
CCC GGT GAC TGT CCC AAC TTT GCG GCT CCC CGC AGA GAG GTG GCA
CCA CCC TAT CAG GGG GCT GAC CCC ATC CTT GCG ACA GCC CTC GCC
TCC GAC CCC ATC CCC AAC CCC CTT CAG AAG TGG GAG GAC AGC GCC
CAC AAG CCA CAG AGC CTA GAC ACT GAT GAC CCC GCG ACG CTG TAC
GCC GTG GTG GAG AAC GTG CCC CCG TTG CGC TGG AA GGAATTC
 ou un fragment, une variante ou modification de cette séquence d'acides nucléiques, qui code pour un polypeptide ayant la capacité de lier le TNF.

14. Molécule d'ADN recombinante, **caractérisée en ce qu'**elle comprend l'ADN selon l'une des revendications 1 à 13.

15. Molécule d'ADN recombinante selon la revendication 14, réplicable dans des cellules hôtes procaryotes ou eucaryotes, **caractérisée en ce qu'**elle comprend des séquences de contrôle de l'expression liées de façon fonctionnelle à l'ADN selon l'une des revendications 1 à 13.

16. Molécule d'ADN recombinante selon la revendication 15, réplicable dans des cellules de mammifère.

17. Molécule d'ADN recombinante selon la revendication 14, **caractérisée en ce qu'**elle comprend l'ADN défini dans la revendication 9.

18. Molécule d'ADN recombinante selon la revendication 17, désignée pADTNF-BP, présentée sur la figure 7a.

19. Molécule d'ADN recombinante selon la revendication 17, désignée pADBTNF-BP, présentée sur la figure 7b.

20. Molécule d'ADN recombinante selon la revendication 17, désignée pADTNF-R, présentée sur la figure 7c.

21. Molécule d'ADN recombinante selon la revendication 17, désignée pADBTNF-R, présentée sur la figure 7d.

22. Cellule hôte, comprenant au moins une des molécules d'ADN recombinantes définies selon l'une des revendications 14 à 21, qui présente une séquence partielle de l'ADN codant pour la protéine liant le TNF.

23. Cellule hôte selon la revendication 22, **caractérisé en ce qu'**elle est une cellule hôte eucaryote, de préférence une cellule COS ou une cellule CHO.

24. Procédé de fabrication d'une protéine recombinante liant le TNF ou d'un dérivé fonctionnel de celle-ci, comprenant la culture d'un hôte selon la revendication 22 et l'expression de la protéine.

25. Procédé selon la revendication 24, comprenant comme autre étape l'isolement de la protéine exprimée.

26. Polypeptide recombinant, **caractérisé en ce qu'**il est codé par un ADN, qui est choisi dans le groupe qui code pour l'un des polypeptides suivants :

(i) leu val pro his leu gly asp arg glu lys arg
asp ser val cys pro gln gly lys tyr ile his pro
gln asn asn ser ile cys cys thr lys cys his lys
gly thr tyr leu tyr asn asp cys pro gly pro gly
gln asp thr asp cys arg glu cys glu ser gly ser
phe thr ala ser glu asn his leu arg his cys leu
ser cys ser lys cys arg lys glu met gly gln val
glu ile ser ser cys thr val asp arg asp thr val
cys gly cys arg lys asn gln tyr arg his tyr trp
ser glu asn leu phe gln cys phe asn cys ser leu
cys leu asn gly thr val his leu ser cys gln glu
lys gln asn thr val cys thr cys his ala gly phe
phe leu arg glu asn glu cys val ser cys ser asn
cys lys lys ser leu glu cys thr lys leu cys leu
pro gln ile glu asn,

(B)

met gly leu ser thr val pro asp leu leu leu pro
leu val leu leu glu leu leu val gly ile tyr pro
ser gly val ile gly leu val pro his leu gly asp
arg glu lys arg asp ser val cys pro gln gly lys
tyr ile his pro gln asn asn ser ile cys cys thr
lys cys his lys gly thr tyr leu tyr asn asp cys
pro gly pro gly gln asp thr asp cys arg glu cys
glu ser gly ser phe thr ala ser glu asn his leu
arg his cys leu ser cys ser lys cys arg lys glu
met gly gln val glu ile ser ser cys thr val asp
arg asp thr val cys gly cys arg lys asn gln tyr
arg his tyr trp ser glu asn leu phe gln cys phe
asn cys ser leu cys leu asn gly thr val his leu
ser cys gln glu lys gln asn thr val cys thr cys
his ala gly phe phe leu arg glu asn glu cys val
ser cys ser asn cys lys lys ser leu glu cys thr
lys leu cys leu pro gln ile glu asn

(ii)
leu val pro his leu gly asp
arg glu lys arg asp ser val cys pro gln gly lys
tyr ile his pro gln asn asn ser ile cys cys thr
lys cys his lys gly thr tyr leu tyr asn asp cys
pro gly pro gly gln asp thr asp cys arg glu cys
glu ser gly ser phe thr ala ser glu asn his leu
arg his cys leu ser cys ser lys cys arg lys glu
met gly gln val glu ile ser ser cys thr val asp
arg asp thr val cys gly cys arg lys asn gln tyr
arg his tyr trp ser glu asn leu phe gln cys phe
asn cys ser leu cys leu asn gly thr val his leu
ser cys gln glu lys gln asn thr val cys thr cys
his ala gly phe phe leu arg glu asn glu cys val
ser cys ser asn cys lys lys ser leu glu cys thr
lys leu cys leu pro gln ile glu asn val lys gly
thr glu asp ser gly thr thr

27. Polypeptide, caractérisé en qu'il comprend une protéine liant le TNF selon la formule suivante :

asp ser val cys pro gln gly lys tyr ile his pro
gln asn asn ser ile cys cys thr lys cys his lys

gly thr tyr leu tyr asn asp cys pro gly pro gly
gln asp thr asp cys arg glu cys glu ser gly ser
phe thr ala ser glu asn his leu arg his cys leu
ser cys ser lys cys arg lys glu met gly gln val
glu ile ser ser cys thr val asp arg asp thr val
cys gly cys arg lys asn gln tyr arg his tyr trp
ser glu asn leu phe gln cys phe asn cys ser leu
cys leu asn gly thr val his leu ser cys gln glu
lys gln asn thr val cys thr cys his ala gly phe
phe leu arg glu asn glu cys val ser cys ser asn
cys lys lys ser leu glu cys thr lys leu cys leu
pro gln ile glu asn

28. Polypeptide selon la revendication 27, caractérisé en qu'il comprend le récepteur de TNF de formule

met gly leu ser thr val pro asp leu leu leu pro
leu val leu leu glu leu leu val gly ile tyr pro
ser gly val ile gly leu val pro his leu gly asp
arg glu lys arg asp ser val cys pro gln gly lys
tyr ile his pro gln asn asn ser ile cys cys thr
lys cys his lys gly thr tyr leu tyr asn asp cys
pro gly pro gly gln asp thr asp cys arg glu cys
glu ser gly ser phe thr ala ser glu asn his leu
arg his cys leu ser cys ser lys cys arg lys glu
met gly gln val glu ile ser ser cys thr val asp
arg asp thr val cys gly cys arg lys asn gln tyr
arg his tyr trp ser glu asn leu phe gln cys phe
asn cys ser leu cys leu asn gly thr val his leu
ser cys gln glu lys gln asn thr val cys thr cys
his ala gly phe phe leu arg glu asn glu cys val
ser cys ser asn cys lys lys ser leu glu cys thr
lys leu cys leu pro gln ile glu asn val lys gly
thr glu asp ser gly thr thr val leu leu pro leu
val ile phe phe gly leu cys leu leu ser leu leu
phe ile gly leu met tyr arg tyr gln arg trp lys
ser lys leu tyr ser ile val cys gly lys ser thr
pro glu lys glu gly glu leu glu gly thr thr thr
lys pro leu ala pro asn pro ser phe ser pro thr
pro gly phe thr pro thr leu gly phe ser pro val
pro ser ser thr phe thr ser ser ser thr tyr thr
pro gly asp cys pro asn phe ala ala pro arg arg
glu val ala pro pro tyr gln gly ala asp pro ile
leu ala thr ala leu ala ser asp pro ile pro asn
pro leu gln lys trp glu asp ser ala his lys pro
gln ser leu asp thr asp asp pro ala thr leu tyr
ala val val glu asn val pro pro leu arg trp

29. Utilisation d'un polypeptide défini dans les revendications 26 à 28 pour l'étude de substances portant sur leurs interactions avec ce polypeptide et/ou TNF-α et/ou TNF-β et/ou sur leur influence sur l'effet biologique de TNF-α et/ou TNF-β in vitro.

30. Utilisation d'une cellule hôte définie dans la revendication 24 pour l'étude de substances portant sur leurs interactions avec le récepteur de TNF et/ou leur influence sur l'effet biologique de TNF-α et/ou TNF-β.

31. Utilisation d'un polypeptide défini dans les revendications 26 à 28 pour la fabrication d'un médicament pour le traitement de maladies auto-immunes.

32. Utilisation d'un polypeptide défini dans les revendications 26 à 28 pour la fabrication d'un médicament pour le traitement de l'arthrite rhumatoïde.

33. Utilisation d'un polypeptide défini dans les revendications 26 à 28 pour la fabrication d'un médicament pour le traitement du syndrome de détresse respiratoire de l'adulte.

34. Utilisation d'un polypeptide défini dans les revendications 26 à 28 pour la fabrication d'un médicament pour le traitement de l'anorexie/la cachexie.

35. Utilisation d'un polypeptide défini dans les revendications 26 à 28 pour la fabrication d'un médicament pour le traitement de la réaction du greffon contre l'hôte.

36. Utilisation d'un polypeptide défini dans les revendications 26 à 28 pour la fabrication d'un médicament pour le traitement d'un choc.

37. Utilisation d'un polypeptide défini dans les revendications 26 à 28 comme diagnostic pour la détermination de TNF-α et/ou TNF-β.

38. Préparation pharmaceutique, **caractérisée en ce qu'**elle comprend comme composants thérapeutiquement actifs l'un des polypeptides définis dans l'une des revendications 26 à 28.

## Revendications (Revendications pour l'(les) Etat(s) contractant(s) suivant(s): ES)

1. Procédé de fabrication d'un ADN, comprenant l'amplification d'un ADN, qui code pour un polypeptide ayant la capacité de lier le TNF, le polypeptide comprenant une séquence d'acides aminés de formule : 
asp ser val cys pro gln gly lys tyr ile his pro
gln asn asn ser ile cys cys thr lys cys his lys
gly thr tyr leu tyr asn asp cys pro gly pro gly
gln asp thr asp cys arg glu cys glu ser gly ser
phe thr ala ser glu asn his leu arg his cys leu
ser cys ser lys cys arg lys glu met gly gln val
glu ile ser ser cys thr val asp arg asp thr val
cys gly cys arg lys asn gln tyr arg his tyr trp
ser glu asn leu phe gln cys phe asn cys ser leu
cys leu asn gly thr val his leu ser cys gln glu
lys gln asn thr val cys thr cys his ala gly phe
phe leu arg glu asn glu cys val ser cys ser asn
cys lys lys ser leu glu cys thr lys leu cys leu
pro gln ile glu asn
 ou un fragment fonctionnel ou une modification de celui-ci ayant la capacité de lier le TNF.

2. Procédé selon la revendication 1, **caractérisé en ce que** le polypeptide comprend une séquence d'acides aminés de formule : 
leu val pro his leu gly asp arg glu lys arg
asp ser val cys pro gln gly lys tyr ile his pro
gln asn asn ser ile cys cys thr lys cys his lys
gly thr tyr leu tyr asn asp cys pro gly pro gly
gln asp thr asp cys arg glu cys glu ser gly ser
phe thr ala ser glu asn his leu arg his cys leu
ser cys ser lys cys arg lys glu met gly gln val
glu ile ser ser cys thr val asp arg asp thr val
cys gly cys arg lys asn gln tyr arg his tyr trp
ser glu asn leu phe gln cys phe asn cys ser leu
cys leu asn gly thr val his leu ser cys gln glu
lys gln asn thr val cys thr cys his ala gly phe
phe leu arg glu asn glu cys val ser cys ser asn
cys lys lys ser leu glu cys thr lys leu cys leu
pro gln ile glu asn
 ou un fragment fonctionnel ou une modification de celui-ci ayant la capacité de lier le TNF.

3. Procédé selon la revendication 2, **caractérisé en ce que** le polypeptide comprend une séquence d'acides aminés de formule : 
met gly leu ser thr val pro asp leu leu leu pro
leu val leu leu glu leu leu val gly ile tyr pro
ser gly val ile gly leu val pro his leu gly asp
arg glu lys arg asp ser val cys pro gln gly lys
tyr ile his pro gln asn asn ser ile cys cys thr
lys cys his lys gly thr tyr leu tyr asn asp cys
pro gly pro gly gln asp thr asp cys arg glu cys
glu ser gly ser phe thr ala ser glu asn his leu
arg his cys leu ser cys ser lys cys arg lys glu
met gly gln val glu ile ser ser cys thr val asp
arg asp thr val cys gly cys arg lys asn gln tyr
arg his tyr trp ser glu asn leu phe gln cys phe
asn cys ser leu cys leu asn gly thr val his leu
ser cys gln glu lys gln asn thr val cys thr cys
his ala gly phe phe leu arg glu asn glu cys val
ser cys ser asn cys lys lys ser leu glu cys thr
lys leu cys leu pro gln ile glu asn
 ou un fragment fonctionnel ou une modification de celui-ci ayant la capacité de lier le TNF.

4. Procédé selon la revendication 3, **caractérisé en ce que** le polypeptide comprend une séquence d'acides aminés de formule : 
met gly leu ser thr val pro asp leu leu leu pro
leu val leu leu glu leu leu val gly ile tyr pro
ser gly val ile gly leu val pro his leu gly asp
arg glu lys arg asp ser val cys pro gln gly lys
tyr ile his pro gln asn asn ser ile cys cys thr
lys cys his lys gly thr tyr leu tyr asn asp cys
pro gly pro gly gln asp thr asp cys arg glu cys
glu ser gly ser phe thr ala ser glu asn his leu
arg his cys leu ser cys ser lys cys arg lys glu
met gly gln val glu ile ser ser cys thr val asp
arg asp thr val cys gly cys arg lys asn gln tyr
arg his tyr trp ser glu asn leu phe gln cys phe
asn cys ser leu cys leu asn gly thr val his leu
ser cys gln glu lys gln asn thr val cys thr cys
his ala gly phe phe leu arg glu asn glu cys val
ser cys ser asn cys lys lys ser leu glu cys thr
lys leu cys leu pro gln ile glu asn val lys gly
thr glu asp ser gly thr thr val leu leu pro leu
val ile phe phe gly leu cys leu leu ser leu leu
phe ile gly leu met tyr arg tyr gln arg trp lys
ser lys leu tyr ser ile val cys gly lys ser thr
pro glu lys glu gly glu leu glu gly thr thr thr
lys pro leu ala pro asn pro ser phe ser pro thr
pro gly phe thr pro thr leu gly phe ser pro val
pro ser ser thr phe thr ser ser ser thr tyr thr
pro gly asp cys pro asn phe ala ala pro arg arg
glu val ala pro pro tyr gln gly ala asp pro ile
leu ala thr ala leu ala ser asp pro ile pro asn
pro leu gln lys trp glu asp ser ala his lys pro
gln ser leu asp thr asp asp pro ala thr leu tyr

ala val val glu asn val pro pro leu arg trp
 ou un fragment fonctionnel ou une modification de celui-ci ayant la capacité de lier le TNF.

5. Procédé selon la revendication 1, **caractérisé en ce que** le polypeptide comprend une séquence d'acides aminés de formule : 
asp ser val cys pro gln gly lys
tyr ile his pro gln asn asn ser ile cys cys thr
lys cys his lys gly thr tyr leu tyr asn asp cys
pro gly pro gly gln asp thr asp cys arg glu cys
glu ser gly ser phe thr ala ser glu asn his leu
arg his cys leu ser cys ser lys cys arg lys glu
met gly gln val glu ile ser ser cys thr val asp
arg asp thr val cys gly cys arg lys asn gln tyr
arg his tyr trp ser glu asn leu phe gln cys phe
asn cys ser leu cys leu asn gly thr val his leu
ser cys gln glu lys gln asn thr val cys thr cys
his ala gly phe phe leu arg glu asn glu cys val
ser cys ser asn cys lys lys ser leu glu cys thr
lys leu cys leu pro gln ile glu asn val lys gly
thr glu asp ser gly thr thr
 ou un fragment fonctionnel ou une modification de celui-ci ayant la capacité de lier le TNF.

6. Procédé selon la revendication 5, **caractérisé en ce que** le polypeptide comprend une séquence d'acides aminés de formule : 
leu val pro his leu gly asp
arg glu lys arg asp ser val cys pro gln gly lys
tyr ile his pro gln asn asn ser ile cys cys thr
lys cys his lys gly thr tyr leu tyr asn asp cys
pro gly pro gly gln asp thr asp cys arg glu cys
glu ser gly ser phe thr ala ser glu asn his leu
arg his cys leu ser cys ser lys cys arg lys glu
met gly gln val glu ile ser ser cys thr val asp
arg asp thr val cys gly cys arg lys asn gln tyr
arg his tyr trp ser glu asn leu phe gln cys phe
asn cys ser leu cys leu asn gly thr val his leu
ser cys gln glu lys gln asn thr val cys thr cys
his ala gly phe phe leu arg glu asn glu cys val
ser cys ser asn cys lys lys ser leu glu cys thr
lys leu cys leu pro gln ile glu asn val lys gly
thr glu asp ser gly thr thr
 ou un fragment fonctionnel ou une modification de celui-ci ayant la capacité de lier le TNF.

7. Procédé selon la revendication 1, **caractérisé en ce que** la séquence d'acides aminés du polypeptide est modifiée en un ou plusieurs sites de glycosylation.

8. Procédé selon la revendication 1, dans lequel l'ADN code pour une protéine pouvant être sécrétée liant le TNF, **caractérisé en ce que** l'ADN comprend une séquence d'acides nucléiques de formule 
R= GAT AGT GTG TGT CCC CAA GGA AAA TAT ATC CAC
CCT CAA AAT AAT TCG ATT TGC TGT ACC AAG TGC CAC
AAA GGA ACC TAC TTG TAC AAT GAC TGT CCA GGC CCG
GGG CAG GAT ACG GAC TGC AGG GAG TGT GAG AGC GGC
TCC TTC ACC GCT TCA GAA AAC CAC CTC AGA CAC TGC
CTC AGC TGC TCC AAA TGC CGA AAG GAA ATG GGT CAG
GTG GAG ATC TCT TCT TGC ACA GTG GAC CGG GAC ACC
GTG TGT GGC TGC AGG AAG AAC CAG TAC CGG CAT TAT
TGG AGT GAA AAC CTT TTC CAG TGC TTC AAT TGC AGC
CTC TGC CTC AAT GGG ACC GTG CAC CTC TCC TGC CAG
GAG AAA CAG AAC ACC GTG TGC ACC TGC CAT GCA GGT
TTC TTT CTA AGA GAA AAC GAG TGT GTC TCC TGT AGT
AAC TGT AAG AAA AGC CTG GAG TGC ACG AAG TTG TGC
CTA CCC CAG ATT GAG AAT
 ou un fragment, une variante ou modification de cette séquence d'acides nucléiques, qui code pour un polypeptide ayant la capacité de lier le TNF, dans lequel R² représente un ADN codant pour un (poly)peptide pouvant être excisé in vivo.

9. Procédé selon la revendication 8, dans lequel l'ADN code pour une protéine pouvant être sécrétée liant le TNF, **caractérisé en ce que** R² représente un ADN codant pour tout ou partie d'une séquence signal.

10. Procédé selon la revendication 8, caractérisé en que R² comprend une séquence d'acides nucléiques de formule

CTG GTC CCT CAC CTA GGG GAC AGG GAG AAG AGA

11. Procédé selon la revendication 9, caractérisé en que R² comprend une séquence d'acides nucléiques de formule R3 CTG GTC CCT CAC CTA GGG GAC AGG GAG AAG AGA , dans laquelle R³ représente un ADN codant pour un peptide signal.

12. Procédé selon la revendication 11, caractérisé en que R³ comprend une séquence d'acides nucléiques de formule

ATG GGC CTC TCC ACC GTG CCT GAC CTG CTG CTG CGA
CTG GTG CTC CTG GAG CTG TTG GTG GGA ATA TAC CCC
TCA GGG GTT ATT GGA

13. Procédé selon la revendication 1, **caractérisé en ce que** l'ADN comprend un ADN de formule 
ATG GGC CTC TCC ACC GTG CCT GAC CTG CTG CTG CCA CTG GTG CTC
CTG GAG CTG TTG GTG GGA ATA TAC CCC TCA GGG GTT ATT GGA CTG
GTC CCT CAC CTA GGG GAC AGG GAG AAG AGA|GAT AGT GTG TGT CCC
CAA GGA AAA TAT ATC CAC CCT CAA AAT AAT TCG ATT TGC TGT ACC
AAG TGC CAC AAA GGA ACC TAC TTG TAC AAT GAC TGT CCA GGC CCG
GGG CAG GAT ACG GAC TGC AGG GAG TGT GAG AGC GGC TCC TTC ACC
GCT TCA GAA AAC CAC CTC AGA CAC TGC CTC AGC TGC TCC AAA TGC
CGA AAG GAA ATG GGT CAG GTG GAG ATC TCT TCT TGC ACA GTG GAC
CGG GAC ACC GTG TGT GGC TGC AGG AAG AAC CAG TAC CGG CAT TAT
TGG AGT GAA AAC CTT TTC CAG TGC TTC AAT TGC AGC CTC TGC CTC
 
AAT GGG ACC GTG CAC CTC TCC TGC CAG GAG AAA CAG AAC ACC GTG
TGC ACC TGC CAT GCA GGT TTG TTT CTA AGA GAA AAC GAG TGT GTC
TCC TGT AGT AAC TGT AAG AAA AGC CTG GAG TGC ACG AAG TTG TGC
CTA CCC CAG ATT GAG AAT GTT AAG GGC ACT GAG GAC TCA GGC ACC
ACA GTG CTG TTG CCC CTG GTC ATT TTC TTT GGT CTT TGC CTT TTA
TCC CTC CTC TTC ATT GGT TTA ATG TAT CGC TAC CAA CGG TGG AAG
TCC AAG CTC TAC TCC ATT GTT TGT GGG AAA TCG ACA CCT GAA AAA
GAG GGG GAG CTT GAA GGA ACT ACT ACT AAG CCC CTG GCC CCA AAC
CCA [?] [?] AGT CCC ACT CCA GGC TTC ACC CCC ACC CTG GGC TTC
[?] CCC GTG CCC AGT [?] ACC TTC ACC TCC AGC TCC ACC TAT ACC
[?] GGT GAC TGT CCG AAC TTT GCG GCT CCC CGC AGA GAG GTG GCA
CCA CCC TAT CAG GGG GCT GAC CCC ATC CTT GCG ACA GCC CTC GCC
TCC GAC CCC ATC CCC AAC CCC CTT CAG AAG TGG GAG GAC AGC GCC
CAC AAG CCA CAG AGC CTA GAC ACT GAT GAC CCC GCG ACG CTG TAC
GCC GTG GTG GAG AAC GTG CCC CCG TTG CGC TGG AA GGAATTC
 ou un fragment, une variante ou modification de cette séquence d'acides nucléiques, qui code pour un polypeptide ayant la capacité de lier le TNF.

14. Procédé de fabrication d'une molécule d'ADN recombinante, comprenant l'amplification d'un ADN, dans lequel la molécule d'ADN comprend un ADN que l'on peut obtenir par un procédé selon l'une des revendications 1 à 13.

15. Procédé selon la revendication 14, dans lequel la molécule d'ADN est réplicable dans des cellules hôtes procaryotes ou eucaryotes, et qui comprend des séquences de contrôle de l'expression liées de façon fonctionnelle à l'ADN que l'on peut obtenir par un procédé selon l'une des revendications 1 à 13.

16. Procédé selon la revendication 15, dans lequel la molécule d'ADN recombinante est réplicable dans des cellules de mammifère.

17. Procédé selon la revendication 14, **caractérisé en ce que** la molécule d'ADN recombinante comprend l'ADN défini dans la revendication 9.

18. Procédé selon la revendication 17, **caractérisé en ce que** la molécule d'ADN recombinante est pADTNF-BP, présentée sur la figure 7a.

19. Procédé selon la revendication 17, **caractérisé en ce que** la molécule d'ADN recombinante est pADBTNF-BP, présentée sur la figure 7b.

20. Procédé selon la revendication 17, **caractérisé en ce que** la molécule d'ADN recombinante est pADTNF-R, présentée sur la figure 7c.

21. Procédé selon la revendication 17, **caractérisé en ce que** la molécule d'ADN recombinante est pADBTNF-R, présentée sur la figure 7d.

22. Procédé de fabrication d'une cellule hôte, comprenant la transformation d'une cellule avec au moins une des molécules d'ADN recombinantes, que l'on peut obtenir par un procédé selon l'une des revendications 14 à 21, dans lequel la molécule d'ADN représente une séquence partielle de l'ADN codant pour la protéine liant le TNF.

23. Procédé selon la revendication 22, **caractérisé en ce que** la cellule hôte est une cellule hôte eucaryote, de préférence une cellule COS ou une cellule CHO.

24. Procédé de fabrication d'une protéine recombinante liant le TNF ou d'un dérivé fonctionnel de celle-ci, comprenant la culture d'une cellule hôte que l'on peut obtenir par un procédé selon la revendication 22 et l'expression de la protéine.

25. Procédé selon la revendication 24, comprenant comme autre étape l'isolement de la protéine exprimée.

26. Procédé de fabrication d'un polypeptide recombinant, comprenant l'expression d'un ADN, qui est choisi dans le groupe qui code pour l'un des polypeptides suivants :

(I) leu val pro his leu gly asp arg glu lys arg
asp ser val cys pro gln gly lys tyr ile his pro
glu asn asn ser ile cys cys thr lys cys his lys
gly thr tyr leu tyr asn asp cys pro gly pro gly
gln asp thr asp cys arg glu cys glu ser gly ser
phe thr ala ser glu asn his leu arg his cys leu
ser cys ser lys cys arg lys glu met gly gln val
glu ile ser ser cys thr val asp arg asp thr val
cys gly cys arg lys asn gln tyr arg his tyr trp
ser glu asn leu phe gln cys phe asn cys ser leu
cys leu asn gly thr val his leu ser cys gln glu
lys gln asn thr val cys thr cys his ala gly phe
phe leu arg glu asn glu cys val ser cys ser asn
cys lys lys ser leu glu cys thr lys leu cys leu
pro gln ile glu asn,

(ii)

met gly leu ser thr val pro asp leu leu leu pro
leu val leu leu glu leu leu val gly ile tyr pro
ser gly val ile gly leu val pro his leu gly asp
arg glu lys arg asp ser val cys pro gln gly lys
tyr ile his pro gln asn asn ser ile cys cys thr
lys cys his lys gly thr tyr leu tyr asn asp cys
pro gly pro gly gln asp thr asp cys arg glu cys
glu ser gly ser phe thr ala ser glu asn his leu
arg his cys leu ser cys ser lys cys arg lys glu
met gly gln val glu ile ser ser cys thr val asp
arg asp thr val cys gly cys arg lys asn gln tyr
arg his tyr trp ser glu asn leu phe gln cys phe
asn cys ser leu cys leu asn gly thr val his leu
ser cys gln glu lys gln asn thr val cys thr cys
his ala gly phe phe leu arg glu asn glu cys val
ser cys ser asn cys lys lys ser leu glu cys thr
lys leu cys leu pro gln ile glu asn

(iii)

leu val pro his leu gly asp
arg glu lys arg asp ser val cys pro gln gly lys
tyr ile his pro gln asn asn ser ile cys cys thr
lys cys his lys gly thr tyr leu tyr asn asp cys
pro gly pro gly gln asp thr asp cys arg glu cys
glu ser gly ser phe thr ala ser glu asn his leu
arg his cys leu ser cys ser lys cys arg lys glu
met gly gln val glu ile ser ser cys thr val asp
arg asp thr val cys gly cys arg lys asn gln tyr
arg his tyr trp ser glu asn leu phe gln cys phe
asn cys ser leu cys leu asn gly thr val his leu
ser cys gln glu lys gln asn thr val cys thr cys
his ala gly phe phe leu arg glu asn glu cys val
ser cys ser asn cys lys lys ser leu glu cys thr
lys leu cys leu pro gln ile glu asn val lys gly
thr glu asp ser gly thr thr

27. Procédé de fabrication d'un polypeptide, caractérisé en qu'il exprime de façon recombinante une protéine liant le TNF selon la formule suivante :

asp ser val cys pro gln gly lys tyr ile his pro
gln asn asn ser ile cys cys thr lys cys his lys

gly thr tyr leu tyr asn asp cys pro gly pro gly
gln asp thr asp cys arg glu cys glu ser gly ser
phe thr ala ser glu asn his leu arg his cys leu
ser cys ser lys cys arg lys glu met gly gln val
glu ile ser ser cys thr val asp arg asp thr val
cys gly cys arg lys asn gln tyr arg his tyr trp
ser glu asn leu phe gln cys phe asn cys ser leu
cys leu asn gly thr val his leu ser cys gln glu
lys gln asn thr val cys thr cys his ala gly phe
phe leu arg glu asn glu cys val ser cys ser asn
cys lys lys ser leu glu cys thr lys leu cys leu
pro gln ile glu asn

28. Procédé selon la revendication 27, caractérisé en que le polypeptide comprend le récepteur de TNF de formule

met gly leu ser thr val pro asp leu leu leu pro
leu val leu leu glu leu leu val gly ile tyr pro
ser gly val ile gly leu val pro his leu gly asp
arg glu lys arg asp ser val cys pro gln gly lys
tyr ile his pro gln asn asn ser ile cys cys thr
lys cys his lys gly thr tyr leu tyr asn asp cys
pro gly pro gly gln asp thr asp cys arg glu cys
glu ser gly ser phe thr ala ser glu asn his leu
arg his cys leu ser cys ser lys cys arg lys glu
met gly gln val glu ile ser ser cys thr val asp
arg asp thr val cys gly cys arg lys asn gln tyr
arg his tyr trp ser glu asn leu phe gln cys phe
asn cys ser leu cys leu asn gly thr val his leu
ser cys gln glu lys gln asn thr val cys thr cys
his ala gly phe phe leu arg glu asn glu cys val
ser cys ser asn cys lys lys ser leu glu cys thr
lys leu cys leu pro gln ile glu asn val lys gly
thr glu asp ser gly thr thr val leu leu pro leu
val ile phe phe gly leu cys leu leu ser leu leu
phe ile gly leu met tyr arg tyr gln arg trp lys
ser lys leu tyr ser ile val cys gly lys ser thr
pro glu lys glu gly glu leu glu gly thr thr thr
lys pro leu ala pro asn pro ser phe ser pro thr
pro gly phe thr pro thr leu gly phe ser pro val
pro ser ser thr phe thr ser ser ser thr tyr thr
pro gly asp cys pro asn phe ala ala pro arg arg
glu val ala pro pro tyr gln gly ala asp pro ile
leu ala thr ala leu ala ser asp pro ile pro asn
pro leu gln lys trp glu asp ser ala his lys pro
gln ser leu asp thr asp asp pro ala thr leu tyr
ala val val glu asn val pro pro leu arg trp

29. Procédé pour l'étude de substances, **caractérisé en ce qu'** on utilise un polypeptide, que l'on peut obtenir par un procédé selon l'une des revendications 26 à 28, pour déterminer les interactions entre ces substances et ce polypeptide et/ou TNF-α et/ou TNF-β et/ou l'influence de ces substances sur l'effet biologique de TNF-α et/ou TNF-*β in vitro*.

30. Procédé d'étude de substances, **caractérisé en ce que** l'on détermine les interactions entre ces substances et le récepteur de TNF et/ou leur influence sur l'effet biologique de TNF-α et/ou TNF-β avec une cellule hôte que l'on peut obtenir selon la revendication 24.

31. Procédé de fabrication d'un médicament pour le traitement de maladies auto-immunes, **caractérisé en ce qu'**un polypeptide, que l'on peut obtenir par un procédé selon l'une des revendications 26 à 28, est combiné à un additif pharmaceutiquement acceptable.

32. Procédé de fabrication d'un médicament pour le traitement de l'arthrite rhumatoïde, **caractérisé en ce qu'**un polypeptide, que l'on peut obtenir par un procédé selon l'une des revendications 26 à 28, est combiné à des additifs pharmaceutiquement acceptables.

33. Procédé de fabrication d'un médicament pour le traitement du syndrome de détresse respiratoire de l'adulte, **caractérisé en ce qu'**un polypeptide, que l'on peut obtenir par un procédé selon l'une des revendications 26 à 28, est combiné à des additifs pharmaceutiquement acceptables.

34. Procédé de fabrication d'un médicament pour le traitement de l'anorexie/la cachexie, **caractérisé en ce qu'**un polypeptide, que l'on peut obtenir par un procédé selon l'une des revendications 26 à 28, est combiné à des additifs pharmaceutiquement acceptables.

35. Procédé de fabrication d'un médicament pour le traitement de la réaction du greffon contre l'hôte, **caractérisé en ce qu'**un polypeptide, que l'on peut obtenir par un procédé selon l'une des revendications 26 à 28, est combiné à des additifs pharmaceutiquement acceptables.

36. Procédé de fabrication d'un médicament pour le traitement d'un choc, **caractérisé en ce qu'**un polypeptide, que l'on peut obtenir par un procédé selon l'une des revendications 26 à 28, est combiné à des additifs pharmaceutiquement acceptables.

37. Procédé de fabrication d'un diagnostic pour la détermination de TNF-α et/ou TNF-β, **caractérisé en ce qu'**un polypeptide, que l'on peut obtenir par un procédé selon l'une des revendications 26 à 28, est préparé conjointement avec des anticorps contre le TNF.

38. Procédé de fabrication d'une préparation pharmaceutique, **caractérisé en ce qu'**un polypeptide, que l'on peut obtenir par un procédé selon l'une des revendications 26 à 28, est combiné à des additifs pharmaceutiquement acceptables.

## Revendications (Revendications pour l'(les) Etat(s) contractant(s) suivant(s): GR)

1. ADN, codant pour un polypeptide ayant la capacité de lier le TNF, dans lequel le polypeptide comprend une séquence d'acides aminés de formule : 
asp ser val cys pro gln gly lys tyr ile his pro
gln asn asn ser ile cys cys thr lys cys his lys
gly thr tyr leu tyr asn asp cys pro gly pro gly
gln asp thr asp cys arg glu cys glu ser gly ser
phe thr ala ser glu asn his leu arg his cys leu
ser cys ser lys cys arg lys glu met gly gln val
glu ile ser ser cys thr val asp arg asp thr val
cys gly cys arg lys asn gln tyr arg his tyr trp
ser glu asn leu phe gln cys phe asn cys ser leu
cys leu asn gly thr val his leu ser cys gln glu
lys gln asn thr val cys thr cys his ala gly phe
phe leu arg glu asn glu cys val ser cys ser asn
cys lys lys ser leu glu cys thr lys leu cys leu
pro gln ile glu asn
 ou un fragment fonctionnel ou une modification de celui-ci ayant la capacité de lier le TNF.

2. ADN selon la revendication 1, **caractérisé en ce que** le polypeptide comprend une séquence d'acides aminés de formule : 
leu val pro his leu gly asp arg glu lys arg
asp ser val cys pro gln gly lys tyr ile his pro
gln asn asn ser ile cys cys thr lys cys his lys
gly thr tyr leu tyr asn asp cys pro gly pro gly
gln asp thr asp cys arg glu cys glu ser gly ser
phe thr ala ser glu asn his leu arg his cys leu
ser cys ser lys cys arg lys glu met gly gln val
glu ile ser ser cys thr val asp arg asp thr val
cys gly cys arg lys asn gln tyr arg his tyr trp
ser glu asn leu phe gln cys phe asn cys ser leu
cys leu asn gly thr val his leu ser cys gln glu
lys gln asn thr val cys thr cys his ala gly phe
phe leu arg glu asn glu cys val ser cys ser asn
cys lys lys ser leu glu cys thr lys leu cys leu
pro gln ile glu asn
 ou un fragment fonctionnel ou une modification de celui-ci ayant la capacité de lier le TNF.

3. ADN selon la revendication 2, **caractérisé en ce que** le polypeptide comprend une séquence d'acides aminés de formule : 
met gly leu ser thr val pro asp leu leu leu pro
leu val leu leu glu leu leu val gly ile tyr pro
ser gly val ile gly leu val pro his leu gly asp
arg glu lys arg asp ser val cys pro gln gly lys
tyr ile his pro gln asn asn ser ile cys cys thr
lys cys his lys gly thr tyr leu tyr asn asp cys
pro gly pro gly gln asp thr asp cys arg glu cys
glu ser gly ser phe thr ala ser glu asn his leu
arg his cys leu ser cys ser lys cys arg lys glu
met gly gln val glu ile ser ser cys thr val asp
arg asp thr val cys gly cys arg lys asn gln tyr
arg his tyr trp ser glu asn leu phe gln cys phe
asn cys ser leu cys leu asn gly thr val his leu
ser cys gln glu lys gln asn thr val cys thr cys
his ala gly phe phe leu arg glu asn glu cys val
ser cys ser asn cys lys lys ser leu glu cys thr
lys leu cys leu pro gln ile glu asn
 ou un fragment fonctionnel ou une modification de celui-ci ayant la capacité de lier le TNF.

4. ADN selon la revendication 3, **caractérisé en ce que** le polypeptide comprend une séquence d'acides aminés de formule : 
met gly leu ser thr val pro asp leu leu leu pro
leu val leu leu glu leu leu val gly ile tyr pro
ser gly val ile gly leu val pro his leu gly asp
arg glu lys arg asp ser val cys pro gln gly lys
tyr ile his pro gln asn asn ser ile cys cys thr
lys cys his lys gly thr tyr leu tyr asn asp cys
pro gly pro gly gln asp thr asp cys arg glu cys
glu ser gly ser phe thr ala ser glu asn his leu
arg his cys leu ser cys ser lys cys arg lys glu
met gly gln val glu ile ser ser cys thr val asp
arg asp thr val cys gly cys arg lys asn gln tyr
arg his tyr trp ser glu asn leu phe gln cys phe
asn cys ser leu cys leu asn gly thr val his leu
ser cys gln glu lys gln asn thr val cys thr cys
his ala gly phe phe leu arg glu asn glu cys val
ser cys ser asn cys lys lys ser leu glu cys thr
lys leu cys leu pro gln ile glu asn val lys gly
thr glu asp ser gly thr thr val leu leu pro leu
val ile phe phe gly leu cys leu leu ser leu leu
phe ile gly leu met tyr arg tyr gln arg trp lys
ser lys leu tyr ser ile val cys gly lys ser thr
pro glu lys glu gly glu leu glu gly thr thr thr
lys pro leu ala pro asn pro ser phe ser pro thr
pro gly phe thr pro thr leu gly phe ser pro val
pro ser ser thr phe thr ser ser ser thr tyr thr
pro gly asp cys pro asn phe ala ala pro arg arg
glu val ala pro pro tyr gln gly ala asp pro ile
leu ala thr ala leu ala ser asp pro ile pro asn
pro leu gln lys trp glu asp ser ala his lys pro
gln ser leu asp thr asp asp pro ala thr leu tyr

ala val val glu asn val pro pro leu arg trp
 ou un fragment fonctionnel ou une modification de celui-ci ayant la capacité de lier le TNF.

5. ADN selon la revendication 1, **caractérisé en ce que** le polypeptide comprend une séquence d'acides aminés de formule : 
asp ser val cys pro gln gly lys
tyr ile his pro gln asn asn ser ile cys cys thr
lys cys his lys gly thr tyr leu tyr asn asp cys
pro gly pro gly gln asp thr asp cys arg glu cys
glu ser gly ser phe thr ala ser glu asn his leu
arg his cys leu ser cys ser lys cys arg lys glu
met gly gln val glu ile ser ser cys thr val asp
arg asp thr val cys gly cys arg lys asn gln tyr
arg his tyr trp ser glu asn leu phe gln cys phe
asn cys ser leu cys leu asn gly thr val his leu
ser cys gln glu lys gln asn thr val cys thr cys
his ala gly phe phe leu arg glu asn glu cys val
ser cys ser asn cys lys lys ser leu glu cys thr
lys leu cys leu pro gln ile glu asn val lys gly
thr glu asp ser gly thr thr
 ou un fragment fonctionnel ou une modification de celui-ci ayant la capacité de lier le TNF.

6. ADN selon la revendication 5, **caractérisé en ce que** le polypeptide comprend une séquence d'acides aminés de formule : 
leu val pro his leu gly asp
arg glu lys arg asp ser val cys pro gln gly lys
tyr ile his pro gln asn asn ser ile cys cys thr
lys cys his lys gly thr tyr leu tyr asn asp cys
pro gly pro gly gln asp thr asp cys arg glu cys
glu ser gly ser phe thr ala ser glu asn his leu
arg his cys leu ser cys ser lys cys arg lys glu
met gly gln val glu ile ser ser cys thr val asp
arg asp thr val cys gly cys arg lys asn gln tyr
arg his tyr trp ser glu asn leu phe gln cys phe
asn cys ser leu cys leu asn gly thr val his leu
ser cys gln glu lys gln asn thr val cys thr cys
his ala gly phe phe leu arg glu asn glu cys val
ser cys ser asn cys lys lys ser leu glu cys thr
lys leu cys leu pro gln ile glu asn val lys gly
thr glu asp ser gly thr thr
 ou un fragment fonctionnel ou une modification de celui-ci ayant la capacité de lier le TNF.

7. ADN selon la revendication 1, **caractérisé en ce que** la séquence d'acides aminés du polypeptide est modifiée en un ou plusieurs sites de glycosylation.

8. ADN selon la revendication 1, codant pour une protéine pouvant être sécrétée liant le TNF, **caractérisé en ce qu'**elle comprend la séquence d'acides nucléiques de formule 
R= GAT AGT GTG TGT CCC CAA GGA AAA TAT ATC CAC
CCT CAA AAT AAT TCG ATT TGC TGT ACC AAG TGC CAC
AAA GGA ACC TAC TTG TAC AAT GAC TGT CCA GGC CCG
GGG CAG GAT ACG GAC TGC AGG GAG TGT GAG AGC GGC
TCC TTC ACC GCT TCA GAA AAC CAC CTC AGA CAC TGC
CTC AGC TGC TCC AAA TGC CGA AAG GAA ATG GGT CAG
GTG GAG ATC TCT TCT TGC ACA GTG GAC CGG GAC ACC
GTG TGT GGC TGC AGG AAG AAC CAG TAC CGG CAT TAT
TGG AGT GAA AAC CTT TTC CAG TGC TTC AAT TGC AGC
CTC TGC CTC AAT GGG ACC GTG CAC CTC TCC TGC CAG
GAG AAA CAG AAC ACC GTG TGC ACC TGC CAT GCA GGT
TTC TTT CTA AGA GAA AAC GAG TGT GTC TCC TGT AGT
AAC TGT AAG AAA AGC CTG GAG TGC ACG AAG TTG TGC
CTA CCC CAG ATT GAG AAT
 ou un fragment, une variante ou modification de cette séquence d'acides nucléiques, qui code pour un polypeptide ayant la capacité de lier le TNF, dans lequel R² représente un ADN codant pour un (poly)peptide pouvant être excisé in vivo.

9. ADN selon la revendication 8, codant pour une protéine pouvant être sécrétée liant le TNF, **caractérisé en ce que** R² est un ADN codant pour tout ou partie d'une séquence signal.

10. ADN selon la revendication 8, caractérisé en que R² comprend une séquence d'acides nucléiques de formule

CTG GTC CCT CAC CTA GGG GAC AGG GAG AAG AGA

11. ADN selon la revendication 9, caractérisé en que R² comprend une séquence d'acides nucléiques de formule R3 CTG GTC CCT GAC CTA GGG GAC AGG GAG AAG AGA , dans laquelle R³ représente un ADN codant pour un peptide signal.

12. ADN selon la revendication 11, caractérisé en que R³ comprend une séquence d'acides nucléiques de formule

ATG GGC CTC TCC ACC GTG CCT GAC CTG CTG CTG CGA
CTG GTG CTC CTG GAG CTG TTG GTG GGA ATA TAC CCC
TCA GGG GTT ATT GGA

13. ADN selon la revendication 1, **caractérisé en ce que** l'ADN comprend un ADN de formule 
ATG GGC CTC TCC ACC GTG CCT GAC CTG CTG CTG CCA CTG GTG CTC
CTG GAG CTG TTG GTG GGA ATA TAC CCC TCA GGG GTT ATT GGA CTG
GTC CCT CAC CTA GGG GAC AGG GAG AAG AGA|GAT AGT GTG TGT CCC
CAA GGA AAA TAT ATC CAC CCT CAA AAT AAT TCG ATT TGC TGT ACC
AAG TGC CAC AAA GGA ACC TAC TTG TAC AAT GAC TGT CCA GGC CCG
GGG CAG GAT ACG GAC TGC AGG GAG TGT GAG AGC GGC TCC TTC ACC
GCT TCA GAA AAC CAC CTC AGA CAC TGC CTC AGC TGC TCC AAA TGC
CGA AAG GAA ATG GGT CAG GTG GAG ATC TCT TCT TGC ACA GTG GAC
CGG GAC ACC GTG TGT GGC TGC AGG AAG AAC CAG TAC CGG CAT TAT
TGG AGT GAA AAC CTT TTC CAG TGC TTC AAT TGC AGC CTC TGC CTC

AAT GGG ACC GTG CAC CTC TCC TGC CAG GAG AAA CAG AAC ACC GTG
TGC ACC TGC CAT GCA GGT TTC TTT CTA AGA GAA AAC GAG TGT GTC
TCC TGT AGT AAC TGT AAG AAA AGC CTG GAG TGC ACG AAG TTG TGC
CTA CCC CAG ATT GAG AAT GTT AAG GGC ACT GAG GAC TCA GGC ACC
ACA GTG CTG TTG CCC CTG GTC ATT TTC TTT GGT CTT TGC CTT TTA
TCC CTC CTC TTC ATT GGT TTA ATG TAT CGC TAC CAA CGG TGG AAG
TCC AAG CTC TAC TCC ATT GTT TGT GGG AAA TCG ACA CCT GAA AAA
GAG GGG GAG CTT GAA GGA ACT ACT ACT AAG CCC CTG GCC CCA AAC
CCA [?] [?] [?] [?] ACT CCA GGC TTC ACC CCC ACC CTG GGC TTC
AGT CCC GTG CCC AGT TCC ACC TTC ACC TCC AGC TCC ACC TAT ACC
CCC GGT GAC TGT CCC AAC TTT GCG GCT CCC CGC AGA GAG GTG GCA
CCA CCC TAT CAG GGG GCT GAC CCC ATC CTT GCG ACA GCC CTC GCC
TCC GAC CCC ATC CCC AAC CCC CTT CAG AAG TGG GAG GAC AGC GCC
CAC AAG CCA CAG AGC CTA GAC ACT GAT GAC CCC GCG ACG CTG TAC
GCC GTG GTG GAG AAC GTG CCC CCG TTG CGC TGG AA GGAATTC
 ou un fragment, une variante ou modification de cette séquence d'acides nucléiques, qui code pour un polypeptide ayant la capacité de lier le TNF.

14. Molécule d'ADN recombinante, **caractérisée en ce qu'**elle comprend la molécule d'ADN selon l'une des revendications 1 à 13.

15. Molécule d'ADN recombinante selon la revendication 14, réplicable dans des cellules hôtes procaryotes ou eucaryotes, **caractérisée en ce qu'**elle comprend des séquences de contrôle de l'expression liées de façon fonctionnelle à l'ADN selon l'une des revendications 1 à 13.

16. Molécule d'ADN recombinante selon la revendication 15, réplicable dans des cellules de mammifère.

17. Molécule d'ADN recombinante selon la revendication 14, **caractérisée en ce qu'**elle comprend l'ADN défini dans la revendication 9.

18. Molécule d'ADN recombinante selon la revendication 17, désignée pADTNF-BP, présentée sur la figure 7a.

19. Molécule d'ADN recombinante selon la revendication 17, désignée pADBTNF-BP, présentée sur la figure 7b.

20. Molécule d'ADN recombinante selon la revendication 17, désignée pADTNF-R, présentée sur la figure 7c.

21. Molécule d'ADN recombinante selon la revendication 17, désignée pADBTNF-R, présentée sur la figure 7d.

22. Cellule hôte, comprenant au moins une des molécules d'ADN recombinantes définies selon l'une des revendications 14 à 21, qui comprend une séquence partielle de l'ADN codant pour la protéine liant le TNF.

23. Cellule hôte selon la revendication 22, **caractérisé en ce qu'**elle est une cellule hôte eucaryote, de préférence une cellule COS ou une cellule CHO.

24. Procédé de fabrication d'une protéine recombinante liant le TNF ou d'un dérivé fonctionnel de celle-ci, comprenant la culture d'un hôte selon la revendication 22 et l'expression de la protéine.

25. Procédé selon la revendication 24, comprenant comme autre étape l'isolement de la protéine exprimée.

26. Polypeptide recombinant, **caractérisé en ce qu'**il est codé par un ADN, qui est choisi dans le groupe qui code pour l'un des polypeptides suivants :

(I) leu val pro his leu gly asp arg glu lys arg
asp ser val cys pro gln gly lys tyr ile his pro
gln asn asn ser ile cys cys thr lys cys his lys
gly thr tyr leu tyr asn asp cys pro gly pro gly
gln asp thr asp cys arg glu cys glu ser gly ser
phe thr ala ser glu asn his leu arg his cys leu
ser cys ser lys cys arg lys glu met gly gln val
glu ile ser ser cys thr val asp arg asp thr val
cys gly cys arg lys asn gln tyr arg his tyr trp
ser glu asn leu phe gln cys phe asn cys ser leu
cys leu asn gly thr val his leu ser cys gln glu
lys gln asn thr val cys thr cys his ala gly phe
phe leu arg glu asn glu cys val ser cys ser asn
cys lys lys ser leu glu cys thr lys leu cys leu
pro gln ile glu asn ,

(ii)

met gly leu ser thr val pro asp leu leu leu pro
leu val leu leu glu leu leu val gly ile tyr pro
ser gly val ile gly leu val pro his leu gly asp
arg glu lys arg asp ser val cys pro gln gly lys
tyr ile his pro gln asn asn ser ile cys cys thr
lys cys his lys gly thr tyr leu tyr asn asp cys
pro gly pro gly gln asp thr asp cys arg glu cys
glu ser gly ser phe thr ala ser glu asn his leu
arg his cys leu ser cys ser lys cys arg lys glu
met gly gln val glu ile ser ser cys thr val asp
arg asp thr val cys gly cys arg lys asn gln tyr
arg his tyr trp ser glu asn leu phe gln cys phe
asn cys ser leu cys leu asn gly thr val his leu
ser cys gln glu lys gln asn thr val cys thr cys
his ala gly phe phe leu arg glu asn glu cys val
ser cys ser asn cys lys lys ser leu glu cys thr
lys leu cys leu pro gln ile glu asn

(iii)
leu val pro his leu gly asp
arg glu lys arg asp ser val cys pro gln gly lys
tyr ile his pro gln asn asn ser ile cys cys thr
lys cys his lys gly thr tyr leu tyr asn asp cys
pro gly pro gly gln asp thr asp cys arg glu cys
glu ser gly ser phe thr ala ser glu asn his leu
arg his cys leu ser cys ser lys cys arg lys glu
met gly gln val glu ile ser ser cys thr val asp
arg asp thr val cys gly cys arg lys asn gln tyr
arg his tyr trp ser glu asn leu phe gln cys phe
asn cys ser leu cys leu asn gly thr val his leu
ser cys gln glu lys gln asn thr val cys thr cys
his ala gly phe phe leu arg glu asn glu cys val
ser cys ser asn cys lys lys ser leu glu cys thr
lys leu cys leu pro gln ile glu asn val lys gly
thr glu asp ser gly thr thr

27. Polypeptide, caractérisé en qu'il comprend de façon recombinante une protéine liant le TNF selon la formule suivante :

asp ser val cys pro gln gly lys tyr ile his pro
gln asn asn ser ile cys cys thr lys cys his lys

gly thr tyr leu tyr asn asp cys pro gly pro gly
gln asp thr asp cys arg glu cys glu ser gly ser
phe thr ala ser glu asn his leu arg his cys leu
ser cys ser lys cys arg lys glu met gly gln val
glu ile ser ser cys thr val asp arg asp thr val
cys gly cys arg lys asn gln tyr arg his tyr trp
ser glu asn leu phe gln cys phe asn cys ser leu
cys leu asn gly thr val his leu ser cys gln glu
lys gln asn thr val cys thr cys his ala gly phe
phe leu arg glu asn glu cys val ser cys ser asn
cys lys lys ser leu glu cys thr lys leu cys leu
pro gln ile glu asn

28. Polypeptide selon la revendication 27, caractérisé en qu'il comprend la récepteur de TNF de formule

met gly leu ser thr val pro asp leu leu leu pro
leu val leu leu glu leu leu val gly ile tyr pro
ser gly val ile gly leu val pro his leu gly asp
arg glu lys arg asp ser val cys pro gln gly lys
tyr ile his pro gln asn asn ser ile cys cys thr
lys cys his lys gly thr tyr leu tyr asn asp cys
pro gly pro gly gln asp thr asp cys arg glu cys
glu ser gly ser phe thr ala ser glu asn his leu
arg his cys leu ser cys ser lys cys arg lys glu
met gly gln val glu ile ser ser cys thr val asp
arg asp thr val cys gly cys arg lys asn gln tyr
arg his tyr trp ser glu asn leu phe gln cys phe
asn cys ser leu cys leu asn gly thr val his leu
ser cys gln glu lys gln asn thr val cys thr cys
his ala gly phe phe leu arg glu asn glu cys val
ser cys ser asn cys lys lys ser leu glu cys thr
lys leu cys leu pro gln ile glu asn val lys gly
thr glu asp ser gly thr thr val leu leu pro leu
val ile phe phe gly leu cys leu leu ser leu leu
phe ile gly leu met tyr arg tyr gln arg trp lys
ser lys leu tyr ser ile val cys gly lys ser thr
pro glu lys glu gly glu leu glu gly thr thr thr
lys pro leu ala pro asn pro ser phe ser pro thr
pro gly phe thr pro thr leu gly phe ser pro val
pro ser ser thr phe thr ser ser ser thr tyr thr
pro gly asp cys pro asn phe ala ala pro arg arg
glu val ala pro pro tyr gln gly ala asp pro ile
leu ala thr ala leu ala ser asp pro ile pro asn
pro leu gln lys trp glu asp ser ala his lys pro
gln ser leu asp thr asp asp pro ala thr leu tyr
ala val val glu asn val pro pro leu arg trp

29. Utilisation d'un polypeptide défini dans les revendications 26 à 28 pour l'étude de substances portant sur leurs interactions avec ce polypeptide et/ou avec TNF-α et/ou avec TNF-β et/ou sur leur influence sur l'effet biologique de TNF-α et/ou TNF-β in vitro.

30. Utilisation d'une cellule hôte définie dans la revendication 24 pour l'étude de substances portant sur leurs interactions avec le récepteur de TNF et/ou leur influence sur l'effet biologique de TNF-α et/ou TNF-β.

31. Utilisation d'un polypeptide défini dans les revendications 26 à 28 pour la fabrication d'un médicament pour le traitement de maladies auto-immunes.

32. Utilisation d'un polypeptide défini dans les revendications 26 à 28 pour la fabrication d'un médicament pour le traitement de l'arthrite rhumatoïde.

33. Utilisation d'un polypeptide défini dans les revendications 26 à 28 pour la fabrication d'un médicament pour le traitement du syndrome de détresse respiratoire de l'adulte.

34. Utilisation d'un polypeptide défini dans les revendications 26 à 28 pour la fabrication d'un médicament pour le traitement de l'anorexie/la cachexie.

35. Utilisation d'un polypeptide défini dans les revendications 26 à 28 pour la fabrication d'un médicament pour le traitement de la réaction du greffon contre l'hôte.

36. Utilisation d'un polypeptide défini dans les revendications 26 à 28 pour la fabrication d'un médicament pour le traitement d'un choc.

37. Utilisation d'un polypeptide défini dans les revendications 26 à 28 comme diagnostic pour la détermination de TNF-α et/ou TNF-β.

38. Procédé de fabrication d'une préparation pharmaceutique, **caractérisé en ce qu'**un polypeptide selon l'une des revendications 26 à 28 est combiné à des additifs pharmaceutiquement acceptables.
